# EUROPEAN PATENT SPECIFICATION

(11) **EP 3 267 983 B1**
(45) Date of publication and mention of the grant of the patent: **20.12.2023**
(21) Application number: 16711088.1
(22) Date of filing: 09.03.2016
(51) Int. Cl.: A61K 31/00, A61K 31/439, A61P 25/16, A61P 25/28

(54) **METHODS FOR TREATING PROTEINOPATHIES**
VERFAHREN ZUR BEHANDLUNG VON PROTEINOPATHIEN
PROCÉDÉS DE TRAITEMENT DE PROTÉINOPATHIES

(30) Priority: 10.03.2015 US 201562131071 P
(43) Date of publication of application: 17.01.2018
(73) Proprietor: Genzyme Corporation, Cambridge, MA 02141 (US)
(72) Inventor: CHENG, Seng, H., Cambridge, MA 02141 (US); SHIHABUDDIN, Lamya, Cambridge, MA 02141 (US); SARDI, Sergio, Pablo, Cambridge, MA 02141 (US)
(74) Representative: Garner, Stephen
(86) International application number: PCT/US2016/021512
(87) International publication number: WO 2016/145046

(56) References cited:
- WO-A1-2012/177997
- WO-A1-2014/043068
- WO-A2-2012/129084
- WEI SHEN ET AL: "Inhibition of glucosylceramide synthase stimulates autophagy flux in neurons", JOURNAL OF NEUROCHEMISTRY, vol. 129, no. 5, 3 March 2014 (2014-03-03) , pages 884-894, XP055270020, NEW YORK, NY, US ISSN: 0022-3042, DOI: 10.1111/jnc.12672
- PABLO SARDI S ET AL: "Glucosylceramide synthase inhibition reduces [alpha]-synuclein pathology and improves cognition in murine models of synucleinopathy", MOLECULAR GENETICS AND METABOLISM, vol. 117, no. 2, 2016, XP029416563, ISSN: 1096-7192, DOI: 10.1016/J.YMGME.2015.12.427
- NOELKER CARMEN ET AL: "Glucocerebrosidase deficiency and mitochondrial impairment in experimental Parkinson disease", JOURNAL OF NEUROLOGICAL SCIENCES, ELSEVIER SCIENTIFIC PUBLISHING CO, AMSTERDAM, NL, vol. 356, no. 1, 17 June 2015 (2015-06-17) , pages 129-136, XP029259820, ISSN: 0022-510X, DOI: 10.1016/J.JNS.2015.06.030

## Description

This disclosure relates to methods for preventing, reducing or reversing loss of neural function in subjects diagnosed as having, or at risk of developing, proteinopathies, wherein the loss of neural function comprises loss of cognitive function, and to quinuclidine compounds for use in said methods. The disclosure relates particularly to the oral administration of quinuclidine compounds for preventing, reducing or reversing loss of neural function in subjects diagnosed as having, or at risk of developing, tauopathies and/or synucleinopathies, e.g. Parkinson's disease, wherein the loss of neural function comprises loss of cognitive function.

### SUMMARY OF THE INVENTION

In medicine, proteinopathy refers to a class of diseases in which certain proteins become structurally abnormal, and thereby disrupt the function of cells, tissues and organs of the body. Often the proteins fail to fold into their normal configuration. In this misfolded state, the proteins can become toxic in some way (a gain of toxic function) or they can lose their normal function. The proteinopathies include diseases such as Alzheimer's disease, Parkinson's disease, amyloidosis, and a wide range of other disorders.

Proteinopathies are widespread throughout the population. For example, nearly one million people in the US are living with Parkinson's disease and as many as 5.1 million Americans have Alzheimer's disease. There are currently no cures for these diseases, and many of the molecular mechanisms underlying the disease and progression of the disease are unknown.

Tauopathies form one particular class of proteinopathies. These are a collection of neurodegenerative disorders characterised pathologically by the presence of aggregates of phosphorylated tau protein, typically in the form of neurofibrillary tangles or Pick's bodies. These disorders are age-related and are often, to a greater or lesser extent, inherited. For example, mutations in *MAPT* (encoding the microtubule-associated protein tau in humans, located on chromosome 17q21) account for around 30% of inherited cases of frontotemporal dementia. Several human tau isoforms are known to be generated by alternative splicing of *MAPT* and mutations in this gene can result in altered levels of these isoforms which may lead to protein aggregation and disease progression.

Another class of proteinopathies is characterised by structurally abnormal α-synuclein proteins. These diseases are known collectively as synucleinopathies. α-synuclein is a protein encoded by the *SNCA* gene in humans. In particular, α-synuclein can aggregate to form insoluble fibrils in pathological disorders characterized by Lewy bodies. These disorders include, for example, Parkinson's disease and Lewy body dementia.

Tau- and α-synuclein-associated pathologies are frequently found in tandem in patients with Parkinson's disease and in patients with Lewy body dementia. In these cases, the diseases may be characterized both as tauopathies and as synucleinopathies.

Although there are no cures for these devastating diseases, there are a number of small molecule drugs available to help alleviate the symptoms of some proteinopathies. Examples of lysosomal activating agents which may be useful in the treatment of proteinopathies are described in WO 2012/177997 A1. However, there is a real need in the art to develop therapeutics effective in alleviating or managing the symptoms associated with proteinopathies, especially proteinopathies such as Parkinson's disease and Lewy body dementia. There is a particular need to develop therapeutics effective in treating the underlying pathophysiology of proteinopathies.

The present inventors have determined that certain quinuclidine compounds can reduce, reverse or prevent protein aggregation in tissues of subjects with proteinopathies. The inventors have also determined that these quinuclidine compounds can ameliorate memory deficits in animal models of proteinopathies. These results indicate that treatments with quinuclidine compounds as described herein will be effective to treat the underlying pathophysiology of proteinopathies.

Accordingly, the present invention provides a compound of formula (I), or a pharmaceutically acceptable salt thereof, for use in a method of preventing, reducing or reversing loss of neural function in a subject diagnosed as having, or at risk of developing, a proteinopathy, 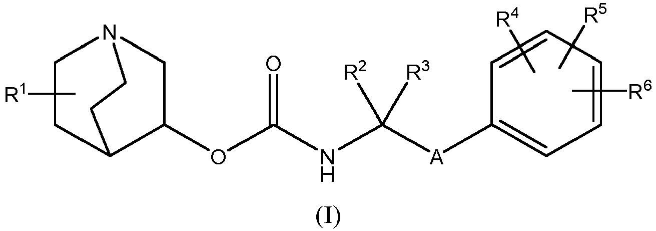 wherein:
R¹ is hydrogen;
   a halogen, or a cyano, nitro, hydroxy, thio or amino group; or
   a C₁₋₆-alkyl, C₂₋₆-alkenyl, C₂₋₆-alkynyl, C₁₋₆-alkyloxy, C₂₋₆-alkenyloxy or C₂₋₆-alkynyloxy group, optionally substituted by one or more (*e.g.* 1, 2 or 3) groups independently selected from a halogen; and a cyano, nitro, hydroxy, thio, or amino group; R² and R³ are each independently selected from a C₁₋₃-alkyl group, optionally substituted by one or more halogens; or R² and R³ together form a cyclopropyl or cyclobutyl group, optionally substituted by one or more halogens;
R⁴, R⁵ and R⁶ are each independently selected from hydrogen; a halogen; a nitro, hydroxy, thio or amino group; and a C₁₋₆-alkyl or C₁₋₆-alkyloxy group, optionally substituted by one or more groups selected from a halogen; a hydroxy or cyano group; and a C₁₋₆-alkyloxy group; and
A is a 5- or 6-membered aryl or heteroaryl group
wherein the loss of neural function comprises loss of cognitive function.

In one embodiment, R¹ is hydrogen; fluorine; or a methyl or ethyl group optionally substituted by a halogen, or a hydroxy, thio or amino group.

In embodiments, (i) R² and R³ are each independently selected from methyl and ethyl groups, optionally substituted with one or more fluorine atoms; (ii) R⁴ is selected from a halogen; and a C₁₋₃-alkyl or C₁₋₃-alkyloxy group, optionally substituted by one or more groups selected from a halogen and a C₁₋₃-alkyloxy group; and/or (iii) R⁵ and R⁶ are both hydrogen. In embodiments, R⁴ is fluorine or a 2-methoxyethoxy group, and R⁵ and R⁶ are hydrogen. In embodiments, R⁴ is in a position on the benzene ring *para* to the group A.

In embodiments, A is phenyl, optionally substituted with 1, 2 or 3 groups independently selected from a halogen; and a hydroxy, thio, amino, nitro, oxo or methyl group. In one embodiment, the groups -C(R²R³)- and -(C₆H₂R⁴R⁵R⁶) are attached to group A in a 1,3- or a 1,4- relationship.

In other embodiments, A is a 5-membered heteroaryl group which contains 1 or 2 heteroatoms selected from N and S. In one embodiment, the groups -C(R²R³)- and -(C₆H₂R⁴R⁵R⁶) are attached to group A in a 1,3- relationship.

In embodiments, the compound or pharmaceutically acceptable salt is a compound of formula (II), (III) or (IV), 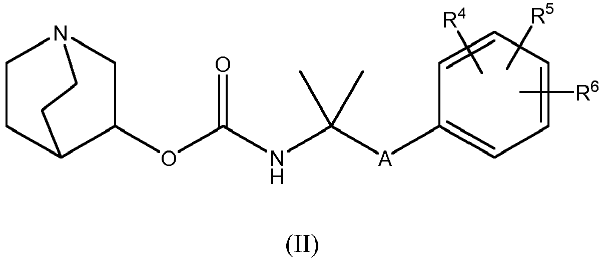 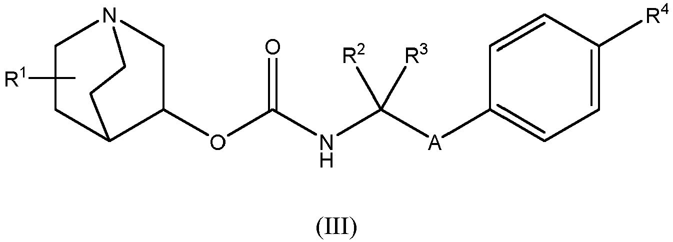 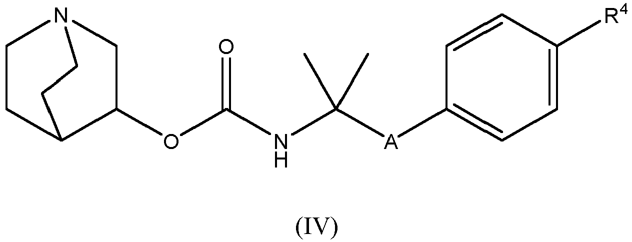 or a pharmaceutically acceptable salt thereof.

In one embodiment, the compound or pharmaceutically acceptable salt is a compound of formula (V), 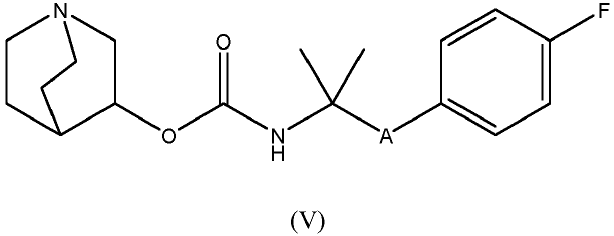 or a pharmaceutically acceptable salt thereof.

In other embodiments, the compound or pharmaceutically acceptable salt is a compound of formula (VI), (VII) or (VIII), 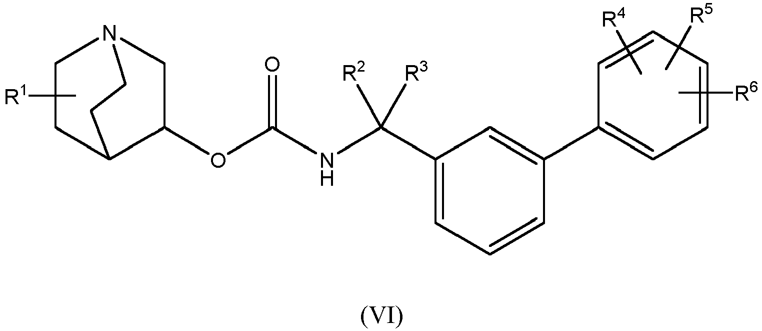 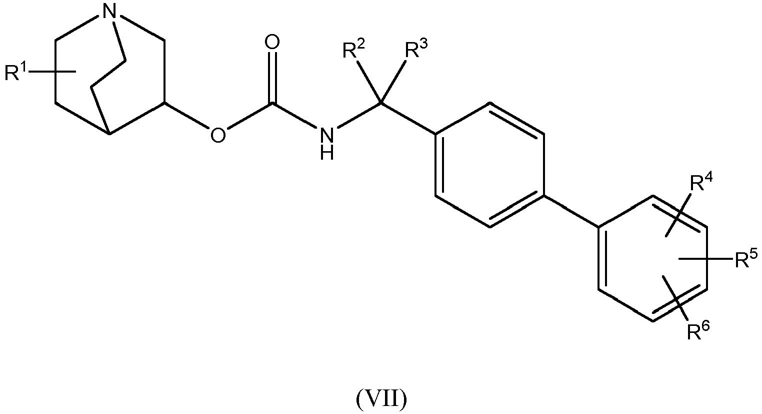 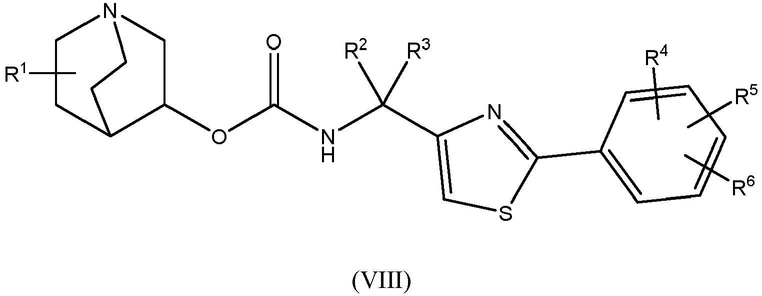 or a pharmaceutically acceptable salt thereof.

In one embodiment, the compound or pharmaceutically acceptable salt is a compound of formula (IX) or (XI), 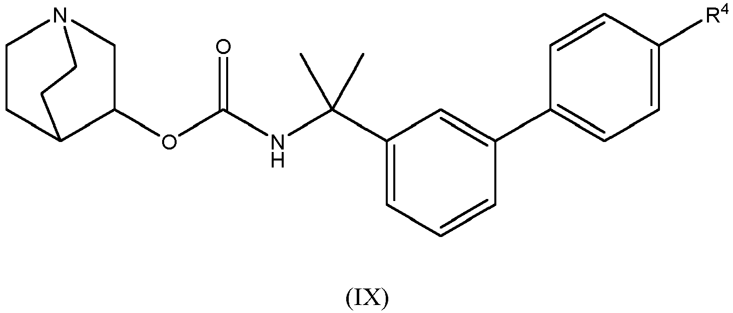 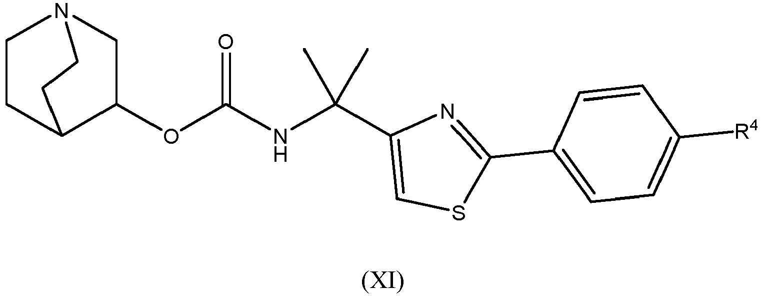 or a pharmaceutically acceptable salt thereof. In one embodiment, R⁴ is fluorine.

In particular embodiments, the compound or pharmaceutically acceptable salt is selected from: quinuclidin-3-yl (2-(4'-fluoro-[1,1'-biphenyl]-3-yl)propan-2-yl)carbamate; (S)-quinuclidin-3-yl (2-(2-(4-fluorophenyl)thiazol-4-yl)propan-2-yl)carbamate; (S)-quinuclidin-3-yl (2-(4'-(2-methoxyethoxy)-[1, 1 '-biphenyl]-4-yl)propan-2-yl)carbamate; and the pharmaceutically acceptable salts thereof.

In embodiments, the proteinopathy is a tauopathy. In one embodiment, said tauopathy is selected from Parkinson's disease, Alzheimer's disease, Lewy Body Dementia, Pick's disease, progressive supranuclear palsy, dementia pugilistica, parkinsonism linked to chromosome 17, Lytico-Bodig disease, tangle predominant dementia, Argyrophilic grain disease, ganglioglioma, gangliocytoma, meningioangiomatosis, subacute sclerosing panencephalitis, lead encephalopathy, tuberous sclerosis, Hallervorden-Spatz disease, lipofuscinosis, corticobasal degeneration, frontotemporal dementia, frontotemporal lobar degeneration and Huntington's disease.

In embodiments, said subject does not have protein aggregates comprising α-synuclein in their CNS (*e.g.* in neurons of the substantia nigra, cerebral cortex, hippocampus, frontal lobes and/or temporal lobes).

In embodiments, said tauopathy is Parkinson's disease characterised by the presence of protein tau, but not α-synuclein, within protein aggregates in the CNS of said subject (e.g. in neurons of the substantia nigra, cerebral cortex, hippocampus, frontal lobes and/or temporal lobes).

In other embodiments, the proteinopathy is a synucleinopathy. In one embodiment, said synucleinopathy is selected from Lewy Body Dementia, Parkinson's disease and multiple system atrophy.

In embodiments, said method prevents, reduces or reverses the progression of dementia in the subject.

In embodiments, said subject is a mammal, *e.g.* a human.

In embodiments, said compound, or pharmaceutically acceptable salt thereof, is administered by systemic administration, *e.g.* via a non-parenteral route. In one embodiment, said compound, or pharmaceutically acceptable salt thereof, is administered orally.

In embodiments, the method prevents, reduces or reverses deterioration in cognitive domains in the subject. In one embodiment, the method prevents, reduces or reverses deterioration in attention and concentration, executive functions, memory (e.g. working memory), language, visuo-constructional skills, conceptual thinking, calculations, orientation, decision making and/or problem solving.

In embodiments, the loss of neural function comprises loss of autonomic function, in addition to loss of cognitive function, and the method additionally prevents, reduces or reverses orthostatic hypotension, constipation, dysphagia, nausea, hypersalivation, hyperhydrosis and/or urinary and sexual dysfunction.

In embodiments, the loss of neural function comprises loss of motor function, in addition to loss of cognitive function, and the method additionally prevents, reduces or reverses Parkinsonism. In one embodiment, the method prevents, reduces or reverses motor dysfunction (*e.g.* tremor), bradykinesia, rigidity, postural instability and/or impaired balance.

In embodiments, the method prevents, reduces or reverses early symptoms of dementia (*e.g.* difficulty remembering recent conversations, names or events, and/or apathy and depression). In one embodiment, the method prevents, reduces or reverses later symptoms of dementia (*e.g.* impaired communication, poor judgment, disorientation, confusion, behavior changes and/or difficulty in speaking, swallowing and/or walking).

Additional features and advantages of compounds, compositions and methods disclosed herein will be apparent from the following detailed description.

### BRIEF DESCRIPTION OF THE DRAWINGS

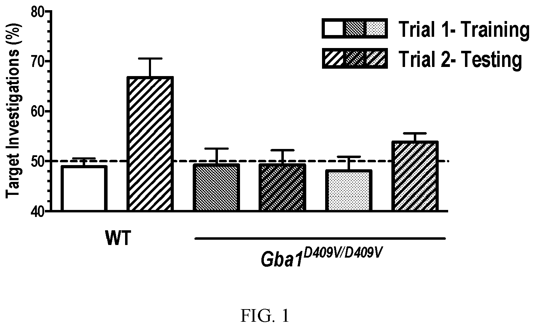

**FIG. 1** shows the results of a novel object recognition test carried out on mice, both wild-type (WT) and also in a proteinopathy model *(Gba1*^{*D409V*/}*^{D409V}).* Solid bars show target investigations during training and hatched bars show target investigations during testing. White bars (left of the figure) show results in WT mice, dark grey bars (middle of the figure) show results in untreated *Gba1*^{*D409V*/*D409V*} mice, and light grey bars (right of the figure) show results in *Gba1*^{*D409V*/*D409V*} mice treated with **Compound 1.**

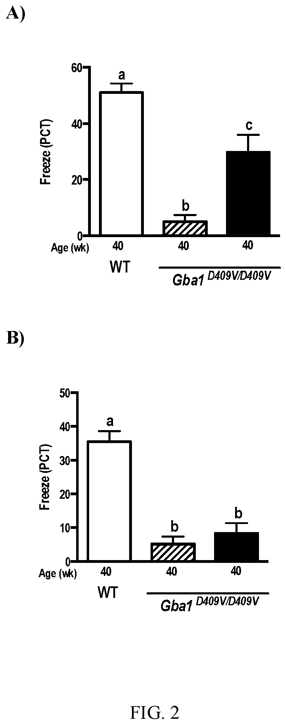

**FIG. 2** shows the results of fear conditioning tests carried out on mice, both wild-type (WT) and also in a proteinopathy model *(Gba1*^{*D409V*/}*^{D409V}).* Figure 2A shows results relating to contextual memory. Figure 2B shows the results relating to cued memory. White bars (left of the figures) show results in WT mice, hatched bars (middle of the figures) show results in untreated *Gba1*^{*D409V*/*D409V*} mice, and black bars (right of the figures) show results in *Gba1*^{*D409V*/*D409V*} mice treated with **Compound** 1.

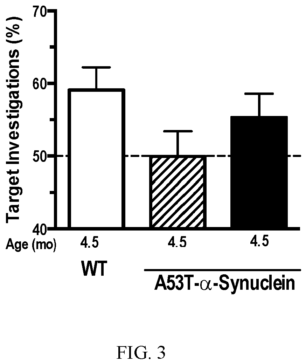

**FIG. 3** shows the results of target investigations during testing in a novel object recognition test carried out on mice, both wild-type (WT) and also in a proteinopathy model overexpressing A53T α-synuclein (training results not shown). White bars (left of the figure) show results in WT mice, hatched bars (middle of the figure) show results in untreated A53T mice, and black bars (right of the figure) show results in A53T mice treated with **Compound 1.**

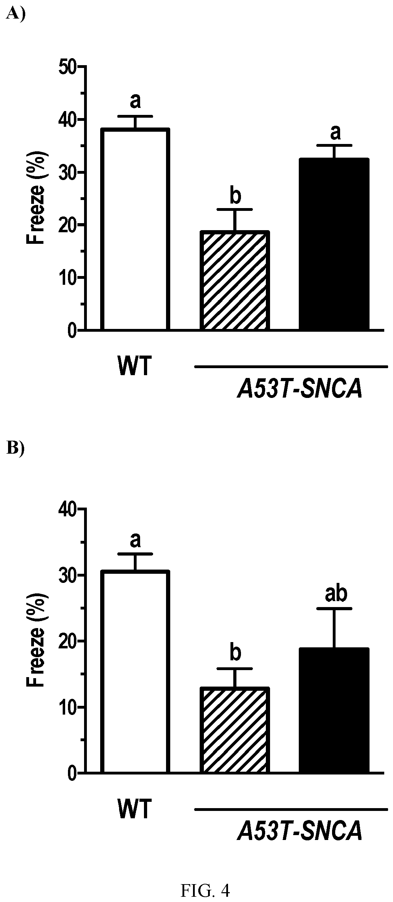

**FIG. 4** shows the results of fear conditioning tests carried out on mice, both wild-type (WT) and also in a proteinopathy model (overexpressing A53T α-synuclein). Figure 4A shows results relating to contextual memory. Figure 4B shows the results relating to cued memory. White bars (left of the figures) show results in WT mice, hatched bars (middle of the figures) show results in untreated A53T mice, and black bars (right of the figures) show results in A53T mice treated with **Compound 1.**

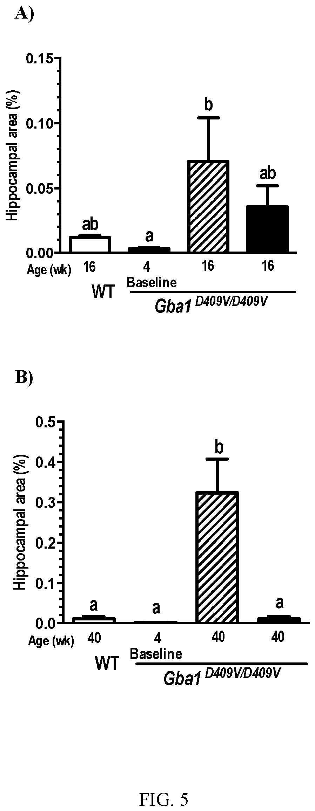

**FIG.** 5 shows hippocampal quantification of ubiquitin aggregates in both wild-type and *Gba1*^{*D409V*/*D409V*} mice. Figure 5A shows results at 16 weeks and Figure 5B shows results at 40 weeks. White bars (far left of the figures) show results in WT mice, solid bars (second left of the figures) show baseline levels in untreated *Gba1*^{*D409V*/*D409V*} mice at 4 weeks, hatched bars (second right of the figures) show results in untreated *Gba1*^{*D409V*/*D409V*} mice, and black bars (far right of the figures) show results in *Gba1*^{*D409V*/*D409V*} mice treated with **Compound 1.**

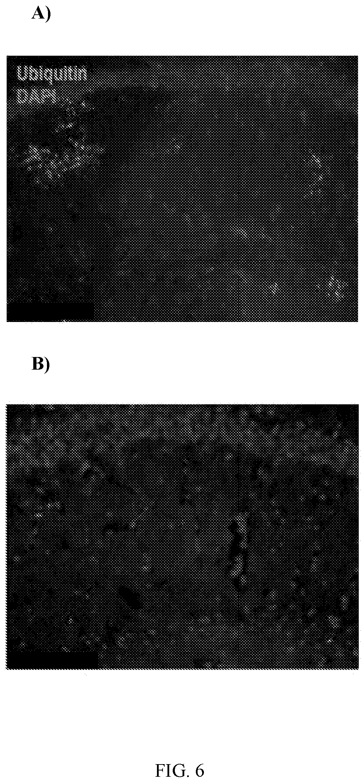

**FIG. 6** shows ubiquitin immunoreactivity (green) in the hippocampi of 40 week-old *Gba1*^{*D409V*/*D409V*} mice, either control (Fig. 6A) or treated with **Compound 1** (Fig. 6B). DAPI nuclear staining is shown in blue.

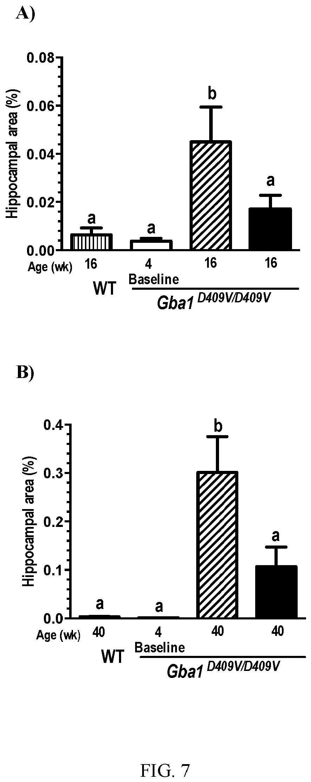

**FIG. 7** shows hippocampal quantification of proteinase K-resistant α-synuclein aggregates in both wild-type and *Gba1*^{*D409V*/*D409V*} mice*.* Figure 7A shows results at 16 weeks and Figure 7B shows results at 40 weeks. Striped bars (far left of the figures) show results in WT mice, white bars (second left of the figures) show baseline levels in untreated *Gba1*^{*D409V*/*D409V*} mice at 4 weeks, hatched bars (second right of the figures) show results in untreated *Gba1*^{*D409V*/*D409V*} mice, and black bars (far right of the figures) show results in *Gba1*^{*D409V*/*D409V*} mice treated with **Compound 1.**

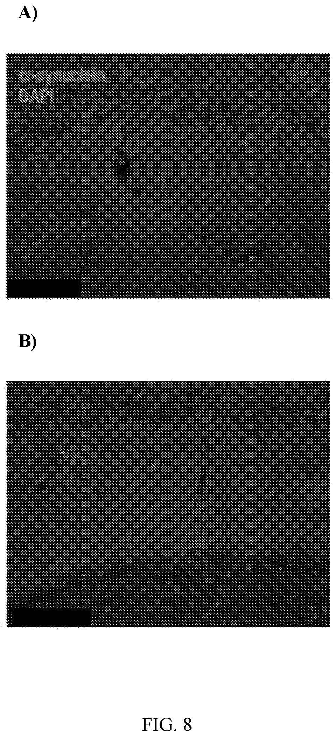

**FIG. 8** shows proteinase K-resistant α-synuclein immunoreactivity (red) in the hippocampi of 40 week-old *Gba1*^{*D409V*/*D409V*} mice, either control (Fig. 8A) or treated with **Compound 1** (Fig. 8B). DAPI nuclear staining is shown in blue.

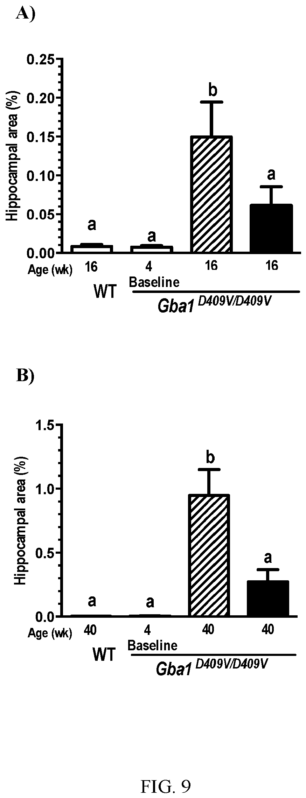

**FIG. 9** shows hippocampal quantification of protein tau aggregates in both wild-type and *Gba1*^{*D409V*/*D409V*} mice. Figure 9A shows results at 16 weeks and Figure 9B shows results at 40 weeks. White bars (far left of the figures) show results in WT mice, solid bars (second left of the figures) show baseline levels in untreated *Gba1*^{*D409V*/*D409V*} mice at 4 weeks, hatched bars (second right of the figures) show results in untreated *Gba1*^{*D409V*/*D409V*} mice, and black bars (far right of the figures) show results in *Gba1*^{*D409V*/*D409V*} mice treated with **Compound 1.**

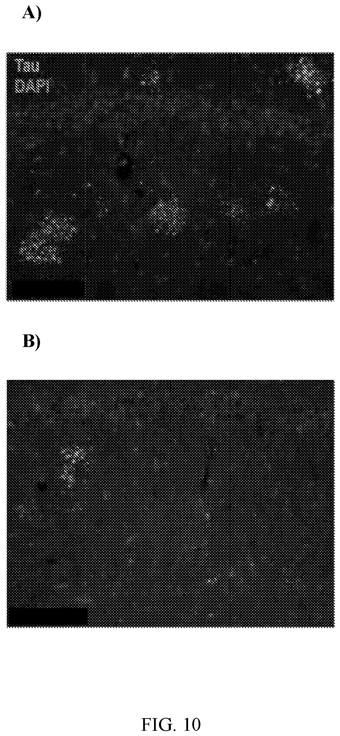

**FIG. 10** shows protein tau immunoreactivity (green) in the hippocampi of 40 week-old *Gba1*^{*D409V*/*D409V*} mice, either control (Fig. 10A) or treated with **Compound 1** (Fig. 10B). DAPI nuclear staining is shown in blue.

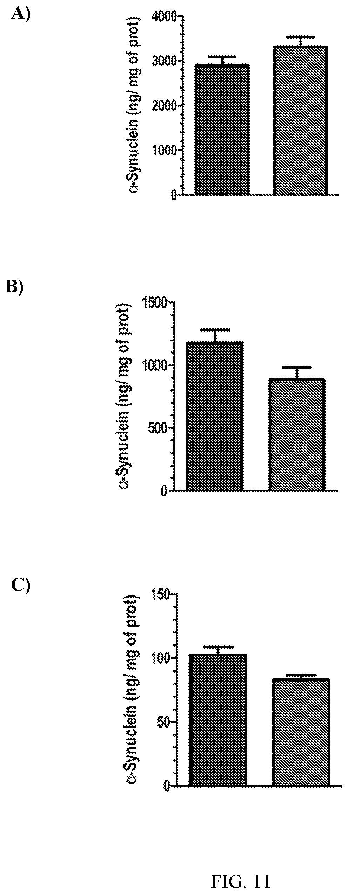

**FIG. 11** shows the subcellular localisation of α-synuclein in cortical tissue homogenates from A53T mice, at 8 months of age. The levels of cytosolic soluble (Fig. 11A), membrane-associated (Fig. 11B), and cytosolic insoluble α-synuclein (Fig. 11C) are shown in untreated (left-hand black bar) and treated (right-hand grey bar) mice.

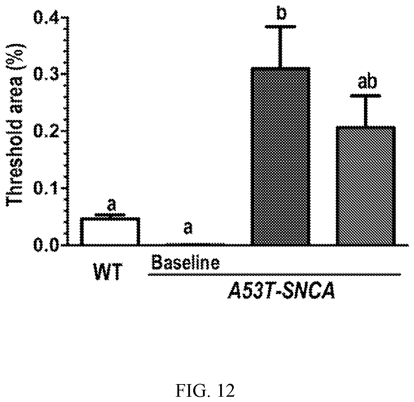

**FIG. 12** shows hippocampal quantification of ubiquitin aggregates in both wild-type and A53T mice, at 8 months of age. The white bar (far left of the figure) shows results in WT mice, the solid bar (second left of the figure) shows baseline levels in untreated A53T mice at 6 weeks of age, the black bar (second right of the figure) shows results in untreated A53T mice, and the grey bar (far right of the figure) shows results in A53T mice treated with **Compound 1.**

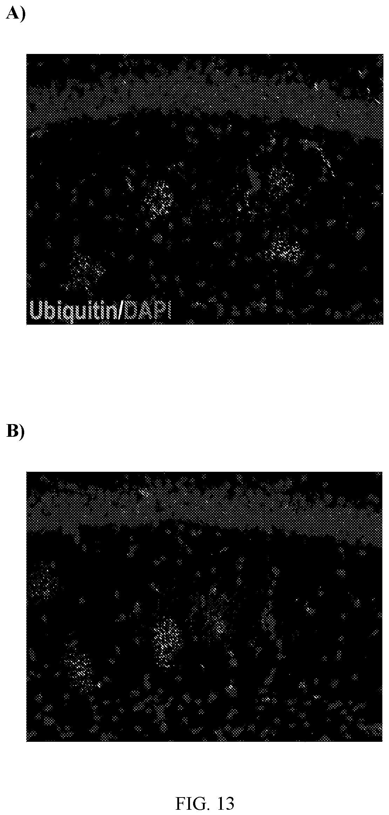

**FIG. 13** shows ubiquitin immunoreactivity (green) in the hippocampi of 8 month old A53T mice, either control (Fig. 13A) or treated with **Compound 1** (Fig. 13B). DAPI nuclear staining is shown in blue.

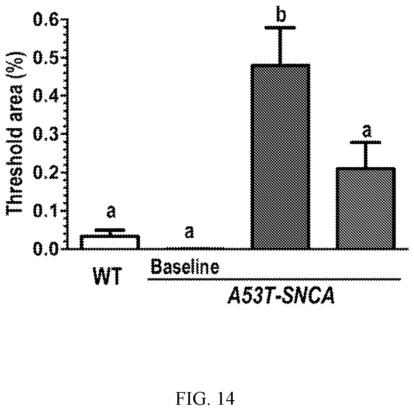

**FIG. 14** shows hippocampal quantification of protein tau aggregates in both wild-type and A53T mice, at 8 months of age. The white bar (far left of the figure) shows results in WT mice, the solid bar (second left of the figure) shows baseline levels in untreated A53T mice at 6 weeks of age, the black bar (second right of the figure) shows results in untreated A53T mice, and the grey bar (far right of the figure) shows results in A53T mice treated with **Compound 1.**

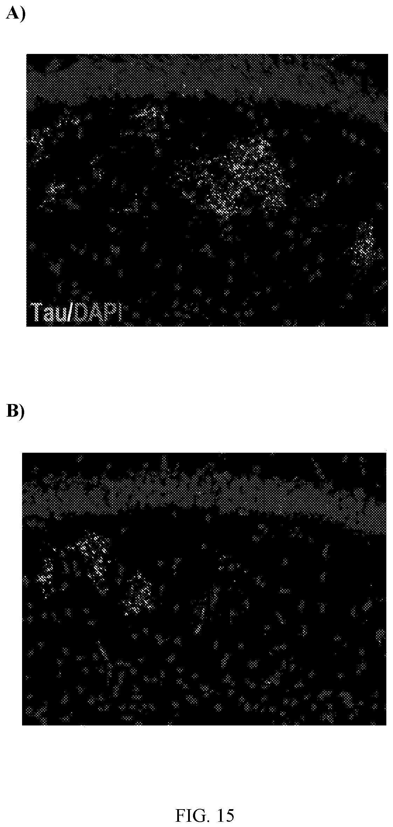

**FIG. 15** shows protein tau immunoreactivity (green) in the hippocampi of 8 month old A53T mice, either control (Fig. 15A) or treated with **Compound 1** (Fig. 15B). DAPI nuclear staining is shown in blue.

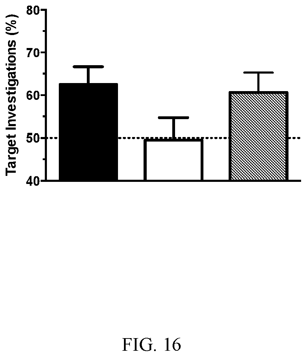

**FIG. 16** shows the results of target investigations during testing in a novel object recognition test carried out on mice, both wild-type (WT) also in the proteinopathy mouse model *Gba1*^{*D409V*/*D409V*} (training results not shown). The black bar (left of the figure) shows results in WT mice, the white bar (middle of the figure) shows results in untreated *Gba1*^{*D409V*/*D409V*} mice, and the grey bar (right of the figure) shows results in symptomatic *Gba1*^{*D409V*/*D409V*} mice treated with **Compound 1.**

### DETAILED DESCRIPTION

Although specific embodiments of the present disclosure will now be described with reference to the preparations and schemes, it should be understood that such embodiments are by way of example only and merely illustrative of but a small number of the many possible specific embodiments which can represent applications of the principles of the present disclosure. The present invention is as defined in the appended claims. Various changes and modifications, in as far as they are covered by the claims, will be obvious to those of skill in the art given the benefit of the present disclosure. Any references to methods of treatment throughout this disclosure are to be interpreted as references to the compounds, pharmaceutical compositions, or medicaments of the present invention for use in those methods.

### Definitions

Unless defined otherwise, all technical and scientific terms used herein have the same meanings as commonly understood by one of ordinary skill in the art to which this disclosure belongs. Although any methods and materials similar or equivalent to those described herein can be used in the practice or testing of the present invention, exemplary methods, devices, and materials are now described.

The practice of the present disclosure will employ, unless otherwise indicated, conventional techniques of tissue culture, immunology, molecular biology, microbiology, cell biology and recombinant DNA, which are within the skill of the art. See, *e.g.,* Michael R. Green and Joseph Sambrook, Molecular Cloning (4th ed., Cold Spring Harbor Laboratory Press 2012); the series Ausubel et al. eds. (2007) Current Protocols in Molecular Biology; the series Methods in Enzymology (Academic Press, Inc., N.Y.); MacPherson et al. (1991) PCR 1: A Practical Approach (IRL Press at Oxford University Press); MacPherson et al. (1995) PCR 2: A Practical Approach; Harlow and Lane eds. (1999) Antibodies, A Laboratory Manual; Freshney (2005) Culture of Animal Cells: A Manual of Basic Technique, 5th edition; Gait ed. (1984) Oligonucleotide Synthesis; U.S. Patent No. 4,683,195; Hames and Higgins eds. (1984) Nucleic Acid Hybridization; Anderson (1999) Nucleic Acid Hybridization; Hames and Higgins eds. (1984) Transcription and Translation; Immobilized Cells and Enzymes (IRL Press (1986)); Perbal (1984) A Practical Guide to Molecular Cloning; Miller and Calos eds. (1987) Gene Transfer Vectors for Mammalian Cells (Cold Spring Harbor Laboratory); Makrides ed. (2003) Gene Transfer and Expression in Mammalian Cells; Mayer and Walker eds. (1987) Immunochemical Methods in Cell and Molecular Biology (Academic Press, London); Herzenberg et al. eds (1996) Weir's Handbook of Experimental Immunology; Manipulating the Mouse Embryo: A Laboratory Manual, 3rd edition (Cold Spring Harbor Laboratory Press (2002)); Sohail (ed.) (2004) Gene Silencing by RNA Interference: Technology and Application (CRC Press).

All numerical designations, *e.g.* pH, temperature, time, concentration, molecular weight, *etc.,* including ranges, are approximations which are varied ( + ) or (-) by increments of 0.1 or 1.0, where appropriate. It is to be understood, although not always explicitly stated, that the reagents described herein are merely exemplary and that equivalents of such are known in the art.

As used in the specification and claims, the singular form "a", "an" and "the" include plural references unless the context clearly dictates otherwise. For example, the term "a cell" includes a plurality of cells, including mixtures thereof. Unless specifically stated or obvious from context, as used herein, the term "or" is understood to be inclusive. The term "including" is used herein to mean, and is used interchangeably with, the phrase "including but not limited to".

As used herein, the term "comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude trace contaminants from the isolation and purification method and pharmaceutically acceptable carriers, such as phosphate buffered saline, preservatives and the like. "Consisting of" shall mean excluding more than trace elements of other ingredients and substantial method steps for administering the compositions of this invention or process steps to produce a composition or achieve an intended result. Embodiments defined by each of these transition terms are within the scope of this invention. Use of the term "comprising" herein is intended to encompass "consisting essentially of" and "consisting of".

The term "proteinopathy" refers to a disease in which certain proteins become structurally abnormal and/or accumulate in a toxic manner, and thereby disrupt the function of cells, tissues and organs of the body. Often the proteins fail to fold into their normal configuration. In this misfolded state, the proteins can become toxic or can lose their normal function. Non-limiting examples of proteinopathies include Alzheimer's disease, frontotemporal dementia, progressive supranuclear palsy, dementia pugilistica, Parkinsonism, Parkinson's disease, dementia with Lewy bodies (also known as Lewy body dementia), Pick's disease, corticobasal degeneration, Argyrophilic grain disease, ganglioglioma and gangliocytoma, meningioangiomatosis, subacute sclerosing panencephalitis, lead encephalopathy, tuberous sclerosis, Hallervorden-Spatz disease, and lipofuscinosis, cerebral β-amyloid angiopathy, retinal ganglion cell degeneration in glaucoma, prion diseases, type 2 diabetes, amyotrophic lateral sclerosis (ALS), Huntington's disease and other triplet repeat disorders, Alexander disease, seipinopathies, amyloidotic neuropathy, senile systemic amyloidosis, serpinopathies, amyloidosis, inclusion body myositis/myopathy, Mallory bodies, pulmonary alveolar proteinosis, and critical illness myopathy (CIM).

As used herein, the term "chaperone" refers to a molecule, such as a small molecule, polypeptide, nucleic acid, and the like that specifically binds to a protein (which is aberrant in a proteinopathy). The chaperone may restore or enhance at least partial wild-type function and/or activity of the protein (see *e.g.* Patnaik et al. (2012) J. Med Chem. 55:5734-5748).

A "subject," "individual" or "patient" is used interchangeably herein, and refers to a vertebrate, such as a mammal. Mammals include, but are not limited to, murines, rats, rabbit, simians, bovines, ovine, porcine, canines, felines, farm animals, sport animals, pets, equines, primates, and humans. In one embodiment, the mammals include horses, dogs, and cats. In one embodiment, the mammal is a human.

"Administering" is defined herein as a means of providing an agent or a composition containing the agent to a subject in a manner that results in the agent being inside the subject's body. Such an administration can be by any route including, without limitation, oral, transdermal (*e.g.* vagina, rectum, oral mucosa), by injection (*e.g.* subcutaneous, intravenous, parenterally, intraperitoneally, into the CNS), or by inhalation (*e.g.* oral or nasal). Pharmaceutical preparations are, of course, given by forms suitable for each administration route.

"Treating" or "treatment" of a disease includes: (1) preventing the disease, *i.e.* causing the clinical symptoms of the disease not to develop in a patient that may be predisposed to the disease but does not yet experience or display symptoms of the disease; (2) inhibiting the disease, *i.e.* arresting or reducing the development of the disease or its clinical symptoms; and/or (3) relieving the disease, *i.e.* causing regression of the disease or its clinical symptoms.

The term "suffering" as it relates to the term "treatment" refers to a patient or individual who has been diagnosed with or is predisposed to the disease. A patient may also be referred to being "at risk of suffering" from a disease because of a history of disease in their family lineage or because of the presence of genetic mutations associated with the disease. A patient at risk of a disease has not yet developed all or some of the characteristic pathologies of the disease.

An "effective amount" or "therapeutically effective amount" is an amount sufficient to effect beneficial or desired results. An effective amount can be administered in one or more administrations, applications or dosages. Such delivery is dependent on a number of variables including the time period for which the individual dosage unit is to be used, the bioavailability of the therapeutic agent, the route of administration, *etc..* It is understood, however, that specific dose levels of the therapeutic agents of the present disclosure for any particular subject depends upon a variety of factors including, for example, the activity of the specific compound employed, the age, body weight, general health, sex, and diet of the subject, the time of administration, the rate of excretion, the drug combination, and the severity of the particular disorder being treated and form of administration. Treatment dosages generally may be titrated to optimize safety and efficacy. Typically, dosage-effect relationships from *in vitro* and/or *in vivo* tests initially can provide useful guidance on the proper doses for patient administration. In general, one will desire to administer an amount of the compound that is effective to achieve a serum level commensurate with the concentrations found to be effective *in vitro.* Determination of these parameters is well within the skill of the art. These considerations, as well as effective formulations and administration procedures are well known in the art and are described in standard textbooks. Consistent with this definition, as used herein, the term "therapeutically effective amount" is an amount sufficient to treat (e.g. improve) one or more symptoms associated with a proteinopathy or with aberrant/increased levels of α-synuclein, tau, or other protein aggregates *ex vivo, in vitro* or *in vivo.*

As used herein, the term "pharmaceutically acceptable excipient" encompasses any of the standard pharmaceutical excipients, including carriers such as a phosphate buffered saline solution, water, and emulsions, such as an oil/water or water/oil emulsion, and various types of wetting agents. Pharmaceutical compositions also can include stabilizers and preservatives. For examples of carriers, stabilizers and adjuvants, see Remington's Pharmaceutical Sciences (20th ed., Mack Publishing Co. 2000).

As used herein, the term "prodrug" means a pharmacological derivative of a parent drug molecule that requires biotransformation, either spontaneous or enzymatic, within the organism to release the active drug. For example, prodrugs are variations or derivatives of the quinuclidine compounds described herein that have groups cleavable under certain metabolic conditions, which when cleaved, become the quinuclidine compounds described herein, *e.g.* a compound of Formula I. Such prodrugs then are pharmaceutically active *in vivo* when they undergo solvolysis under physiological conditions or undergo enzymatic degradation. Prodrug compounds herein may be called single, double, triple, *etc.,* depending on the number of biotransformation steps required to release the active drug within the organism, and the number of functionalities present in a precursor-type form. Prodrug forms often offer advantages of solubility, tissue compatibility, or delayed release in the mammalian organism (Bundgard, Design of Prodrugs, pp. 7-9, 21-24, Elsevier, Amsterdam 1985 and Silverman, "The Organic Chemistry of Drug Design and Drug Action" pp. 352-401, Academic Press, San Diego, Calif., 1992).

Prodrugs commonly known in the art include well-known acid derivatives, such as, for example, esters prepared by reaction of acid compounds with a suitable alcohol, amides prepared by reaction of acid compounds with an amine, basic groups reacted to form an acylated base derivative, *etc..* Other prodrug derivatives may be combined with other features disclosed herein to enhance bioavailability. As such, those of skill in the art will appreciate that certain of the presently disclosed compounds having, for example, free amino or hydroxy groups can be converted into prodrugs. Prodrugs include compounds having an amino acid residue, or a polypeptide chain of two or more (e.g. two, three or four) amino acid residues which are covalently joined through peptide bonds to free amino, hydroxy or carboxylic acid groups of the presently disclosed compounds. The amino acid residues include the 20 naturally occurring amino acids commonly designated by three letter symbols and also include 4-hydroxyproline, hydroxylysine, demosine, isodemosine, 3-methylhistidine, norvalin, beta-alanine, gamma-aminobutyric acid, citrulline, homocysteine, homoserine, ornithine and methionine sulfone. Prodrugs also include compounds having a carbonate, carbamate, amide or alkyl ester moiety covalently bonded to any of the above substituents disclosed herein.

As used herein, the term "pharmaceutically acceptable salt" means a pharmaceutically acceptable acid addition salt or a pharmaceutically acceptable base addition salt of a currently disclosed compound that may be administered without any resultant substantial undesirable biological effect(s) or any resultant deleterious interaction(s) with any other component of a pharmaceutical composition in which it may be contained.

As used herein, the term "C₁₋₆-alkyl" means a saturated linear or branched free radical consisting essentially of 1 to 6 carbon atoms and a corresponding number of hydrogen atoms. Exemplary C₁₋₆-alkyl groups include methyl, ethyl, n-propyl, isopropyl, n-butyl, isobutyl, etc. Other C₁₋₆-alkyl groups will be readily apparent to those of skill in the art given the benefit of the present disclosure. The terms "C₁₋₃-alkyl", "C₁₋₄-alkyl", etc., have equivalent meanings, *i.e.* saturated linear or branched free radical consisting essentially of 1 to 3 (or 4) carbon atoms and a corresponding number of hydrogen atoms.

As used herein, the term "C₂₋₆-alkenyl" means an unsaturated linear or branched free radical consisting essentially of 2 to 6 carbon atoms and a corresponding number of hydrogen atoms, which free radical comprises at least one carbon-carbon double bond. Exemplary C₂₋₆-alkenyl groups include ethenyl, prop-1-enyl, prop-2-enyl, isopropenyl, but-1-enyl, 2-methyl-prop-1-enyl, 2-methyl-prop-2-enyl, *etc..* Other C₂₋₆-alkenyl groups will be readily apparent to those of skill in the art given the benefit of the present disclosure.

As used herein, the term "C₂₋₆-alkynyl" means an unsaturated linear or branched free radical consisting essentially of 2 to 6 carbon atoms and a corresponding number of hydrogen atoms, which free radical comprises at least one carbon-carbon triple bond. Exemplary C₂₋₆-alkynyl groups include ethynyl, prop-1-ynyl, prop-2-ynyl, but-1-ynyl, 3-methyl-but-1-ynyl, *etc..* Other C₂₋₆-alkynyl groups will be readily apparent to those of skill in the art given the benefit of the present disclosure.

As used herein, the term "C₁₋₆-alkyloxy" means a saturated linear or branched free radical consisting essentially of 1 to 6 carbon atoms (and a corresponding number of hydrogen atoms) and an oxygen atom. A C₁₋₆-alkyloxy group is attached via the oxygen atom. Exemplary C₁₋₆-alkyloxy groups include methyloxy, ethyloxy, n-propyloxy, isopropyloxy, n-butyloxy, isobutyloxy, *etc..* Other C₁₋₆-alkyloxy groups will be readily apparent to those of skill in the art given the benefit of the present disclosure. The terms "C₁₋₃-alkyloxy", "C₁₋₄-alkyloxy", and the like, have an equivalent meaning, *i.e.* a saturated linear or branched free radical consisting essentially of 1 to 3 (or 4) carbon atoms (and a corresponding number of hydrogen atoms) and an oxygen atom, wherein the group is attached via the oxygen atom.

As used herein, the term "C₂₋₆-alkenyloxy" means an unsaturated linear or branched free radical consisting essentially of 2 to 6 carbon atoms (and a corresponding number of hydrogen atoms) and an oxygen atom, which free radical comprises at least one carbon-carbon double bond. A C₂₋₆-alkenyloxy group is attached via the oxygen atom. An exemplary C₂₋₆-alkenyloxy group is ethenyloxy; others will be readily apparent to those of skill in the art given the benefit of the present disclosure.

As used herein, the term "C₂₋₆-alkynyloxy" means an unsaturated linear or branched free radical consisting essentially of 2 to 6 carbon atoms (and a corresponding number of hydrogen atoms) and an oxygen atom, which free radical comprises at least one carbon-carbon triple bond. A C₂₋₆-alkenyloxy group is attached via the oxygen atom. An exemplary C₂₋₆-alkenyloxy group is ethynyloxy; others will be readily apparent to those of skill in the art given the benefit of the present disclosure.

As used herein, the term "heteroaryl" means an aromatic free radical having 5 or 6 atoms *(i.e.* ring atoms) that form a ring, wherein 1 to 5 of the ring atoms are carbon and the remaining 1 to 5 ring atom(s) *(i.e.* hetero ring atom(s)) is selected independently from the group consisting of nitrogen, sulfur, and oxygen. Exemplary 5-membered heteroaryl groups include furyl, thienyl, thiazolyl (*e.g.* thiazol-2-yl), pyrazolyl, isothiazolyl, oxazolyl, isoxazolyl, pyrrolyl, triazolyl, imidazolyl, oxadiazolyl and thiadiazolyl. Exemplary 6-membered heteroaryl groups include pyridyl, pyrimidyl, pyrazinyl, pyridazinyl, 1,2,4-tiiazinyl, benzoxazolyl, benzothiazolyl, benzisothiazolyl, benzisoxazolyl, benzimidazolyl, *etc..* Other heteroaryl groups will be readily apparent to those of skill in the art given the benefit of the present disclosure. In general, the heteroaryl group typically is attached to the main structure via a carbon atom. However, those of skill in the art will realize that certain other atoms, e.g. hetero ring atoms, can be attached to the main structure.

As used herein, the term "aryl" means an aromatic free radical having 5 or 6 atoms (*i.e.* ring atoms) that form a ring, wherein all of the ring atoms are carbon. An exemplary aryl group is benzyl.

As used herein, the term "aliphatic" means a non-aromatic compound containing carbon and hydrogen atoms, *e.g.* containing 1 to 9 carbon atoms. Aliphatic compounds may be straight-chained or branched, may contain one or more ring structures, and may contain one or more carbon-carbon double bonds (provided that the compound does not contain an unsaturated ring structure having aromatic character). Examples of aliphatic compounds include ethane, propylene, cyclobutane, cyclohexadiene, *etc..*

As used herein, the terms "halo" and "halogen" mean fluorine, chlorine, bromine, or iodine. These terms are used interchangeably and may refer to a halogen free radical group or to a halogen atom as such. Those of skill in the art will readily be able to ascertain the identification of which in view of the context in which this term is used in the present disclosure.

As used herein, the term "cyano" means a free radical having a carbon atom linked to a nitrogen atom via a triple bond. The cyano radical is attached via its carbon atom.

As used herein, the term "nitro" means an NO₂ radical which is attached via its nitrogen atom.

As used herein, the terms "hydroxy" and "hydroxyl" mean an OH radical which is attached via its oxygen atom. The term "thio" means an SH radical which is attached via its sulphur atom.

As used herein, the term "amino" means a free radical having a nitrogen atom and 1 or 2 hydrogen atoms. As such, the term "amino" generally refers to primary and secondary amines. In that regard, as used herein and in the appended claims, a tertiary amine is represented by the general formula RR'N-, wherein R and R' are carbon radicals that may or may not be identical. Nevertheless, the term "amino" generally may be used herein to describe a primary, secondary, or tertiary amine, and those of skill in the art will readily be able to ascertain the identification of which in view of the context in which this term is used in the present disclosure.

As used herein, the term and "oxo" means an oxygen radical which is attached via a double bond. Where an atom bonded to this oxygen is a carbon atom, the bond is a carbon-oxygen double bond which may be denoted as -(C=O)- and which may be referred to as a ketone.

The recitation of a listing of chemical groups in any definition of a variable herein includes definitions of that variable as any single group or combination of listed groups. The recitation of an embodiment for a variable or aspect herein includes that embodiment as any single embodiment or in combination with any other embodiments or portions thereof.

Any compositions or methods provided herein can be combined with one or more of any of the other compositions and methods provided herein.

The following abbreviations are used herein:
- A53T: Transgenic mice expressing human alpha-synuclein with A53T mutation
- ALS: Amyotrophic lateral sclerosis
- AMTS: Abbreviated mental test score
- ANOVA: Analysis of variance
- br: Broad signal
- CDI: Carbonyldiimidazole
- CIM: Critical Illness Myopathy
- CNS: Central Nervous System
- CS: Conditioned stimulus
- d: Doublet
- DAPI: 4',6-diamidino-2-phenylindole
- dd: Doublet of doublets
- DME: Dimethoxyethane
- DMSO-d6: Dimethyl sulfoxide-d6
- DMF: Dimethylformamide
- DNA: Deoxyribonucleic acid
- DTBZ: Carbon-11 dihydrotetrabenazine
- EDTA: Ethylenediaminetetraacetic acid
- ELISA: Enzyme-linked Immunosorbent Assay
- Et₂O: Diethyl ether
- EtMgBr: Ethylmagnesium bromide
- EtOAc: Ethyl acetate
- FC: Fear conditioning (test)
- *GBA1*: Glucocerebrosidase 1 gene
- HPLC: High pressure/performance liquid chromatography
- HSA: Human serum albumin
- IQCODE: Informant questionnaire on cognitive decline in the elderly
- IPA: Isopropyl alcohol
- ITI: Inter-trial interval
- J: Coupling constant
- LCMS: Liquid chromatography mass spectrometry
- m: Multiplet
- *MAPT*: Microtubule-associated protein tau gene
- MAO-B: Monoamine oxidase B
- MMSE: Mini mental state examination
- NOR: Novel object recognition (test)
- PET: Positron emission tomography
- PIB: Carbon-11 Pittsburgh Compound B
- ppm: Parts per million
- pTau: Phosphorylated Tau protein
- rHA: Recombinant human albumin
- s: Singlet
- *SNCA*: α-synuclein gene
- SPECT: Single-photon emission computed tomography
- SEM: Standard error of mean
- TBME: Tert-Butyl Methyl Ether
- THF: Tetrahydrofuran
- Tris: Tris(hydroxymethyl)aminomethane
- TWEEN20: Polysorbate 20
- TWEEN80: Polysorbate 80
- WT: Wild type
- UPLCMS: Ultra performance liquid chromatography mass spectrometry
- US: Unconditioned stimulus
- US-CS: Unconditioned stimulus - Conditioned stimulus

### Compounds

The present invention relates to quinuclidine compounds for use in therapeutic methods relating to proteinopathies as defined in the appended claims. In one aspect, the quinuclidine compound is a compound of formula (I), 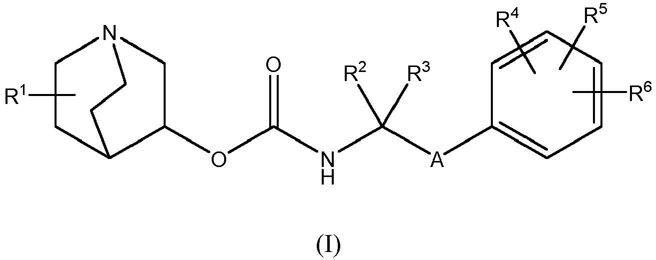 or a pharmaceutically acceptable salt thereof, wherein:
R¹ is hydrogen;
   a halogen, or a cyano, nitro, hydroxy, thio, or amino group; or a C₁₋₆-alkyl, C₂₋₆-alkenyl, C₂₋₆-alkynyl, C₁₋₆-alkyloxy, C₂₋₆-alkenyloxy or C₂₋₆-alkynyloxy group, optionally substituted by one or more (e.g. 1, 2 or 3) groups independently selected from a halogen, and a cyano, nitro, hydroxy, thio, or amino group;
R² and R³ are each independently selected from a C₁₋₃-alkyl group, optionally substituted by one or more (*e.g.* 1, 2 or 3) halogens; or
   R² and R³ together form a cyclopropyl or cyclobutyl group, optionally substituted by one or more (*e.g.* 1 or 2) halogens;
R⁴, R⁵ and R⁶ are each independently selected from hydrogen; a halogen; a nitro,
   hydroxy, thio, or amino group; and a C₁₋₆-alkyl or C₁₋₆-alkyloxy group, optionally substituted by one or more (*e.g.* 1, 2 or 3) groups selected from a halogen; a hydroxy or cyano group; and a C₁₋₆-alkyloxy group; and
A is a 5- or 6-membered aryl or heteroaryl group.

In one embodiment, R¹ is hydrogen; a halogen; or a C₁₋₄-alkyl or C₁₋₄-alkyloxy group, optionally substituted by one or two groups selected independently from a halogen; and a cyano, nitro, hydroxy, thio or amino group. In another embodiment, R¹ is hydrogen; fluorine; or a methyl or ethyl group optionally substituted by a halogen, or a hydroxy, thio or amino group. In a further embodiment, R¹ is hydrogen; or a methyl group optionally substituted by one or more (*e.g.* 1, 2 or 3) halogens. In a yet further embodiment, R¹ is hydrogen. In one embodiment, R¹ is not attached to the nitrogen atom of the quinuclidine moiety.

In one embodiment, R² and R³ are each independently selected from C₁₋₃-alkyl groups, optionally substituted with one or more halogens. In another embodiment, R² and R³ are each independently selected from methyl and ethyl groups, optionally substituted with one or more fluorine atoms. In a further embodiment, R² and R³ are each methyl, optionally substituted with one to three fluorine atoms. In a yet further embodiment, either R² and R³ are both methyl groups, or R² and R³ together form a cyclopropyl group. In a still further embodiment, R² and R³ are both methyl groups.

In one embodiment, R⁶ is hydrogen. In another embodiment, R⁵ and R⁶ are both hydrogen. In another embodiment at least one of R⁴, R⁵ and R⁶ is not hydrogen. In a further embodiment, R⁴ is selected from a halogen; and a C₁₋₃-alkyl or C₁₋₃-alkyloxy group, optionally substituted by one or more groups selected from a halogen; and a cyano or C₁₋₃-alkyloxy group. In another embodiment, R⁴ is selected from a halogen; and a C₁₋₃-alkyl or C₁₋₃-alkyloxy group, optionally substituted by one or more groups selected from a halogen; and a C₁₋₃-alkyloxy group. In a yet further embodiment, R⁴ is selected from fluorine; and a C₁₋₃-alkyloxy group, optionally substituted by one or more groups selected from a halogen; and a cyano or C₁₋₃-alkyloxy group. In a still further embodiment, R⁴ is selected from fluorine; and a C₁₋₃-alkyloxy group, optionally substituted by one or more groups selected from a halogen; and a cyano or C₁₋₃-alkyloxy group; and R⁵ and R⁶ are both hydrogen. For example, where R⁵ and R⁶ are both hydrogen, R⁴ may be fluorine or a 2-methoxyethoxy group, e.g. fluorine.

Where all of R⁴, R⁵ and R⁶ are other than hydrogen, these three groups may be attached to the benzene ring, for example, at positions 2, 4 and 6 (relative to the group A being attached to position 1). Where only one of R⁴, R⁵ and R⁶ is hydrogen, the other two groups may be attached to the benzene ring, for example, at positions 2 and 3, positions 3 and 4, or positions 3 and 5, *e.g.* at positions 3 and 5 (relative to the group A being attached to position 1). Where two of R⁴, R⁵ and R⁶ are hydrogen, the other group may be attached to the benzene ring at position 2, 3 or 4, *e.g.* at position 4 (*i.e.* at the position *para* to the group A). In one embodiment, R⁴ is in a position on the benzene ring *para* to the group A.

In one embodiment, A is a 6-membered aryl group or a 5-membered heteroaryl group. Non-limiting examples of 6-membered aryl groups and 5-membered heteroaryl groups includephenyl, furyl, thienyl, thiazolyl, pyrazolyl, isothiazolyl, oxazolyl, isoxazolyl, pyrrolyl, triazolyl, imidazolyl, oxadiazolyl and thiadiazolyl. In one embodiment, the 6-membered aryl group or 5-membered heteroaryl group is selected fromphenyl, thienyl, thiazolyl, pyrrolyl and imidazolyl. In another embodiment, the 6-membered aryl group or 5-membered heteroaryl group is selected from phenyl and thiazolyl.

In one embodiment, A is phenyl, optionally substituted with 1, 2 or 3 groups independently selected from a halogen; and a hydroxy, thio, amino, nitro, oxo or methyl group. In another embodiment, A isphenyl, optionally substituted with 1 or 2 halogens. In a further embodiment, A isphenyl, optionally substituted with a halogen, e.g. fluorine. In a yet further embodiment, A is an unsubstituted phenyl group.

Where A is 6-membered aryl or heteroaryl, the attached groups -C(R²R³)- and -(C₆H₂R⁴R⁵R⁶) may be in a 1,2- or 1,3- or 1,4- relationship, *i.e.* ortho, meta or para to each other. In one embodiment, the attached groups -C(R²R³)- and -(C₆H₂R⁴R⁵R⁶) are in a 1,3- relationship. In another embodiment, the attached groups are in a 1,4- relationship.

In one embodiment, A is a 5-membered heteroaryl group which contains 1, 2 or 3 heteroatoms selected from N, O and S. In another embodiment, A is a 5-membered heteroaryl group which contains 1 or 2 heteroatoms selected from N and S. In a further embodiment, A is a 5-membered heteroaryl group which contains 2 heteroatoms selected from N and S. In a yet further embodiment, A is a 5-membered heteroaryl group which contains 2 heteroatoms wherein one heteroatom is N and the other heteroatom is S. In a still further embodiment, A is a thiazolyl group.

Where A is a 5-membered heteroaryl group, at least one of the attached groups -C(R²R³)- and -(C_{S}H₂R⁴R⁵R⁶) may be bonded directly to a carbon atom of the heteroaryl group. In one embodiment, both of the attached groups -C(R²R³)- and -(C₆H₂R⁴R⁵R⁶) are bonded directly to a carbon atom of the heteroaryl group. In one embodiment, the attached groups -C(R²R³)- and -(C₆H₂R⁴R⁵R⁶) are in a 1,3- relationship to each other, *e.g.* they are bonded directly to carbon atoms of the heteroaryl group which are separated by a single intervening atom, *e.g.* heteroatom. In the embodiment where A is a thiazolyl group, the attached groups -C(R²R³)- and -(C₆H₂R⁴R⁵R⁶) may be bonded directly at the 4- and 2-positions, respectively.

Thus, in one embodiment the quinuclidine compound is a compound of formula (II) 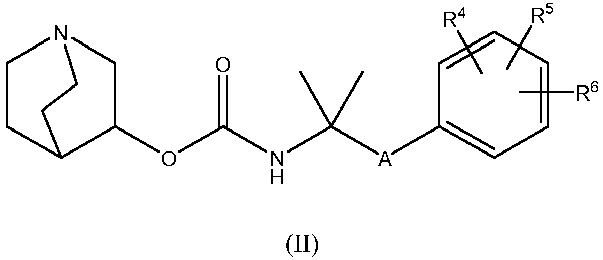 or a pharmaceutically acceptable salt thereof, wherein R⁴, R⁵, R⁶ and A are as defined herein.

In another embodiment, the quinuclidine compound is a compound of formula (III) 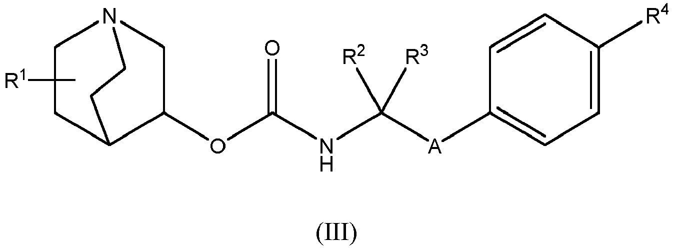 or a pharmaceutically acceptable salt thereof, wherein R¹ to R⁴, and A are as defined herein.

In another embodiment, the quinuclidine compound is a compound of formula (IV) 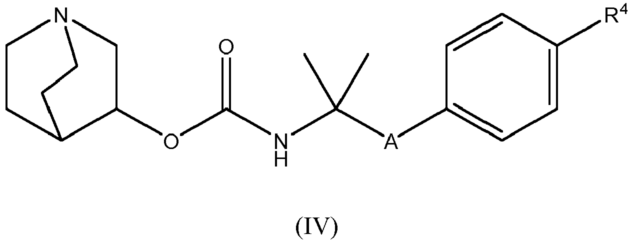 or a pharmaceutically acceptable salt thereof, wherein R⁴ and A are as defined herein.

In one embodiment, R⁴ is a halogen, *e.g.* fluorine. Accordingly, the quinuclidine compound may be a compound of formula (V) 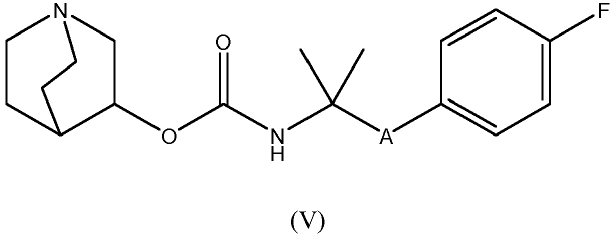 or a pharmaceutically acceptable salt thereof, wherein A is as defined herein.

In another embodiment, the quinuclidine compound is a compound of formula (VI) 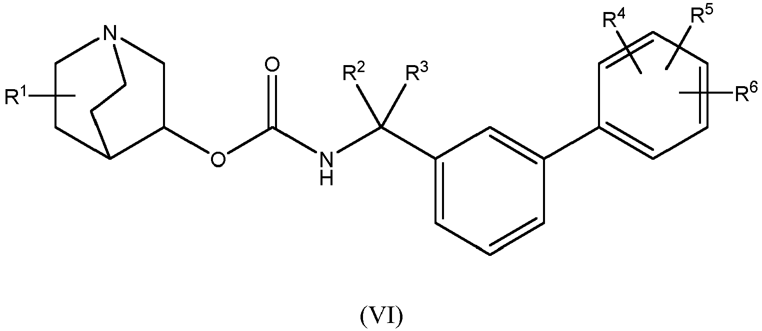 or a pharmaceutically acceptable salt thereof, wherein R¹ to R⁶ are as defined herein.

In another embodiment, the quinuclidine compound is a compound of formula (VII) 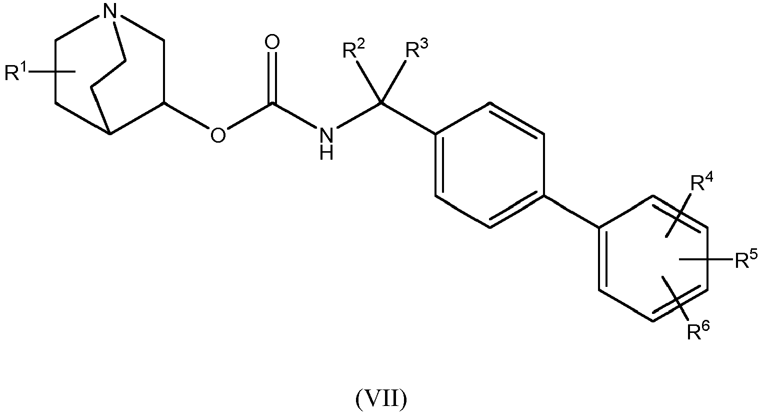 or a pharmaceutically acceptable salt thereof, wherein R¹ to R⁶ are as defined herein.

In another embodiment, the quinuclidine compound is a compound of formula (VIII) 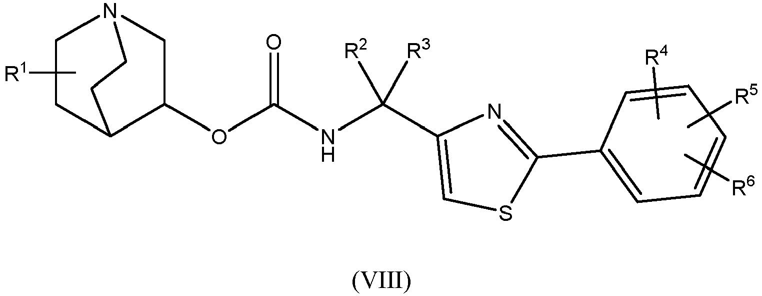 or a pharmaceutically acceptable salt thereof, wherein R¹ to R⁶ are as defined herein.

In another embodiment, the quinuclidine compound is a compound of formula (IX) 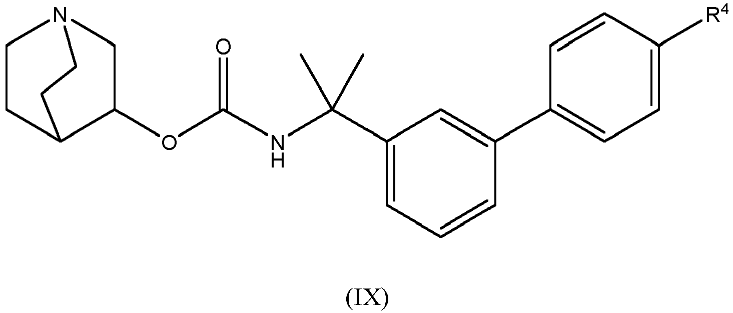 or a pharmaceutically acceptable salt thereof, wherein R⁴ is as defined herein.

In one embodiment, R⁴ is a halogen, e.g. fluorine. Accordingly, the quinuclidine compound may be a compound of formula (X) 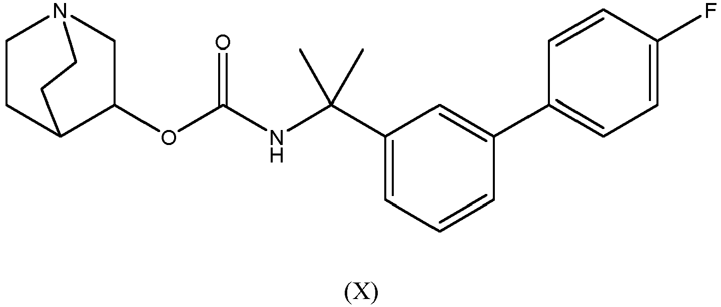 or a pharmaceutically acceptable salt thereof.

In another embodiment, the quinuclidine compound is a compound of formula (XI) 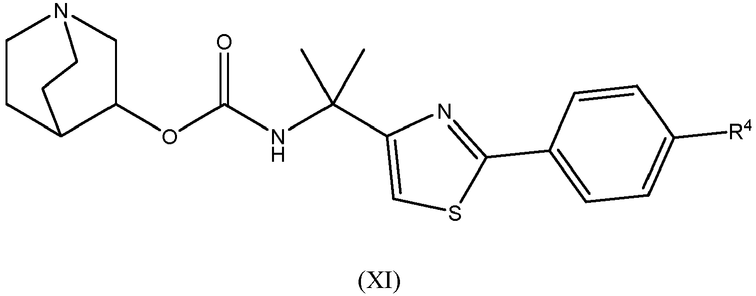 or a pharmaceutically acceptable salt thereof, wherein R⁴ is as defined herein.

In one embodiment, R⁴ is a halogen, e.g. fluorine. Accordingly, the quinuclidine compound may be a compound of formula (XII) 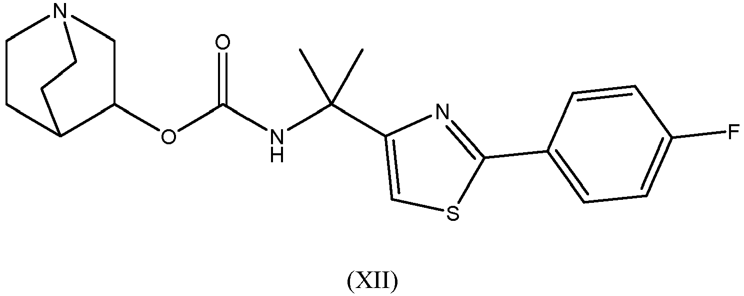 or a pharmaceutically acceptable salt thereof.

In one embodiment, the quinuclidine compound is selected from the group consisting of **Compound** 1 to **Compound 23:**

| **Compound No.** | **Compound** |
|---|---|
| **1** | Quinuclidin-3-yl (2-(4'-fluoro-[1,1'-biphenyl]-3-yl)propan-2-yl)carbamate |
| **2** | (*S*)-quinuclidin-3-yl (2-(2-(4-fluorophenyl)thiazol-4-yl)propan-2-yl)carbamate |
| **3** | (*S*)-quinuclidin-3-yl (2-(4'-(2-methoxyethoxy)-[1, 1'-biphenyl]-4-yl)propan-2-yl)carbamate |
| **4** | 1-azabicyclo[2.2.2]oct-3-yl [2-(biphenyl-3-yl)propan-2-yl]carbamate |
| **5** | (*S*)-quinuclidin-3-yl 2-(biphenyl-4-yl)propan-2-ylcarbamate |
| **6** | Quinuclidin-3-yl 1-(biphenyl-4-yl)cyclopropylcarbamate |
| **7** | (*S*)-quinuclidin-3-yl 1 -(4'-fluorobiphenyl-4-yl)cyclopropylcarbamate |
| **8** | (*S*)-1-azabicyclo[2.2.2]oct-3-yl [1-(2',4'-difluorobiphenyl-4-yl)cyclopropyl]carbamate |
| **9** | 1-azabicyclo[2.2.2]oct-3-yl [1-(4'-methoxybiphenyl-4-yl)cyclopropyl]carbamate |
| **10** | Quinuclidin-3-yl 2-(5-(4-fluorophenyl)thiophen-3-yl)propan-2-ylcarbamate |
| **11** | (*S*)-quinuclidin-3-yl 2-(3-(4-fluorophenyl)isothiazol-5-yl)propan-2-ylcarbamate |
| **12** | (*S*)-quinuclidin-3-yl 2-(4-(4-fluorophenyl)thiazol-2-yl)propan-2-ylcarbamate |
| **13** | Quinuclidin-3-yl (2-(4'-(2-methoxyethoxy)-[1,1'-biphenyl]-4-yl)propan-2-yl)carbamate |
| **14** | (*S*)-quinuclidin-3-yl (2-(3'-(2-methoxyethoxy)-[1,1'-biphenyl]-4-yl)propan-2-yl)carbamate |
| **15** | Quinuclidin-3-yl (2-(4'-(2-methoxyethoxy)-[1,1'-biphenyl]-3-yl)propan-2-yl)carbamate |
| **16** | Quinuclidin-3-yl (2-(4'-(3-methoxypropoxy)-[1,1'-biphenyl]-4-yl)propan-2-yl)carbamate |
| **17** | Quinuclidin-3-yl (2-(4'-(hydroxymethyl)-[1,1'-biphenyl]-4-yl)propan-2-yl)carbamate |
| **18** | Quinuclidin-3-yl (2-(4'-(2-hydroxyethyl)-[1,1'-biphenyl]-4-yl)propan-2-yl)carbamate |
| **19** | Quinuclidin-3-yl (2-(2-(4-(3-methoxypropoxy)phenyl)thiazol-4-yl)propan-2-yl)carbamate |
| **20** | Quinuclidin-3-yl (2-(2-(4-(2-methoxyethoxy)phenyl)thiazol-4-yl)propan-2-yl)carbamate |
| **21** | Quinuclidin-3-yl 2-(5-(4-(2-methoxyethoxy)phenyl)pyridin-2-yl)propan-2-ylcarbamate |
| **22** | Quinuclidin-3-yl (2-(4'-(3-cyanopropoxy)-[1,1'-biphenyl]-4-yl)propan-2-yl)carbamate |
| **23** | Quinuclidin-3-yl (2-(4'-(cyanomethoxy)-[1,1'-biphenyl]-4-yl)propan-2-yl)carbamate |

and the pharmaceutically acceptable salts thereof.

In one embodiment, the quinuclidine compound is selected from **Compound 1, Compound 2** and **Compound 3,** and the pharmaceutically acceptable salts thereof. In another embodiment, the quinuclidine compound is selected from **Compound 1** and **Compound 3,** and the pharmaceutically acceptable salts thereof. In another embodiment, the quinuclidine compound is **Compound 1,** or a pharmaceutically acceptable salt thereof. In another embodiment, the quinuclidine compound is **Compound 2,** or a pharmaceutically acceptable salt thereof. In another embodiment, the quinuclidine compound is **Compound 3,** or a pharmaceutically acceptable salt thereof.

In another embodiment, the quinuclidine compound is selected from **Compound 1, Compound 2** and **Compound 3.** In one embodiment, the quinuclidine compound is **Compound 1.** In another embodiment, the quinuclidine compound is **Compound 2.** In another embodiment, the quinuclidine compound is **Compound 3.**

### Salts

Presently disclosed compounds that are basic in nature are generally capable of forming a wide variety of different salts with various inorganic and/or organic acids. Although such salts are generally pharmaceutically acceptable for administration to animals and humans, it is often desirable in practice to initially isolate a compound from the reaction mixture as a pharmaceutically unacceptable salt and then simply convert the latter back to the free base compound by treatment with an alkaline reagent, and subsequently convert the free base to a pharmaceutically acceptable acid addition salt. The acid addition salts of the base compounds can be readily prepared using conventional techniques, *e.g.* by treating the base compound with a substantially equivalent amount of the chosen mineral or organic acid in an aqueous solvent medium or in a suitable organic solvent such as, for example, methanol or ethanol. Upon careful evaporation of the solvent, the desired solid salt is obtained. Presently disclosed compounds that are positively charged, *e.g.* containing a quaternary ammonium, may also form salts with the anionic component of various inorganic and/or organic acids.

Acids which can be used to prepare pharmaceutically acceptable salts of quinuclidine compounds are those which can form non-toxic acid addition salts, *e.g.* salts containing pharmacologically acceptable anions, such as chloride, bromide, iodide, nitrate, sulfate or bisulfate, phosphate or acid phosphate, acetate, lactate, citrate or acid citrate, tartrate or bitartrate, succinate, malate, maleate, fumarate, gluconate, saccharate, benzoate, methanesulfonate and pamoate [*i.e.* 1,1'-methylene-bis-(2-hydroxy-3-naphthoate)] salts.

Presently disclosed compounds that are acidic in nature, *e.g.* compounds containing a tetrazole moiety, are generally capable of forming a wide variety of different salts with various inorganic and/or organic bases. Although such salts are generally pharmaceutically acceptable for administration to animals and humans, it is often desirable in practice to initially isolate a compound from the reaction mixture as a pharmaceutically unacceptable salt and then simply convert the latter back to the free acid compound by treatment with an acidic reagent, and subsequently convert the free acid to a pharmaceutically acceptable base addition salt. These base addition salts can be readily prepared using conventional techniques, *e.g.* by treating the corresponding acidic compounds with an aqueous solution containing the desired pharmacologically acceptable cations, and then evaporating the resulting solution to dryness, *e.g.* under reduced pressure. Alternatively, they also can be prepared by mixing lower alkanolic solutions of the acidic compounds and the desired alkali metal alkoxide together, and then evaporating the resulting solution to dryness in the same manner as before. In either case, stoichiometric quantities of reagents may be employed in order to ensure completeness of reaction and maximum product yields of the desired solid salt.

Bases which can be used to prepare the pharmaceutically acceptable base addition salts of quinuclidine compounds are those which can form non-toxic base addition salts, *e.g.* salts containing pharmacologically acceptable cations, such as, alkali metal cations (*e.g.* potassium and sodium), alkaline earth metal cations (*e.g.* calcium and magnesium), ammonium or other water-soluble amine addition salts such as *N*-methylglucamine (meglumine), lower alkanolammonium, and other such bases of organic amines.

In one embodiment, the pharmaceutically acceptable salt is a succinate salt. In another embodiment, the pharmaceutically acceptable salt is a 2-hydroxysuccinate salt, *e.g.* an (S)-2-hydroxysuccinate salt. In another embodiment, the pharmaceutically acceptable salt is a hydrochloride salt *(i.e.* a salt with HCl). In another embodiment, the pharmaceutically acceptable salt is a malate salt.

### Prodrugs

The pharmaceutically acceptable prodrugs disclosed herein are derivatives of quinuclidine compounds which can be converted *in vivo* into the quinuclidine compounds described herein. The prodrugs, which may themselves have some activity, become pharmaceutically active *in vivo* when they undergo, for example, solvolysis under physiological conditions or enzymatic degradation. Methods for preparing prodrugs of compounds as described herein would be apparent to one of skill in the art based on the present disclosure.

In one embodiment, the carbamate moiety of the quinuclidine compound is modified. For example, the carbamate moiety of the quinuclidine compound may be modified by the addition of water and/or one or two aliphatic alcohols. In this case, the carbon-oxygen double bond of the carbamate moiety adopts what could be considered a hemiacetal or acetal functionality. In one embodiment, the carbamate moiety of the quinuclidine compound may be modified by the addition of an aliphatic diol such as 1,2-ethanediol.

In one embodiment, one or more of the hydroxy, thio or amino groups on the quinuclidine compound are modified. For example, one or more of the hydroxy, thio and/or amino groups on the quinuclidine compound may be modified to form acid derivatives, e.g. esters, thioesters (or thiolesters) and/or amides. The acid derivatives can be formed, for example, by reacting a quinuclidine compound which comprises one or more hydroxy, thio or amino groups with an acetylating agent. Examples of acetylating agents include anhydrides such as acetic anhydride, acid chlorides such as benzyl chloride, and dicarbonates such as di-*tert*-butyl dicarbonate.

### Stereochemistry

Stereoisomers (*e.g.* cis and trans isomers) and all optical isomers of a presently disclosed compound (*e.g. R-* and *S*- enantiomers), as well as racemic, diastereomeric and other mixtures of such isomers are within the scope of the present disclosure.

In one embodiment, the quinuclidin-3-yl group of a quinuclidine compound as defined herein has the *R*- configuration. Accordingly, the quinuclidine compound may be selected from the group consisting of compounds of formulae (Ia) to (XIIa): 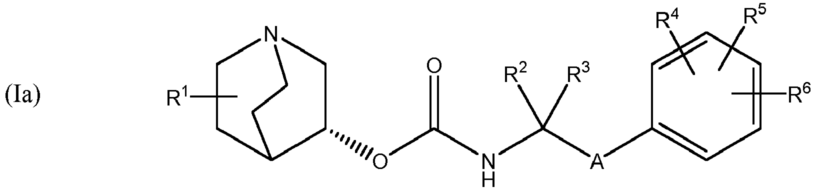 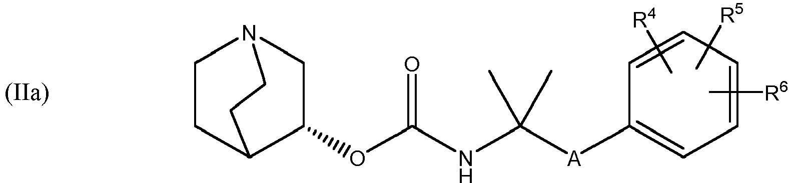 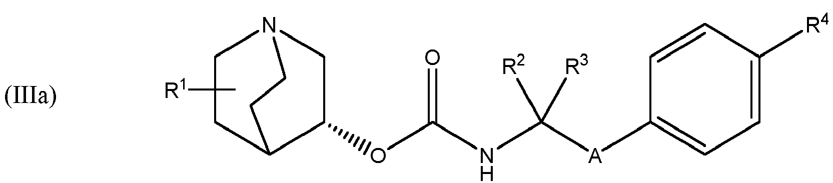 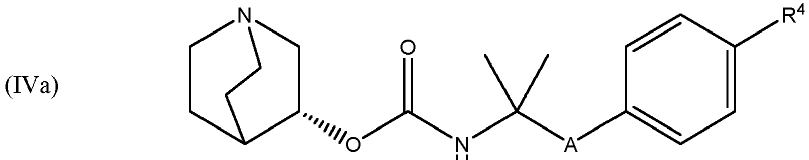 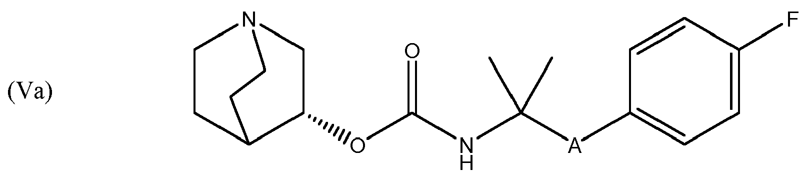 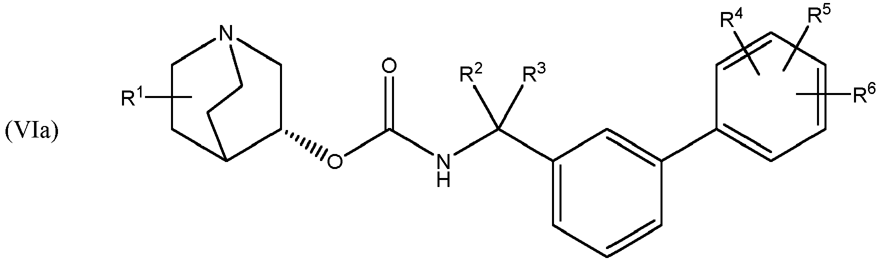 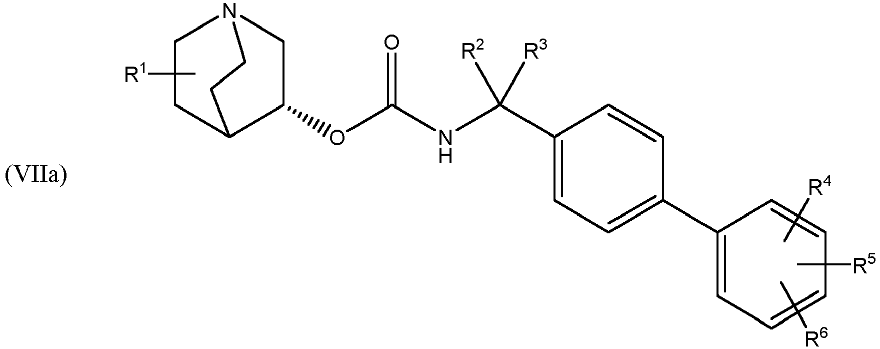 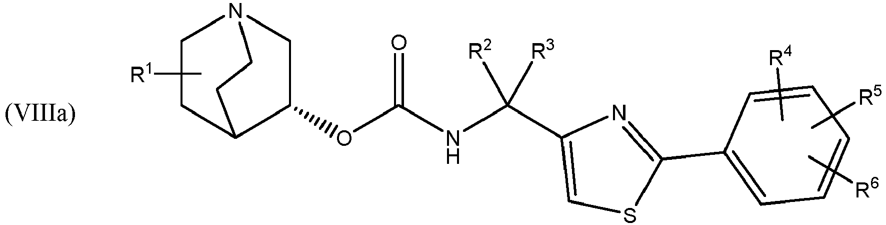 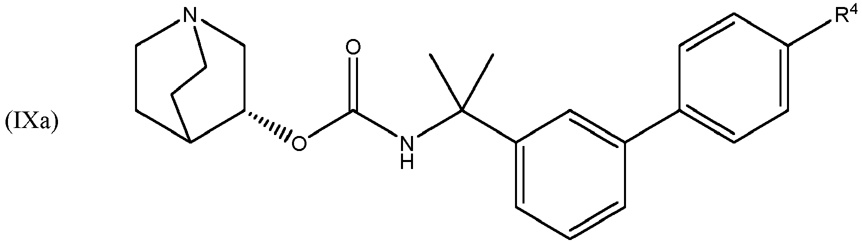 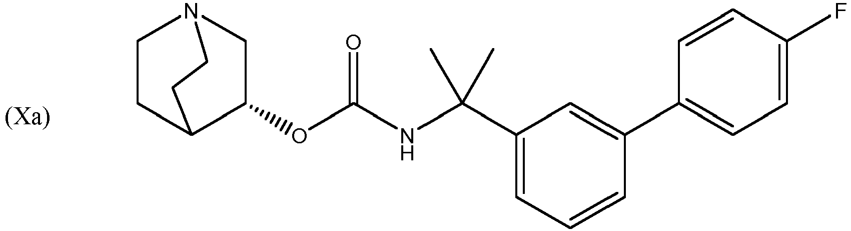 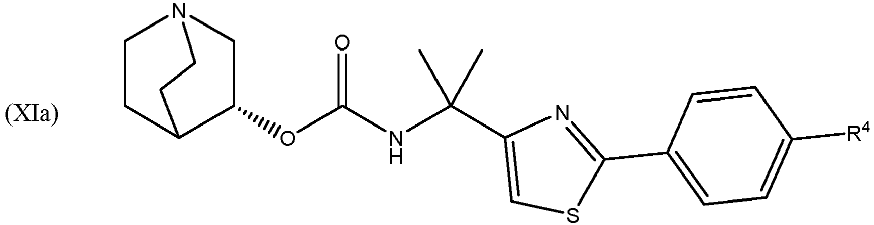 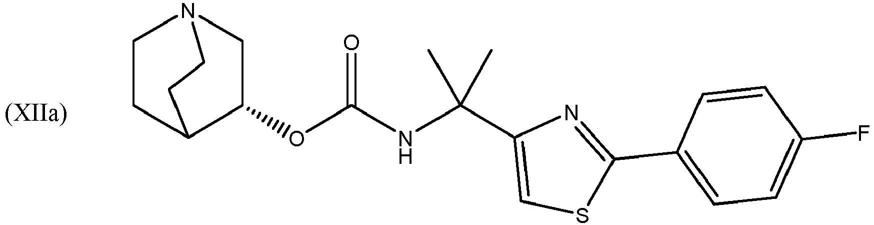 and the pharmaceutically acceptable salts thereof.

In another embodiment, the quinuclidin-3-yl group of the quinuclidine compound as defined herein has the *S*- configuration. Accordingly, the quinuclidine compound may be selected from the group consisting of compounds of formulae (Ib) to (XIIb): 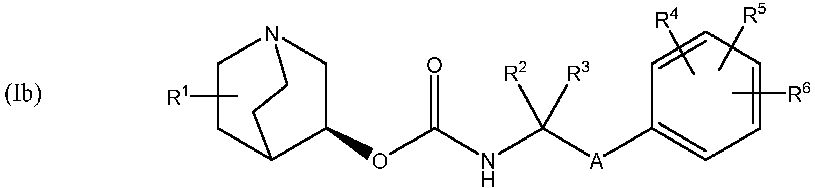 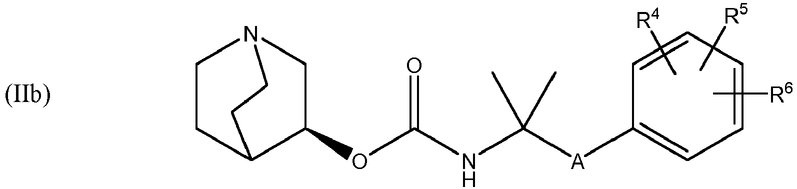 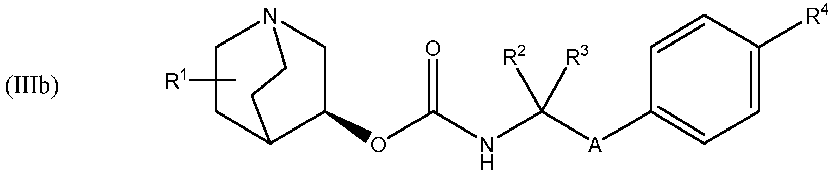 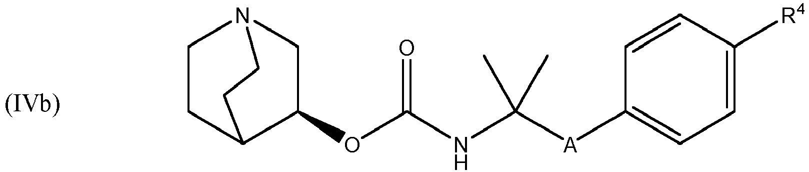 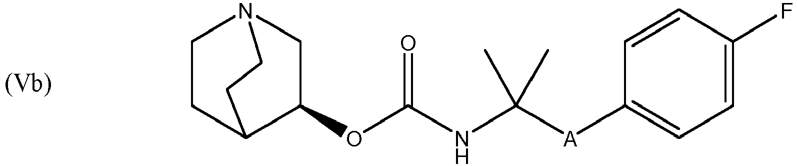 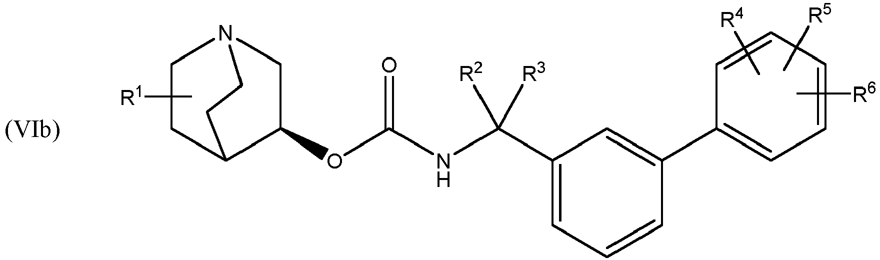 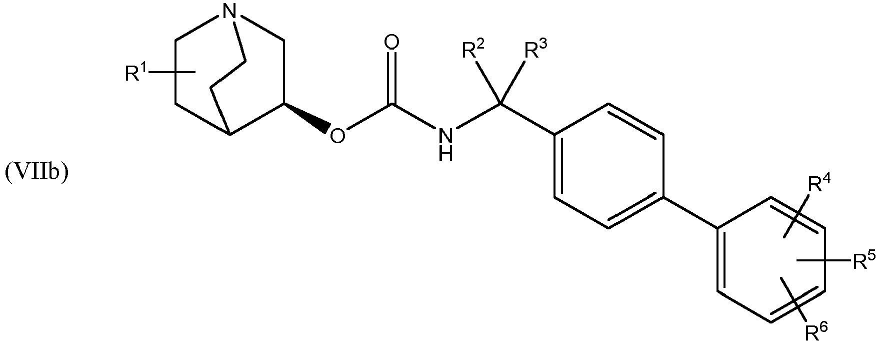 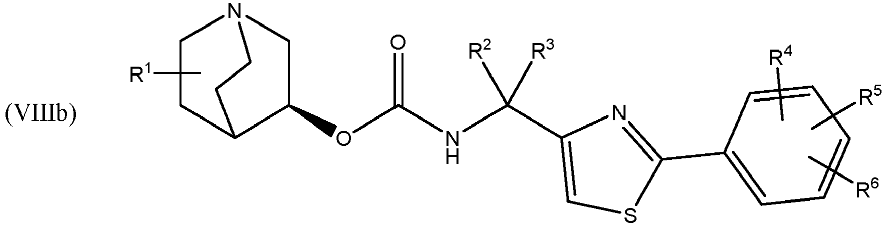 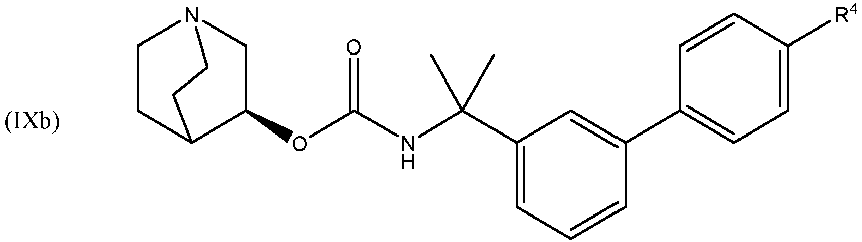 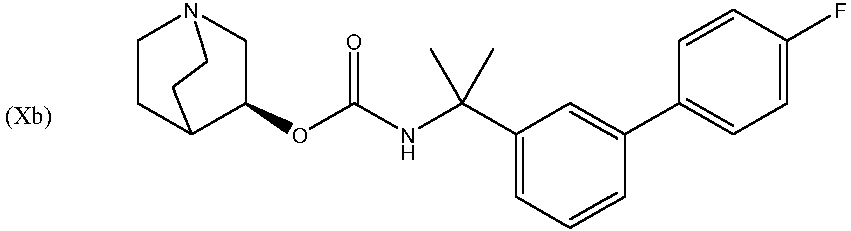 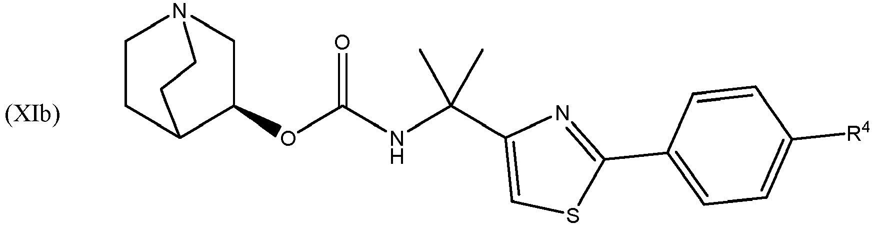 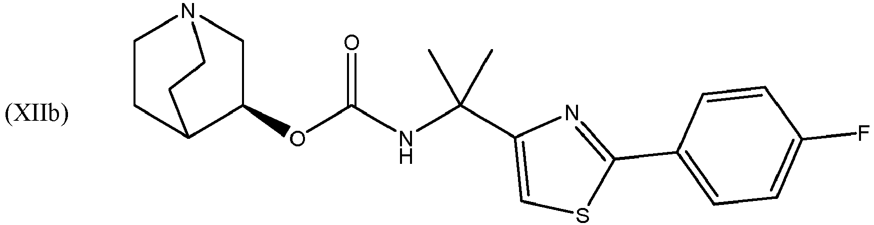 and the pharmaceutically acceptable salts thereof.

In one embodiment the quinuclidine compound is a compound of formula (Xb) or a pharmaceutically acceptable salt thereof. In another embodiment the quinuclidine compound is a compound of formula (XIIb) or a pharmaceutically acceptable salt thereof.

In one embodiment, the quinuclidin-3-yl group of the quinuclidine compound as defined herein exists in a mixture of isomers having the *R*- and *S*- configurations. For example, the quinuclidine compound may be a mixture of compounds selected from the group consisting of compounds of formulae (Ia) and (Ib), (IIa) and (IIb), (IIIa) and (IIIb), (IVa) and (TVb), (Va) and (Vb), (VIa) and (VIb), (VIIa) and (VIIb), (VIIIa) and (VIIIb), (IXa) and (IXb), (Xa) and (Xb), (XIa) and (XIb), and (XIIa) and (XIIb), and the pharmaceutically acceptable salts thereof. In one embodiment the quinuclidine compound is present as a racemic mixture, *e.g.* the *R-* and *S-* isomers of the quinuclidin-3-yl group are present in about equal amounts. In another embodiment the quinuclidine compound is present as a mixture of isomers having the *R-* and *S-* configurations, wherein the *R-* and *S-*isomers are present in different amounts. In one embodiment the S- isomer is present in an enantiomeric excess of at least about 5%, 10%, 25%, 40%, 70%, 80%, 90%, 95%, 97%, 98% or 99%, e.g. about 100%. In another embodiment, the R- isomer is present in an enantiomeric excess of at least about 5%, 10%, 25%, 40%, 70%, 80%, 90%, 95%, 97%, 98% or 99%, *e.g.* about 100%.

Methods for preparing enantioenriched and/or enantiopure quinuclidine compounds would be apparent to the person of skill in the art based on the present disclosure.

The compounds presently disclosed can exist in several tautomeric forms, including the enol and imine form, and the keto and enamine form and geometric isomers and mixtures thereof. Tautomers exist as mixtures of a tautomeric set in solution. In solid form, usually one tautomer predominates. Even though one tautomer may be described, all tautomers are within the scope of the present disclosure.

Atropisomers are also within the scope of the present disclosure. Atropisomers refer to compounds that can be separated into rotationally restricted isomers.

### Other forms

Pharmaceutically acceptable hydrates, solvates, polymorphs, *etc.,* of the quinuclidine compounds described herein are within the scope of the present disclosure. Quinuclidine compounds as described herein may be in an amorphous form and/or in one or more crystalline forms.

Isotopically-labeled compounds are also within the scope of the present disclosure. As used herein, an "isotopically-labeled compound" refers to a presently disclosed compound including pharmaceutical salts and prodrugs thereof, each as described herein, in which one or more atoms are replaced by an atom having an atomic mass or mass number different from the atomic mass or mass number usually found in nature. Examples of isotopes that can be incorporated into compounds presently disclosed include isotopes of hydrogen, carbon, nitrogen, oxygen, phosphorous, fluorine and chlorine, such as ²H, ³H, ¹³C, ¹⁴C, ¹⁵N, ¹⁸O, ¹⁷O, ³¹P, ³²P, ³⁵S, ¹⁸F, and ³⁶Cl, respectively.

### Medical indications

The quinuclidine compounds, and pharmaceutical compositions containing them, described herein are useful in therapy, in particular in a method of preventing, reducing or reversing loss of neural function in a subject diagnosed as having, or at risk of developing, a proteinopathy, wherein the loss of neural function comprises loss of cognitive function. Subjects to be treated according to the methods described herein, which include the methods recited in the present claims, include vertebrates, such as mammals. In particular embodiments the mammal is a human patient.

In one instance, the proteinopathy recited in the methods disclosed herein is a disease selected from the group consisting of Alzheimer's disease, frontotemporal dementia, progressive supranuclear palsy, Parkinsonism, Parkinson's disease, Lytico-Bodig disease, dementia with Lewy bodies, tangle- predominant dementia, dementia pugilistica, Pick's disease, corticobasal degeneration, Argyrophilic grain disease, ganglioglioma and gangliocytoma, meningioangiomatosis, subacute sclerosing panencephalitis, lead encephalopathy, tuberous sclerosis, Hallervorden-Spatz disease, and lipofuscinosis. In one instance the proteinopathy is Alzheimer's disease. In another instance the proteinopathy is dementia with Lewy bodies. In another instance the proteinopathy is Parkinson's disease.

In one instance, the proteinopathy is a tauopathy. Tauopathies are neurodegenerative disorders characterized by accumulation of tau. Exemplary tauopathies include, for example, Alzheimer's disease, progressive supranuclear palsy, dementia pugilistica, Parkinson's disease, parkinsonism linked to chromosome 17, Lytico-Bodig disease, tangle-predominant dementia, Argyrophilic grain disease, ganglioglioma, gangliocytoma, meningioangiomatosis, subacute sclerosing panencephalitis, lead encephalopathy, tuberous sclerosis, Hallervorden-Spatz disease, lipofuscinosis, dementia with Lewy bodies, Pick's disease, corticobasal degeneration, frontotemporal dementia, frontotemporal lobar degeneration and Huntington's disease. In one instance, the proteinopathy is a synucleinopathy. Examples of synucleinopathies include, for example, Parkinson's disease, multiple system atrophy, and Lewy Body dementia. Some diseases classified as synucleinopathies may also have accumulation on the tau protein, and some diseases classified as tauopathies may have also have accumulation of the α-synuclein protein. Accordingly, in one instance the proteinopathy is characterized by the accumulation of α-synuclein and tau.

The methods disclosed herein are useful for treating subjects (*e.g.* mammals such as humans) with a proteinopathy. In certain instances, the proteinopathy involves protein aggregates. Protein "aggregation" refers to the biological phenomenon in which misfolded proteins aggregate either intra- or extra-cellularly. These protein aggregates may be toxic. In certain instances, the protein aggregates comprise a protein selected from the group consisting of ubiquitin, tau, and α-synuclein.

Ubiquitin is a small protein that is found in almost all tissues of eukaryotic organisms. It is a 76 amino acid protein that can be attached to a substrate protein. Addition of ubiquitin can result in protein degradation; modulation of transcription, translation, and protein localization; or modulation of protein activity/interactions. Tau proteins function to stabilize microtubules and tau protein can be found in different parts of the cell, such as in the membrane, soluble in the cytosol, and insoluble in the cytosol. They are abundant in neurons of the central nervous system and in astrocytes and oligodendrocytes. Hyperphosphorylation of the tau protein (tau inclusions, "pTau") can result in the self-assembly of tangles of paired helical filaments and straight filaments, which are involved in the pathogenesis of Alzheimer's disease and other tauopathies. All of the six tau isoforms are present in an often hyperphosphorylated state in paired helical filaments from Alzheimer's disease brain. In other neurodegenerative diseases, the deposition of aggregates enriched in certain tau isoforms has been reported. When misfolded, this otherwise very soluble protein can form extremely insoluble aggregates that contribute to a number of neurodegenerative diseases. α-synuclein is a protein that, in humans, is encoded by the *SNCA* gene. α-synuclein can be found in different parts of the cell such as in the membrane, soluble in the cytosol, and insoluble in the cytosol. The protein is found primarily in neural tissue and is predominantly expressed in the neocortex, hippocampus, substantia nigra, thalamus, and cerebellum. Besides neurons, the protein can also be found in neuroglial cells and melanocytic cells, α-synuclein can aggregate to form insoluble fibrils in pathological conditions that are, in some instances, characterized by Lewy bodies.

The methods of the present disclosure may also reduce, reverse or prevent the accumulation of protein aggregates in tissue of a subject diagnosed as having a proteinopathy, or diagnosed as being at risk of developing a proteinopathy.

In one instance the protein aggregates comprise ubiquitin, protein tau and/or α-synuclein, *e.g.* protein tau and/or α-synuclein. In another instance, the protein aggregates comprise protein tau or α-synuclein. In one instance, the protein aggregates comprise protein tau aggregates and α-synuclein aggregates. In one instance, the subject does not have protein aggregates comprising α-synuclein in said tissue. In one instance, the protein aggregates are aggregates of protein tau and the subject does not have protein aggregates comprising α-synuclein in said tissue. In a particular instance, the protein aggregates are aggregates of α-synuclein and the proteinopathy is a synucleinopathy. In another instance, the protein aggregates are aggregates of protein tau and the proteinopathy is a tauopathy, e.g. Parkinson's disease.

In one instance, the tissue is a neuron in the central nervous system of the subject, e.g. a neuron in the substantia nigra, cerebral cortex, hippocampus, frontal lobes and/or temporal lobes of the subject. In another instance, the subject does not have protein aggregates comprising α-synuclein in their central nervous system (CNS), *e.g.* in neurons of the substantia nigra, cerebral cortex, hippocampus, frontal lobes and/or temporal lobes. In a further instance, the proteinopathy is Parkinson's disease characterised by the presence of protein tau, but not α-synuclein, within protein aggregates in the central nervous system of the subject, *e.g.* in neurons of the substantia nigra, cerebral cortex, hippocampus, frontal lobes and/or temporal lobes of the subject.

In certain instances, the methods described herein are effective in reducing a specific fraction of α-synuclein. In one instance, cytosolic insoluble α-synuclein is reduced. In another instance, membrane-associated α-synuclein is reduced. In a further instance, extra-cellular α-synuclein is reduced. In instances, aggregated α-synuclein is reduced by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%. In one instance, aggregated α-synuclein is reduced to a level not significantly different to that of a subject (*e.g.* a mammal such as a human) without a proteinopathy characterized by an increase in α-synuclein.

Administration of quinuclidine compounds as described herein to a subject can alter the processing and localization of α-synuclein within the CNS of the subject, *e.g.* within cortical tissue in the brain. In one instance of the methods described herein, levels of membrane-associated α-synuclein and/or insoluble cytoplasmic α-synuclein are reduced. In certain instances, the levels of membrane-associated α-synuclein and/or insoluble cytoplasmic α-synuclein are reduced, but levels of soluble cytosolic α-synuclein are not reduced (*e.g.* are not significantly altered). In particular instances, the subject is a human subject diagnosed as having a synucleinopathy, or is diagnosed as being at risk of developing a synucleinopathy, especially Parkinson's Disease or Lewy Body Dementia.

In certain instances, the methods described herein are effective in reducing a specific fraction of tau. In one instance, cytosolic insoluble tau is reduced. In another instance, the membrane-associated tau is reduced. In a further instance, extra-cellular tau is reduced. In instances, aggregated tau is reduced by at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%. In one instance, aggregated tau is reduced to a level not significantly different to that of a subject *(e.g.* a mammal such as a human) without a proteinopathy characterized by an increase in tau.

Proteinopathies, especially when present in the central nervous system, can result in an impairment of neural function, *e.g.* cognitive function, autonomic function and/or motor function. Administration of quinuclidine compounds as described herein can result in the improvement of neural function in subjects, *e.g.* in subjects exhibiting cognitive impairment due to a proteinopathy. Accordingly, a quinuclidine compound as described herein may be administered to a subject having impaired neural (*e.g.* neurologic) function. In particular, administration of the quinuclidine compound may be initiated after the subject has been diagnosed with impaired neural *(*e.g. neurologic) function. Diagnosis of a cognitive impairment is within the routine skill of a medical practitioner. Cognitive tests are known in the art and can include tests such as the abbreviated mental test score (AMTS), the mini mental state examination (MMSE), informant questionnaire on cognitive decline in the elderly (IQCODE), and the General Practitioner Assessment of Cognition that test for cognitive impairment. These tests can assess impairments in, for example, memory, reasoning skills, problem solving skills, decision making skills, attention span, and language skills. Imaging methods are also available to diagnose cognitive decline. For example, the functional neuroimaging modalities of single-photon emission computed tomography (SPECT) and positron emission tomography (PET) are useful in assessing cognitive dysfunction. In some aspects, the improvement of neural function is measured by evaluating the cognitive function of the patient. Cognitive deterioration, e.g. associated with mild cognitive impairment, may also be assessed by monitoring different cognitive domains. Cognitive domains include, for example, attention and concentration, executive functions, memory, language, visuo-constructional skills, conceptual thinking, calculations and orientation. Diagnosis of other impairments associated with proteinopathies is also within the routine skill of a medical practitioner. For example, clinical criteria for a diagnosis of Parkinson's disease involve assessing impairments in motor and/or autonomic functions, *e.g.* slowness of movement (bradykinesia), plus either rigidity, resting tremor, or postural instability. Responsiveness to dopamine (symptomatic treatment) and reduced dopaminergic activity in the basal ganglia can also aid in diagnosing Parkinson's disease.

In relation to methods for preventing cognitive decline, such as memory loss, PET imaging using carbon-11 Pittsburgh Compound B as a radiotracer (PIB-PET) has been useful in predictive diagnosis of various kinds of proteinopathies. For example, studies have found PIB-PET to be 86% accurate in predicting which patients with mild cognitive impairment would develop Alzheimer's disease within two years. In another study, using either PIB or another radiotracer, carbon-11 dihydrotetrabenazine (DTBZ), led to more accurate diagnosis for more than one-fourth of patients with mild cognitive impairment or mild dementia.

The methods described herein can prevent, reduce or reverse loss of neural function in a subject diagnosed as having, or at risk of developing, a proteinopathy. Viewed from this aspect, the invention provides a quinuclidine compound as defined in the appended claims for use in a method of preventing, reducing or reversing loss of neural function in a subject diagnosed as having, or at risk of developing, a proteinopathy, wherein the loss of neural function comprises loss of cognitive function.

The methods described herein can also prevent, reduce or reverse the progression of dementia. Symptoms of dementia which may be prevented, reduced or reversed include early symptoms of dementia, such as difficulty remembering recent conversations, names or events, and apathy and depression, as well as later symptoms, such as impaired communication, poor judgment, disorientation, confusion, behavior changes and difficulty in speaking, swallowing and/or walking.

The methods described herein may also be used to prevent or treat cognitive impairment, *e.g.* mild cognitive impairment. Mild cognitive impairment is an intermediate stage between the expected cognitive decline of normal aging and the more serious decline of dementia. The methods described herein can prevent, reduce or reverse loss of cognitive function, and optionally also autonomic function and/or motor function. In certain embodiments, the method prevents, reduces or reverses deterioration in cognitive domains in a subject, *e.g.* the method prevents, reduces or reverses deterioration in attention and concentration, executive functions, memory (*e.g.* working memory), language, visuo-constructional skills, conceptual thinking, calculations, orientation, decision making, problem solving, and the like. The loss of neural function may comprise loss of autonomic function, in addition to loss of cognitive function, such that the method additionally prevents, reduces or reverses orthostatic hypotension, constipation, dysphagia, nausea, hypersalivation, hyperhidrosis, urinary dysfunction, sexual dysfunction, and the like. The loss of neural function may comprise loss of motor function, in addition to loss of cognitive function, such that the method additionally prevents, reduces or reverses Parkinsonism. Parkinsonism is a clinical definition of a variety of underlying pathologies that can result in Parkinson's-like symptoms; these pathologies are caused by a number of disorders, including Parkinson's disease. Symptoms of Parkinsonism which may additionally be prevented, reduced or reversed by the methods disclosed herein include, for example, motor dysfunctions such as tremor, bradykinesia, rigidity, postural instability, impaired balance, and the like.

In one embodiment, the subject does not have protein aggregates comprising α-synuclein in their central nervous system, e.g. in neurons of the substantia nigra, cerebral cortex, hippocampus, frontal lobes and/or temporal lobes. In one embodiment, the proteinopathy is Parkinson's disease characterised by the presence of protein tau, but not α-synuclein, within protein aggregates in the central nervous system of the subject, *e.g.* in neurons of the substantia nigra, cerebral cortex, hippocampus, frontal lobes and/or temporal lobes of the subject.

The methods disclosed herein may be beneficial for subjects who have been diagnosed with a proteinopathy but are not yet experiencing the typical symptoms associated with the disease state, *e.g.* signs of cognitive impairment. Said methods may also be beneficial for subjects who are at risk of developing a proteinopathy due to, for example, a mutation in the subject or the subject's family lineage known to cause a proteinopathy. In one embodiment of the methods described herein, the subject has been diagnosed as being at risk of developing said proteinopathy, and the method prevents or delays the onset and/or development of the proteinopathy in the subject.

For example, mutations in the glucocerebrosidase 1 gene (*GBA1*), which can cause a lysosomal storage disease - Gaucher, are known to be associated with an increased risk of developing certain proteinopathies. Mutations in *GBA1* are known in the art and include, for example, the mutations L444P, D409H, D409V, E235A, and E340A. Accordingly, in one embodiment the subject to be treated has one or more mutations in *GBAl.* In one embodiment the subject suffers from a lysosomal storage disease such as, for example, Gaucher, Fabry, G_{M1}-gangliosidosis, G_{M2} Activator deficiency, Tay-Sachs or Sandhoff. In one embodiment, the subject suffers from Gaucher. In an alternative embodiment the subject to be treated by a method of the invention does not suffer from a lysosomal storage disease such as, for example, Gaucher, Fabry, G_{M1}-gangliosidosis, G_{M2} Activator deficiency, Tay-Sachs or Sandhoff. In one embodiment, the subject does not suffer from Gaucher. In a related embodiment, the subject has one (or more than one) mutation in *GBA1* but does not suffer from a lysosomal storage disease (*e.g.* Gaucher). For example, the subject may be a heterozygous carrier for a *GBA1* mutation. In another embodiment, the subject does not have a deleterious *GBA1* mutation, *e.g.* the gene functions substantially normally in that it encodes a protein with essentially the same structure, activity and/or tissue levels and distribution as the protein encoded by the wild-type gene. Wild-type *GBA1* sequences are known in the art and include GenBank accession number NM_000157.3 (mRNA). In one embodiment, the subject does not have a D409V mutation in *GBA1.*

### Pharmaceutical compositions

The present disclosure also describes pharmaceutical compositions comprising at least one quinuclidine compound as described herein and at least one pharmaceutically acceptable excipient for use according to the methods disclosed herein and recited in the appended claims. The pharmaceutically acceptable excipient can be any such excipient known in the art including those described in, for example, Remington's Pharmaceutical Sciences, Mack Publishing Co. (A. R. Gennaro edit. 1985). Pharmaceutical compositions of the compounds presently disclosed may be prepared by conventional means known in the art including, for example, mixing at least one presently disclosed compound with a pharmaceutically acceptable excipient.

Thus, a pharmaceutical dosage form may comprise a quinuclidine compound as described herein and a pharmaceutically acceptable excipient, wherein the dosage form is formulated to provide, when administered (*e.g.* when administered orally), an amount of said compound sufficient to prevent, reduce or reverse the accumulation of protein aggregates in tissue of a subject (*e.g.* a human subject) diagnosed as having, or being at risk of developing, a proteinopathy. The tissue of the subject may be a neuron of the substantia nigra, cerebral cortex, hippocampus, frontal lobes and/or temporal lobes.

The dosage form may be formulated to provide an amount of said quinuclidine compound sufficient to prevent, reduce or reverse the accumulation of protein tau-containing aggregates in tissue of a subject diagnosed as having, or being at risk of developing, Parkinson's disease. Alternatively, the dosage form may be formulated to provide an amount of said quinuclidine compound sufficient to prevent, reduce or reverse the accumulation of α-synuclein-containing aggregates in tissue of a subject diagnosed as having, or being at risk of developing, Parkinson's disease, Lewy Body Dementia or Alzheimer's disease, *e.g.* Lewy Body Dementia.

A pharmaceutical composition or dosage form can include an agent and another carrier, e.g. compound or composition, inert or active, such as a detectable agent, label, adjuvant, diluent, binder, stabilizer, buffers, salts, lipophilic solvents, preservative, adjuvant or the like. Carriers also include pharmaceutical excipients and additives, for example, proteins, peptides, amino acids, lipids, and carbohydrates (*e.g.* sugars, including monosaccharides, di-, tri-, tetra-, and oligosaccharides; derivatized sugars such as alditols, aldonic acids, esterified sugars and the like; and polysaccharides or sugar polymers), which can be present singly or in combination, comprising alone or in combination 1 to 99.99% by weight or volume. Exemplary protein excipients include serum albumin such as human serum albumin (HSA), recombinant human albumin (rHA), gelatin, casein, and the like. Representative amino acid/antibody components, which can also function in a buffering capacity, include alanine, glycine, arginine, betaine, histidine, glutamic acid, aspartic acid, cysteine, lysine, leucine, isoleucine, valine, methionine, phenylalanine, aspartame, and the like. Carbohydrate excipients are also intended within the scope of this disclosure, examples of which include but are not limited to monosaccharides such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol) and myoinositol.

Carriers which may be used include a buffer or a pH adjusting agent; typically, the buffer is a salt prepared from an organic acid or base. Representative buffers include organic acid salts such as salts of citric acid, ascorbic acid, gluconic acid, carbonic acid, tartaric acid, succinic acid, acetic acid, or phthalic acid; Tris, tromethamine hydrochloride, or phosphate buffers. Additional carriers include polymeric excipients/additives such as polyvinylpyrrolidones, ficolls (a polymeric sugar), dextrates (*e.g.* cyclodextrins, such as 2-hydroxypropyl-β-cyclodextrin), polyethylene glycols, flavoring agents, antimicrobial agents, sweeteners, antioxidants, antistatic agents, surfactants (*e.g.* polysorbates such as "TWEEN 20" and "TWEEN 80"), lipids *(e.g.* phospholipids, fatty acids), steroids *(e.g.* cholesterol), and chelating agents (*e.g.* EDTA).

The present disclosure also describes pharmaceutical compositions, and kits comprising said compositions, which contain at least one quinuclidine compound as described herein and at least one further pharmaceutically-active agent. These pharmaceutical compositions and kits may be adapted to allow simultaneous, subsequent and/or separate administration of the quinuclidine compound and the further active agent. For example, the quinuclidine compound and the further active agent may be formulated in separate dosage forms, *e.g.* in separate tablets, capsules, lyophilisates or liquids, or they may be formulated in the same dosage form, *e.g.* in the same tablet, capsule, lyophilisate or liquid. Where the quinuclidine compound and the further active agent are formulated in the same dosage form, the quinuclidine compound and the further active agent may be present substantially in admixture, *e.g.* within the core of a tablet, or they may be present substantially in discrete regions of the dosage form, *e.g.* in separate layers of the same tablet. The pharmaceutical dosage form may comprise a further agent which is capable of treating or preventing a proteinopathy, *e.g.* a proteinopathy as described herein.

Thus, a pharmaceutical composition may comprise: (i) a quinuclidine compound as described herein; (ii) a further active agent; and (iii) a pharmaceutically acceptable excipient. The further active agent may be an agent which is capable of treating or preventing a proteinopathy, *e.g.* a proteinopathy as described herein. The further active agent may be capable of treating or preventing a proteinopathy when administered orally to a subject.

Examples of further agents capable of treating proteinopathies such as Parkinson's disease include, for example, dopamine precursors (*e.g.* L-DOPA), dopamine agonists (e.g. bromocriptine, cabergoline, pergolide, pramipexole and apomorphine), MAO-B inhibitors *(e.g.* rasagiline and selegiline), anticholinergics *(e.g.* orphenadrine, procyclidine and trihexyphenidyl), enhancers of β-glucocerebrosidase activity (*e.g.* ambroxol and afegostat) and amantadine. Examples of agents capable of treating Alzheimer's include, for example, acetylcholinesterase inhibitors such as tacrine, rivastigmine, galantamine, donepezil, and memantine.

The further active agent may be a chaperone. In one instance, the chaperone is capable of: restoring or enhancing at least partial wild-type function and/or activity of the protein (which is aberrant in the proteinopathy); enhancing the formation of a stable molecular conformation of the protein; inducing trafficking of the protein from the ER to another cellular location, *e.g.* a native cellular location, thereby preventing ER-associated degradation of the protein; and/or preventing aggregation of misfolded protein. In a related instance, the chaperone restores or enhances at least partial wild-type function and/or activity of the protein. In other instances, the chaperone increases the residual activity of a cell *(e.g.* a cell from a mammal suffering from a proteinopathy, synucleinopathy, tauopathy, or the like).

The further active agent may, for example, contain a detectable moiety. Detectable moieties are well known in the art and can be detected by spectroscopic, photochemical, biochemical, immunochemical, physical, or chemical means. Exemplary moieties include, but are not limited to, enzymes, fluorescent molecules, particle labels, electron-dense reagents, radiolabels, biotin, digoxigenin, or a hapten or a protein that has been made detectable. The further active agent may, for example, contain an additional chemical and/or biological moiety not normally part of the agent. Those derivatized moieties can improve delivery, solubility, biological half-life, absorption of the agent, and the like. The moieties can also reduce or eliminate any desirable side effects of the agent and the like. An overview for those moieties can be found in Remington's Pharmaceutical Sciences (20th ed., Mack Publishing Co. 2000) (see also Pathan et al. (2009) Recent Patents on Drug Delivery & Formulation 3:71-89). The agent can be covalently or non-covalently linked to a moiety. In instances, the agent is covalently linked to the moiety. In related instances, the covalent linkage of the moiety is N-terminal to a polynucleotide/polypeptide. In related instances, the covalent linkage of the moiety is C-terminal to a polynucleotide/polypeptide.

Further therapies for proteinopathies which may be combined with the methods described herein include psychosocial interventions, behavioural interventions, reminiscence therapy, validation therapy, supportive psychotherapy, sensory integration, cognitive retraining, rehabilitation, speech therapy, and the like. Other interventions include surgery, rehabilitation, and diet management.

The presently disclosed quinuclidine compounds and pharmaceutical compositions can be used in an animal or human. Thus, a presently disclosed compound can be formulated as a pharmaceutical composition for oral, buccal, parenteral (*e.g.* intravenous, intramuscular or subcutaneous), topical, rectal or intranasal administration or in a form suitable for administration by inhalation or insufflation. In particular embodiments, the quinuclidine compound or pharmaceutical composition is formulated for systemic administration, *e.g.* via a non-parenteral route. In one embodiment, the quinuclidine compound or pharmaceutical composition is formulated for oral administration, *e.g.* in solid form. Such modes of administration and the methods for preparing appropriate pharmaceutical compositions are described, for example, in Gibaldi's Drug Delivery Systems in Pharmaceutical Care (1st ed., American Society of Health-System Pharmacists 2007).

The pharmaceutical compositions can be formulated so as to provide slow, extended, or controlled release of the active ingredient therein using, for example, hydroxypropylmethyl cellulose in varying proportions to provide the desired release profile, other polymer matrices, liposomes and/or microspheres. The pharmaceutical compositions can also optionally contain opacifying agents and may be of a composition that releases the active ingredient(s) only, or preferentially, in a certain portion of the gastrointestinal tract, optionally, in a delayed manner, *e.g.* by using an enteric coating. Examples of embedding compositions include polymeric substances and waxes. The active ingredient can also be in micro-encapsulated form, if appropriate, with one or more pharmaceutically acceptable carriers, excipients, or diluents well known in the art (see, *e.g.,* Remington's). The compounds presently disclosed may be formulated for sustained delivery according to methods well known to those of ordinary skill in the art. Examples of such formulations can be found in United States Patents 3,119,742; 3,492,397; 3,538,214; 4,060,598; and 4,173,626.

In solid dosage forms for oral administration (*e.g.* capsules, tablets, pills, dragees, powders, granules and the like), the active ingredient is mixed with one or more pharmaceutically acceptable carriers, excipients, or diluents, such as sodium citrate or dicalcium phosphate, and/or any of the following: (1) fillers or extenders, such as starches, lactose, sucrose, glucose, mannitol, microcrystalline cellulose, calcium phosphate and/or silicic acid; (2) binders, such as, for example, carboxymethylcellulose, alginates, gelatin, pregelatinized maize starch, polyvinyl pyrrolidone, hydroxypropyl methylcellulose, sucrose and/or acacia; (3) humectants, such as glycerol; (4) disintegrating agents, such as agar-agar, calcium carbonate, sodium starch glycolate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, sodium lauryl sulphate, acetyl alcohol and glycerol monostearate; (8) absorbents, such as kaolin and bentonite clay; (9) lubricants, such as talc, silica, calcium stearate, magnesium stearate, solid polyethylene glycols, sodium lauryl sulfate, and mixtures thereof; and (10) coloring agents. In the case of capsules, tablets, and pills, the pharmaceutical compositions can also comprise buffering agents. Solid compositions of a similar type can also be prepared using fillers in soft and hard-filled gelatin capsules, and excipients such as lactose or milk sugars, as well as high molecular weight polyethylene glycols and the like.

A tablet can be made by compression or molding, optionally with one or more accessory ingredients. Compressed tablets can be prepared using binders (for example, gelatin or hydroxypropylmethyl cellulose), lubricants, inert diluents, preservatives, disintegrants (for example, sodium starch glycolate or cross-linked sodium carboxymethyl cellulose), surface-actives, and/ or dispersing agents. Molded tablets can be made by molding in a suitable machine a mixture of the powdered active ingredient moistened with an inert liquid diluent. The tablets and other solid dosage forms, such as dragees, capsules, pills, and granules, can optionally be scored or prepared with coatings and shells, such as enteric coatings and other coatings well known in the art.

The pharmaceutical compositions may be administered orally in a liquid form. Liquid dosage forms for oral administration of an active ingredient include pharmaceutically acceptable emulsions, microemulsions, solutions, suspensions, syrups and elixirs. Liquid preparations for oral administration may be presented as a dry product for constitution with water or other suitable vehicle before use. In addition to the active ingredient, the liquid dosage forms can contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (*e.g.* cottonseed, groundnut, corn, germ, olive, castor and sesame oils), glycerol, tetrahydrofuryl alcohol, polyethylene glycols and fatty acid esters of sorbitan, and mixtures thereof. In addition to inert diluents, the liquid pharmaceutical compositions can include adjuvants such as wetting agents, emulsifying and suspending agents, sweetening, flavoring, coloring, perfuming and preservative agents, and the like. Suspensions, in addition to the active ingredient(s) can contain suspending agents such as, but not limited to, ethoxylated isostearyl alcohols, polyoxyethylene sorbitol and sorbitan esters, microcrystalline cellulose, aluminum metahydroxide, bentonite, agar-agar and tragacanth, and mixtures thereof. Suitable liquid preparations may be prepared by conventional means with a pharmaceutically acceptable additive(s) such as a suspending agent (*e.g.* sorbitol syrup, methyl cellulose or hydrogenated edible fats); emulsifying agent (*e.g.* lecithin or acacia); non-aqueous vehicle *(e.g.* almond oil, oily esters or ethyl alcohol); and/or preservative *(e.g.* methyl or propyl p-hydroxybenzoates or sorbic acid). The active ingredient(s) can also be administered as a bolus, electuary, or paste.

For buccal administration, the composition may take the form of tablets or lozenges formulated in a conventional manner.

Alternatively, the pharmaceutical compositions may be administered by non-oral means such as by topical application, transdermal application, injection, and the like. The pharmaceutical compositions may be administered parenterally by injection, infusion, or implantation (*e.g.* intravenous, intramuscular, intra-arterial, subcutaneous, and the like).

Presently disclosed compounds may be formulated for parenteral administration by injection, including using conventional catheterization techniques or infusion. Formulations for injection may be presented in unit dosage form, *e.g.* in ampules or in multi-dose containers, with an added preservative. The compositions may take such forms as suspensions, solutions or emulsions in oily or aqueous vehicles, and may contain a formulating agent such as a suspending, stabilizing and/or dispersing agent recognized by those of skill in the art. Alternatively, the active ingredient may be in powder form for reconstitution with a suitable vehicle, e.g. sterile pyrogen-free water, before use.

The pharmaceutical compositions may be administered directly to the central nervous system. Accordingly, in certain embodiments the compositions are administered directly to the central nervous system so as to avoid the blood brain barrier. In some embodiments, the composition can be administered via direct spinal cord injection. In embodiments, the composition is administered by intrathecal injection. In some embodiments, the composition is administered via intracerebroventricular injection. In embodiments, the composition is administered into a cerebral lateral ventricle. In embodiments, the composition is administered into both cerebral lateral ventricles. In additional embodiments, the composition is administered via intrahippocampal injection. The compositions may be administered in one injection or in multiple injections. In other embodiments, the composition is administered to more than one location (*e.g.* to two sites in the central nervous system).

The pharmaceutical compositions can be in the form of sterile injections. The pharmaceutical compositions can be sterilized by, for example, filtration through a bacteria-retaining filter, or by incorporating sterilizing agents in the form of sterile solid compositions which can be dissolved in sterile water, or some other sterile injectable medium immediately before use. To prepare such a composition, the active ingredient is dissolved or suspended in a parenterally acceptable liquid vehicle. Exemplary vehicles and solvents include, but are not limited to, water, water adjusted to a suitable pH by addition of an appropriate amount of hydrochloric acid, sodium hydroxide or a suitable buffer, 1,3-butanediol, Ringer's solution and isotonic sodium chloride solution. The pharmaceutical composition can also contain one or more preservatives, for example, methyl, ethyl or n-propyl p-hydroxybenzoate. To improve solubility, a dissolution enhancing or solubilising agent can be added or the solvent can contain 10-60% w/w of propylene glycol or the like.

The pharmaceutical compositions can contain one or more pharmaceutically acceptable sterile isotonic aqueous or nonaqueous solutions, dispersions, suspensions or emulsions, or sterile powders, which can be reconstituted into sterile injectable solutions or dispersions just prior to use. Such pharmaceutical compositions can contain antioxidants; buffers; bacteriostats; solutes, which render the formulation isotonic with the blood of the intended recipient; suspending agents; thickening agents; preservatives; and the like.

Examples of suitable aqueous and nonaqueous carriers, which can be employed in the pharmaceutical compositions include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants. In order to prolong the effect of an active ingredient, it may be desirable to slow the absorption of the compound from subcutaneous or intramuscular injection. This can be accomplished by the use of a liquid suspension of crystalline or amorphous material having poor water solubility. The rate of absorption of the active ingredient then depends upon its rate of dissolution which, in turn, can depend upon crystal size and crystalline form. Alternatively, delayed absorption of a parenterally-administered active ingredient is accomplished by dissolving or suspending the compound in an oil vehicle. In addition, prolonged absorption of the injectable pharmaceutical form can be brought about by the inclusion of agents that delay absorption such as aluminum monostearate and gelatin.

Controlled release parenteral compositions can be in form of aqueous suspensions, microspheres, microcapsules, magnetic microspheres, oil solutions, oil suspensions, emulsions, or the active ingredient can be incorporated in biocompatible carrier(s), liposomes, nanoparticles, implants or infusion devices. Materials for use in the preparation of microspheres and/or microcapsules include, but are not limited to, biodegradable/bioerodible polymers such as polyglactin, poly-(isobutyl cyanoacrylate), poly(2-hydroxyethyl-L-glutamine) and poly(lactic acid). Biocompatible carriers which can be used when formulating a controlled release parenteral formulation include carbohydrates such as dextrans, proteins such as albumin, lipoproteins or antibodies. Materials for use in implants can be non-biodegradable, *e.g.* polydimethylsiloxane, or biodegradable such as, *e.g.,* poly(caprolactone), poly (lactic acid), poly(glycolic acid) or poly(ortho esters).

For topical administration, a presently disclosed compound may be formulated as an ointment or cream. Presently disclosed compounds may also be formulated in rectal compositions such as suppositories or retention enemas, *e.g.* containing conventional suppository bases such as cocoa butter or other glycerides.

For intranasal administration or administration by inhalation, presently disclosed compounds may be conveniently delivered in the form of a solution or suspension from a pump spray container that is squeezed or pumped by the patient or as an aerosol spray presentation from a pressurized container or a nebulizer, with the use of a suitable propellant, *e.g.* dichlorodifluoromethane, trichlorofluoromethane, dichlorotetrafluoroethane, carbon dioxide or other suitable gas. In the case of a pressurized aerosol, the dosage unit may be determined by providing a valve to deliver a metered amount. The pressurized container or nebulizer may contain a solution or suspension of the presently disclosed compound. Capsules and cartridges (made, for example, from gelatin) for use in an inhaler or insufflator may be formulated containing a powder mix of a presently disclosed compound and a suitable powder base such as lactose or starch.

Generally, the agents and compositions described herein are administered in an effective amount or quantity sufficient to treat or prevent a proteinopathy in a subject. Typically, the dose can be adjusted within this range based on, *e.g.,* age, physical condition, body weight, sex, diet, time of administration, and other clinical factors. Determination of an effective amount is well within the capability of those skilled in the art.

A proposed dose of a quinuclidine compound as described herein for oral, parenteral or buccal administration to the average adult human for the treatment or prevention of a proteinopathy is 0.1 mg to 2000 mg. For example, the proposed dose may be from 0.2 mg to 1000 mg of the active ingredient per unit dose. Irrespective of the amount of the proposed dose, administration of the compound can occur, for example, 1 to 4 times per day. The dose for oral administration may, for example, be 0.5 to 2000 mg, *e.g.* 1 to 750 mg. The dose for direct administration into the central nervous system may, for example, be 1 µg to 1 mg, *e.g.* 5 µg to 0.5 mg, or 10 µg to 0.1 mg. Aerosol formulations for the treatment or prevention of the conditions referred to above in the average adult human may be arranged so that each metered dose or "puff" of aerosol contains 1 mg to 10 g, *e.g.* 2 mg to 1 g of a presently disclosed compound. Administration may be several times daily, for example 2, 3, 4 or 8 times, giving for example, 1, 2 or 3 doses each time.

Having been generally described herein, the follow non-limiting examples are provided to further illustrate this invention.

### EXAMPLES

### General procedures for chemical synthesis

### General Procedure A: Carbamate formation with triphosgene

To a suspension of amine hydrochloride (1 equivalent) and triethylamine (3-4 equivalents) in a THF (concentration - 0.2M) at room temperature was added triphosgene (0.35 equivalents). The reaction mixture was stirred for 10 min and small amount of ether (1-2 mL) was added. The triethylammonium salt was filtered off to afford a clear solution of isocyanate in THF/ether.

To a solution of alcohol (1.5 equivalents) in THF (concentration - 0.2M) at room temperature was added NaH [60%, oil] (1.5 equivalents). The reaction mixture was stirred for 15 min and the above solution (isocyanate in THF/ether) was added dropwise. In a standard workup, the reaction was quenched with brine. The solution was extracted with EtOAc and the organic layer was dried over Na₂SO₄, filtered and concentrated. The crude material was purified on combiflash (SiO₂ cartridge, CHCl₃ and 2N NH₃ in MeOH) to afford the corresponding carbamate.

### General Procedure B: Alkylation with organocerium

A suspension of CeCl₃ (4 equivalents) in THF (concentration - 0.2M) was stirred at room temperature for 1 h. The suspension was cooled to -78°C and MeLi/Ether [1.6M] (4 equivalents) was added dropwise. The organocerium complex was allowed to form for a period of 1 h and a solution of nitrile (1 equivalent) in THF (concentration 2.0M) was added dropwise. The reaction mixture was warmed up to room temperature and stirred for 18 h. The solution was cooled to 0 °C and quenched with water (~ 1 mL) followed by addition of 50% aqueous solution of ammonium hydroxide (-3 mL) until precipitated formed and settled to the bottom of the flask. The mixture was filtered through a pad of celite and concentrated. The crude material was treated with a solution of HCl/dioxane [4.0M]. The intermediate arylpropan-2-amine hydrochloride was triturated in ether and used as is for the next step. Alternatively, the crude free base amine was purified on combiflash (SiO₂ cartridge, CHCl₃ and 2N NH₃ in MeOH) to afford the corresponding arylpropylamine.

### General Procedure C: Suzuki coupling

To a solution of aryl halide (1 equivalent) in a mixture of DME/water [4:1] (concentration - 0.2M) was added boronic acid (2 equivalents), palladium catalyst (0.1-0.25 equivalent) and sodium carbonate (2 equivalents). The reaction mixture was microwaved 25 min at 150°C. After filtering through a celite plug and concentrating, the crude product was purified on combiflash (SiO₂ cartridge, CHCl₃ and 2N NH₃ in MeOH) to afford the corresponding coupling adduct.

Alternatively: To a solution of aryl halide (1 equivalent) in a mixture of toluene/water [20:1] (concentration - 0.2 M) was added boronic acid (1.3-2.5 equivalents), palladium catalyst (0.05-0.15 equivalent), tricyclohexylphosphine (0.15-0.45 equivalent) and potassium phosphate (5 equivalents). The reaction mixture was microwaved 25 min at 150°C. After filtering through a celite plug and concentrating, the crude product was purified on combiflash (SiO₂ cartridge, CHCl₃ and 2N NH₃ in MeOH) to afford the corresponding coupling adduct.

### General Procedure D: Cyclopropanation

To a mixture of aryl nitrile (1 equivalent) and Ti(Oi-Pr)₄ (1.7 equivalents) stirring at -70°C, was added dropwise EtMgBr [3.0 M in ether] (1.1 equivalents). The reaction mixture was allowed to warm to 25°C and stirred for 1 h. To the above mixture was added BF₃·Et₂0 (3 equivalents) dropwise at 25°C. After the addition, the mixture was stirred for another 2 h, and then quenched with aqueous HCI [2M]. The resulting solution was then basified by adding aqueous NaOH [2M]. The organic material was extracted with ethyl ether. The organic layers were combined, dried over Na₂SO₄, filtered and concentrated. The crude material was purified by silica gel column chromatography (eluting with petroleum ether/EtOAc: 10/1 to 1/1) to give the corresponding 1-aryl-cyclopropanamine.

### General Procedure E: Biaryl coupling using Suzuki conditions

To a stirred solution of the aryl halide component (1 equivalent) in 5:1 (v/v) dioxane/water (~0.15 M) or 5:1 (v/v) N,N-dimethylformamide (~0.15 M), was added the arylboronate or arylboronic acid component (1-1.5 equivalents), sodium carbonate (2-3 equivalents) and [1,1'-bis(diphenylphosphino)ferrocene]dichloropalladiurn(II) (0.05 equivalents). The mixture was heated (90 °C) overnight and then filtered through a plug of Celite. The Celite was rinsed with ethyl acetate and the combined filtrate was washed with brine, dried (Na₂SO₄) and concentrated. The residue was purified by flash chromatography over silica.

### General Procedure F: Carbamate formation using an isocyanate generated via a mixed anhydride/Curtius Rearrangement route

To a stirred solution of the carboxylic acid component (1 equivalent) in tetrahydrofuran (~0.1 M) was added triethylamine (2 equivalents). The reaction was cooled (0 °C) and treated with isobutyl chloroformate (1.5 equivalents). After 1 hour at 0 °C, a solution of sodium azide (2 equivalents) in water (~1 M) was added and the reaction was allowed to warm to room temperature. After overnight stirring, the reaction was diluted with water and extracted with ethyl acetate. The combined extracts were washed with aqueous sodium bicarbonate solution and brine, dried (Na₂SO₄) and concentrated. The crude acyl azide was further dried via coevaporation with toluene and then taken up in toluene (~0.1 M). The stirred solution was refluxed for 2-2.5 hours, cooled and treated with an alcohol component (1.25-2 equivalents). The reaction was heated at reflux overnight and then concentrated. The residue was taken up in either ethyl acetate or chloroform and washed with aqueous sodium carbonate, (Na₂SO₄) and concentrated. The crude product was purified by flash chromatography over silica using chloroform/methanol (less polar carbamates) or chloroform/methanol/ammonia (more polar carbamates) solvent gradients.

### Example 1: 1-azabicyclo[2.2.2]oct-3-yl [2-(4'-fluorobiphenyl-3-yl)propan-2-yl]carbamate (Compound 1)

Using General Procedure C, 1-azabicyclo[2.2.2]oct-3-yl [2-(3-bromophenyl)propan-2-yl]carbamate (600 mg, 1.63 mmol), 4-fluorophenyl boronic acid (457 mg, 3.27 mmol) and palladium (II) acetate gave the title compound as a white solid (373 mg; 60%). ¹H NMR (400 MHz, CDCl₃) δ 7.56 (s, 1H), 7.52 (dd, *J* = 5.4, 8.4 Hz, 2H), 7.42-7.38 (m, 3H), 7.12 (m, 2H), 5.18 (5, 1H), 4.62 (s, 1H), 2.66 (m, 6H), 1.72 (s, 6H), 2.01-0.83 (m, 5H) ppm ¹³C NMR (100 MHz, CDCl₃) δ 125.0, 124.0, 123.8, 116.0, 116.0, 71.3, 55.9, 55.5, 47.6, 46.7, 29.6, 25.6, 24.8, 19.8 ppm. Purity: 98.0% UPLCMS (210 nm); retention time 0.95 min; (M+1) 382.9. Anal. Calcd. for C₂₃H₂₇FN₂O₂·0.37(CHCl₃): C, 65.86; H, 6.47; N, 6.57. Found: C, 65.85; H, 6.69; N, 6.49.

### Example 2: (S)-quinuclidin-3-yl 2-(2-(4-fluorophenyl)thiazol-4-yl)propan-2-vlcarbamate (Compound 2)

To a stirred solution of 4-fluorothiobenzamide (8.94 g, 57.6 mmol) in ethanol (70 mL) was added ethyl 4-chloroacetoacetate (7.8 mL, 58 mmol). The reaction was heated at reflux for 4 hours, treated with an addition aliquot of ethyl 4-chloroacetoacetate (1.0 mL, 7.4 mmol) and refluxed for an additional 3.5 hours. The reaction was then concentrated and the residue was partitioned between ethyl acetate (200 mL) and aqueous NaHCO₃ (200 mL). The organic layer was combined with a backextract of the aqueous layer (ethyl acetate, 1 × 75 mL), dried (Na₂SO₄) and concentrated. The resulting amber oil was purified by flash chromatography using a hexane/ethyl acetate gradient to afford ethyl 2-(2-(4-fluorophenyl)1hiazol-4-yl)acetate as a low melting, nearly colourless solid (13.58 g, 89%).

To a stirred solution of ethyl 2-(2-(4-fluorophenyl)thiazol-4-yl)acetate (6.28 g, 23.7 mmol) in DMF (50 mL) was added sodium hydride [60% dispersion in mineral oil] (2.84 g, 71.0 mmol). The frothy mixture was stirred for 15 minutes before cooling in an ice bath and adding iodomethane (4.4 mL, 71 mmol). The reaction was stirred overnight, allowing the cooling bath to slowly warm to room temperature. The mixture was then concentrated and the residue partitioned between ethyl acetate (80 mL) and water (200 mL). The organic layer was washed with a second portion of water (1 × 200 mL), dried (Na₂SO₄) and concentrated. The resulting amber oil was purified by flash chromatography using a hexane/ethyl acetate gradient to afford ethyl 2-(2-(4-fluorophenyl)thiazol-4-yl)-2-methylpropanoate as a colourless oil (4.57 g, 66%).

To a stirred solution of ethyl 2-(2-(4-fluorophenyl)thiazol-4-yl)-2-methylpropanoate (4.56 g, 15.5 mmol) in 1:1:1 TBF/ethanol/water (45 mL) was added lithium hydroxide monohydrate (2.93 g, 69.8 mmol). The reaction was stirred overnight, concentrated and redissolved in water (175 mL). The solution was washed with ether (1 × 100 mL), acidified by the addition of 1.0 N HCl (80 mL) and extracted with ethyl acetate (2 x 70 mL). The combined extracts were dried (Na₂SO₄) and concentrated to afford 2-(2-(4-fluorophenyl)thiazol-4-yl)-2-methylpropanoic acid as a white solid (4.04 g, 98%). This material was used in the next step without purification.

To a stirred and cooled (0 °c) solution of 2-(2-(4-fluorophenyl)thiazol-4-yl)-2-methylpropanoic acid (4.02 g, 15.2 mmol) in THF (100 mL) was added trimethylamine (4.2 mL, 30 mmol) followed by isobutyl chloroformate (3.0 mL, 23 mmol). The reaction was stirred cold for another 1 hour before adding a solution of sodium azide (1.98 g, 30.5 mmol) in water (20 mL). The reaction was stirred overnight, allowing the cooling bath to slowly warm to room temperature. The mixture was then diluted with water (100 mL) and extracted with ethyl acetate (2 × 60 mL). The combined extracts were washed with aqueous NaHCO₃ (1 × 150 mL) and brine (1 × 100 mL), dried (Na₂SO₄) and concentrated. After coevaporating with toluene (2 × 50 mL), the resulting white solid was taken up in toluene (100 mL) and refluxed for 4 hours. (S)-3-quinuclidinol (3.87 g, 30.4 mmol) was then added and reflux was continued overnight. The reaction was concentrated and the residue partitioned between ethyl acetate (100 mL) and aqueous NaHCO₃ (150 mL). The organic layer was washed with water (1 × 150 mL), dried (Na₂SO₄) and concentrated. The resulting off-white solid was purified by flash chromatography using a chloloform/methanol/ammonia gradient to afford the title compound as a white solid (4.34 g, 73%). ¹H NMR (400 MHz, CDCl₃) δ 7.96-7.88 (m, 2H), 7.16-7.04 (m, 3H), 5.55 (br s, 1H), 4.69-4.62 (m, 1H), 3.24-3.11 (m, 1H), 3.00-2.50 (m, 5H), 2.01-1.26 (m, 11H) ppm ¹³C NMR (400 MHz, CDCl₃) δ 166.4,165.1, 163.8 (d, *J*=250.3 Hz), 162.9, 155.0, 130.1 (d, *J*=3.3 Hz), 128.4 (d, *J*= 8.5 Hz), 115.9 (d, *J*= 22.3 Hz), 112.5, 71.2, 55.7, 54.2, 47.5, 46.5, 28.0, 25.5, 24.7, 19.6 ppm Purity: 100 % UPLCMS (210 nm & 254 nm); retention time 0.83 min; (M+1) 390.

### Example 3: (S)-quinuclidin-3-yl (2-(4'-(2-methoxyethoxy)-[1,1'-biphenyl]-4-yl)propan-2-yl)carbamate (Compound 3)

Using General Procedure E and the reaction inputs ethyl 2-(4-bromophenyl)-2-methylpropanoate and 4-(2-methoxyethoxy)phenylboronic acid, ethyl 2-(4'-(2-methoxyethoxy)-[1,1'-biphenyl]-4-yl)-2-methylpropanoate was prepared as an off-white solid. To a stirred solution of this compound (3.01 g, 8.78 mmol) in 1:1:1 (v/v/v) tetrahydrofuran/ethanol/water (45 mL) was added lithium hydroxide monohydrate (1.47 g, 61.4 mmol). The mixture was heated at reflux overnight and then concentrated. The residue was dissolved in water, treated with 1N hydrochloric acid (65 mL) and extracted with ethyl acetate. The combined organic layers were washed with brine, dried (Na₂SO₄) and concentrated to afford 2-(4'-(2-methoxyethoxy)-[1,1'-biphenyl]-4-yl)-2-methylpropanoic acid as a white solid (2.75 g, 100%). This intermediate and (*S*)-quinuclidin-3-ol were reacted according to General Procedure F to generate the title compound as a colourless, glassy solid. ¹H NMR (400 MHz, DMSO-*d₆*) δ 7.62-7.29 (m, 7H), 7.01 (d, *J* = 8.9 Hz, 2H), 4.47-4.37 (m, 1H), 4.17-4.08 (m, 2H), 3.72-3.62 (m, 2H), 3.32 (s, 3H), 3.09-2.25 (m, 6H), 2.05-1.18 (m, 11H) ppm. ¹³C NMR (100 MHz, DMSO-*d₆*) δ 157.9, 154.5, 146.7, 137.4, 132.5, 127.5, 125.7, 125.2, 114.8, 70.4, 70.0, 66.9, 58.2, 55.4, 54.2, 46.9, 45.9, 29.4, 25.3, 24.2, 19.2 ppm. Purity: 100%, 100% (210 & 254 nm) UPLCMS; retention time: 0.87 min; (M+H⁺) 439.5.

### Example 4: 1-azabicyclo[2.2.2]oct-3-yl[2-(biphenyl-3-yl)propan-2-yl]carbamate (Compound 4)

Using General Procedure C, 1-azabicyclo[2.2.2]oct-3-yl [2-(3-bromophenyl)propan-2-yl]carbamate (600 mg, 1.63 mmol), phenylboronic acid (398 mg, 3.27 mmol) and palladium (II) acetate gave the title compound as a white solid (379 mg, 64%). ¹H NMR (400 MHz, CDCl₃) δ 7.61 (s, 1H), 7.56 (d, *J*= 7.4 Hz, 2H), 7.50-7.38 (m, 4H), 7.34 (m, 2H), 5.16 (s, 1H), 4.63 (s, 1H), 3.39-2.09 (m, 6H), 1.72 (s, 6H), 2.02-0.73 (m, 5H) ppm. ¹³C NMR (100 MHz, CDCl₃) δ 154.8, 147.8, 141.6, 129.0, 129.0, 128.6, 127.5, 125.8, 125.0, 124.0, 71.6, 71.3, 55.9, 55.5, 47.6, 46.8, 31.5, 30.2, 30.0, 29.5, 25.6, 24.8, 19.8 ppm. Purity: 99% UPLCMS (210 nm); retention time 0.84 min; (M+1) 365.0. Anal. Calcd. for C₂₃H₂₈N₂O₂·0.29(CHCl₃): C, 70.02; H, 7.14; N, 7.01. Found: C, 70.02; H, 7.37; N, 6.84.

### Example 5: (S)-quinuclidin-3-yl 2-(biphenyl-4-yl)propan-2-ylcarbamate (Compound 5)

Using General Procedure B, bromobenzonitrile (2.00 g, 11.0 mmol) was converted to the corresponding 2-(4-bromophenyl)propan-2-amine (1.20 g, 51%) as a brown oil.

Using General Procedure A, 2-(4-bromophenyl)propan-2-amine (1.0 g, 4.7 mmol) and (S)-quinuclidin-3-ol gave (S)-quinuclidin-3-yl 2-(4-bromophenyI)propan-2-ylcarbamate (1.0 g, 58%) as a brown oil.

Using General Procedure C, the above bromide (200 mg, 0.540 mmol), phenylboronic acid (133 mg, 1.10 mmol) and [PdCl₂(pddf)]CH₂Cl₂ gave the title compound as a white solid (70 mg, 35%). ¹H NMR (500 MHz, CDCl₃) δ 7.60-7.53 (m, 4H), 7.47 (d, *J* = 8.5 Hz, 2H), 7.42 (t, *J* = 7.5 Hz, 2H), 7.33 (t, *J* = 7.5 Hz, 1H), 5.26 (br s, 1H), 4.64 (m, 1H), 3.33-3.15 (m, 1H), 3.10-2.45 (m, 5H), 2.40-1.80 (m, 2H), 1.78-1.58 (m, 7H), 1.55-1.33 (m, 2H) ppm. ¹³C NMR (125 MHz, CDCl₃) δ 154.5, 146.1, 140.8, 139.5, 128.7, 127.2, 127.1, 127.1, 125.2, 70.9, 55.5, 55.1, 47.4, 46.4, 31.1, 29.5, 25.3, 24.5, 19.5 ppm. Purity: 100 % LCMS (214 nm & 254 nm); retention time 1.56 min; (M+1) 365.

### Example 6: Quinuclidin-3-yl 1-(biphenyl-4-yl)cyclopropylcarbamate (Compound 6)

Using General Procedure D, bromobenzonitrile (3.00 g, 16.5 mmol) was converted to the corresponding 1-(4-bromophenyl)cyclopropanamine (1.80 g, 51%) as a yellow solid.

Using General Procedure A, 1-(4-bromophenyl)cyclopropanamine (1.0 g, 4.7 mmol) and quinuclidin-3-ol gave quinuclidin-3-yl 1-(4-bromophenyl)cyclopropyl-carbamate (1.3 g, 75%) as a white semi-solid.

Using General Procedure C, the above carbamate (400 mg, 1.12 mmol), phenylboronic acid (267 mg, 2.22 mmol) and [PdCl₂(pddf)]CH₂Cl₂ the title compound as a viscous oil (100 mg, 25%). ¹H NMR (500 MHz, CDCl₃) δ 7.47 (d, *J* = 7.5 Hz, 2H), 7.43 (d, *J*= 8.0 Hz, 2H), 7.33 (t, *J*= 7.5 Hz, 2H), 7.26-7.15 (m, 3H), 5.93 (br s, 0.6H), 5.89 (br s, 0.4H), 4.67 (m, 1H), 3.20-3.06 (m, 1H), 2.88-2.42 (m, 5H), 1.98-1.08 (m, 9H) ppm ¹³C NMR (125 MHz, CDCl₃) δ 155.0, 141.0, 139.7, 138.2, 127.7, 126.1, 126.0, 124.8, 124.1, 70.0, 54.5, 46.3, 45.4, 34.1, 24.3, 23.2, 18.3, 17.0 ppm. Purity: 100 % LCMC (214 nm & 254 nm); retention time 1.52 min; (M+1) 363.

### Example 7: (S)-quinuclidin-3-yl 1-(4'-fluorobiphenyl-4-yl)cyclopropylcarbamate (Compound 7)

Using General Procedure C, (S)-quinuclidin-3-yl 1-(4-bromophenyl)cyclopropyl carbamate, 4-F-phenylboronic acid and [PdCl₂(pddf)]CH₂Cl₂ gave the title compound as a white solid (45%). ¹H NMR (500 MHz, DMSO-*d₆*) δ 8.06-7.83 (d, 1H), 7.69-7.66 (m, 2H), 7.59-7.55 (m, 2H), 7.29-7.22 (m, 4H), 4.56-4.54 (m, 1H), 3.13-2.32 (m, 6H), 1.91-1.19 (m, 9H) ppm. ¹³C NMR (125 MHz, DMSO-*d₆*) δ 163.2, 161.2, 156.4, 143.7, 136.9, 128.9, 128.8, 126.8, 125.6, 116.2, 116.0, 70.7, 55.8, 47.4, 46.4, 34.8, 25.7, 24.6, 19.6, 18.7, 18.6 ppm Purity: > 97 % LCMS (214 nm & 254 nm); retention time 1.96 min; (M+1) 381.2.

### Example 8: (S)-1-azabicyclo[2.2.2]oct-3-yl [1-(2',4'-difluorobiphenyl-4-yl)cyclopropyl]carbamate (Compound 8)

Using General Procedure C, (S)-quinuclidin-3-yl 1-(4-bromophenyl)cyclopropylcarbamate (0.446 g, 1.22 mmol), 2,4-difluorophenyl boronic acid (0.386 g, 2.44 mmol) and Pd(OAc)₂ (0.015 g, 0.067 mmol) gave the title compound as a tan solid (0.111 g, 23%). ¹H NMR (CDCl₃) δ 7.43 (dd, *J* = 8.4, 1.6 Hz, 2H), 7.40-7.33 (m, 1H), 7.31 (d, *J* = 7.7 Hz, 2H), 6.99-6.81 (m, 2H), 5.54 (d, *J* = 48.0 Hz, 1H), 4.82-4.65 (m, 1H), 3.30-3.07 (m, 1H), 2.98-2.44 (m, 5H), 1.97 (d, *J* = 32.7 Hz, 1H), 1.83 (d, *J* = 10.3 Hz, 1H), 1.64 (s, 1H), 1.52 (s, 1H), 1.39 (s, 1H), 1.31 (d, *J* = 6.8 Hz, 4H) ppm ¹³C NMR major rotomer (CDCl₃) δ 162.2 (dd, *J* = 12.8, 249.1 Hz), 159.8 (dd, *J* = 11.8, 251.0 Hz), 156.9, 156.0, 142.6, 133.1, 131.3 (m), 128.9, 125.6, 124.9, 111.5 (dd, *J* = 3.9, 21.2 Hz) 104.4 (dd, *J* = 25.2, 29.4 Hz), 72.1, 71.6, 55.7, 47.4, 46.5, 35.7, 35.3, 25.5, 24.6, 24.4, 19.5, 18.1 ppm Purity: LCMS > 99.3 % (214 nm & 254 nm); retention time 0.90 min; (M+1) 399.0.

### Example 9: 1-azabicyclo[2.2.2]oct-3-yl[1-(4'-methoxybiphenyl-4-yl)cyclopropyl]carbamate (Compound 9)

Using General Procedure C, quinuclidin-3-yl 1-(4-bromophenyl)cyclopropylcarbamate (0.485 g, 1.33 mmol), 4-methoxyphenyl boronic acid (0.404 g, 2.66 mmol) and Pd(OAc)₂ (0.016 g, 0.071 mmol) gave the title compound as a grey solid (0.337 mg, 65%). ¹H NMR (CDCl₃) δ 7.48 (dd, *J* = 8.6, 5.5 Hz, 4H), 7.29 (d, *J* = 7.6 Hz, 2H), 6.96 (d, *J* = 8.8 Hz, 2H), 5.58 (d, *J* = 48.7 Hz, 1H), 4.83-4.63 (m, 1H), 3.84 (s, 3H), 3.20 (dd, *J* = 24.0, 15.5 Hz, 1H), 2.97-2.42 (m, 5H), 1.97 (d, *J* = 30.9 Hz, 1H), 1.81 (s, 1H), 1.75-1.33 (m, 3H), 1.28 (d, *J* = 6.8 Hz, 4H) ppm ¹³C NMR major rotomer (CDCl₃) δ 159.1, 156.0, 141.4, 139.0, 133.4, 128.0, 126.7, 125.9, 114.2, 71.5, 55.7, 55.3, 47.4, 46.5, 35.3, 25.5, 24.6, 19.6, 17.8 ppm Purity: LCMS >97.1 % (214 nm & 254 nm); retention time 0.88 min; (M+1) 393.4.

### Example 10: Quinuclidin-3-yl 2-(5-(4-fluorophenyl)thiophen-3-yl)propan-2-ylcarbamate (Compound 10)

To a stirred and cooled (0 °C) solution of ethyl 5-bromothiophene-3-carboxylate (13.30 g, 56.57 mmol) in THF (100 mL) was added a solution of methyl magnesium bromide in diethyl ether [3.0 M] (55.0 mL, 165 mmol), dropwise over 20 minutes. After 2 hours, the reaction solution was concentrated. The residue was taken up in aqueous NH₄Cl (200 mL) and extracted with ethyl acetate (2 x 100 mL). The combined extracts were dried (Na₂SO₄) and concentrated. The resulting amber oil was purified by flash chromatography using a hexane/ethyl acetate gradient to afford 2-(5-bromothiophen-3-yl)propan-2-ol as a pale amber oil (8.05 g, 64%).

To a stirred solution of 2-(5-bromothiophen-3-yl)propan-2-ol (8.03 g, 36.3 mmol) in methylene chloride (80 mL) was added sodium azide (7.08 g, 109 mmol) followed by trifluoroacetic acid (8.0 mL; dropwise over 5-6 minutes). The thickening suspension was stirred for 1.5 hour before diluting with water (350 mL) and extracting with ethyl acetate (1 × 200 mL). The organic layer was washed with aqueous NaHCO₃ (1 × 250 mL), dried (Na₂SO₄) and concentrated to afford the crude azide product. To a stirred solution of this material in THF (160 mL) was added water (11 mL) followed by triphenylphosphine (23.8 g, 90.7 mmol). The reaction was stirred for 2 days before concentrating. The resulting residue was dissolved in ethyl acetate (250 mL) and extracted with 1N aqueous HCl (4 x 75 mL). The combined extracts were basified with concentrated NH₄OH and extracted with ethyl acetate (2 x 100 mL). These extracts were, in turn, dried (Na₂SO₄) and concentrated. The resulting amber oil was purified by flash chromatography using a methylene chloride/methanol/ammonia gradient to afford a mixture of 2-(5-bromothiophen-3-yl)propan-2-amine and triphenylphosphine oxide (~70/30 ratio) as a viscous amber oil (1.32 g, 17%).

To a stirred solution of 3-quinuclidinol (3.00 g, 23.6 mmol) in THF (100 mL) was added 4-nitrophenyl chloroformate (5.94 g, 29.5). After stirring for 4 hours, the precipitate was filtered off, rinsed with THF and air dried on the frit under house vacuum. The filtercake was dissolved in ethyl acetate (150 mL) and washed with aqueous NaHCO₃ (1 × 150 mL) and water (2 x 150 mL). The organic layer was dried (Na₂SO₄) and concentrated to afford crude 4-nitrophenyl quinuclidin-3-yl carbonate product, which was used in the next step without purification.

To a stirred solution of 2-(5-bromothiophen-3-yl)propan-2-amine (0.366 g, 1.66 mmol) in THF (10 mL) was added 4-nitrophenyl quinuclidin-3-yl carbonate (0.571 g, 1.95 mmol) and a few granules of 4-(dimethylamino)pyridine. The mixture was refluxed overnight, concentrated and partitioned between ethyl acetate (50 mL) and aqueous NaHCO₃ (50 mL). The organic layer was washed again with aqueous NaHCO₃ (1 × 50 mL), dried (Na₂SO₄) and concentrated. The resulting dirty yellow gum was purified by flash chromatography using a chloloform/methanol/ammonia gradient to afford quinuclidin-3-yl (1-(5-bromothiophen-3-yl)cyclopropyl)carbamate as an off-white solid (0.305 g, 49%).

Using General Procedure C, quinuclidin-3-yl (1-(5-bromothiophen-3-yl)cyclopropyl)carbamate (0.227 g, 0.742 mmol), 4-fluorophenyl boronic acid (0.208 g, 1.49 mmol), tricyclohexylphosphine (0.021 g, 0.075 mmol), potassium phosphate (0.866, 4.08 mmol) and palladium acetate (8.0 mg, 36 µmol) gave the title compound as a grey solid (0.142 g, 49%). ¹H NMR (400 MHz, CDCl₃) δ 7.60-7.45 (m, 2H), 7.24-7.19 (m, 1H), 7.10-6.97 (m, 3H), 5.23 (br s, 1H), 4.72-4.61 (m, 1H), 3.30-3.04 (m, 1H), 3.03-2.25 (m, 5H), 2.09-1.02 (m, 11H) ppm. ¹³C NMR (400 MHz, CDCl₃) δ 162.3 (d, *J* = 247.1 Hz), 154.5, 149.8, 143.6, 130.7, 127.4 (d, *J* = 8.1 Hz), 121.8, 118.9, 115.8 (d, *J* = 21.6 Hz), 70.8, 55.5, 53.4, 47.3, 46.4, 29.0, 25.4, 24.4, 19.4 ppm. Purity: 95.8 % UPLCMS (210 nm & 254 nm); retention time 0.90 min; (M+1) 389.

### Example 11: (S)-quinuclidin-3-yl 2-(3-(4-fluorophenyl)isothiazol-5-yl)propan-2-vlcarbamate (Compound 11)

To stirred solution of 2-(3-(4-fluorophenyl)isothiazol-5-yl)propan-2-amine (1.21 g, 5.12 mmol) in toluene was added a solution of phosgene in toluene [-1.9 M] (10.8 mL, 20.5 mmol). The reaction was heated at reflux for two hours and then concentrated. The residue was coevaporated with toluene (2 x 15 mL) to afford the crude isocyanate intermediate as golden oil. This material was taken up in toluene (10 mL) and treated with (S)-3-quinuclidinol (0.749 g, 5.89 mmol). The reaction was heated at reflux overnight and concentrated. The residue was purified by flash chromatography using a chloloform/methanol/ammonia gradient to afford the title compound as a white solid (0.971 g, 49%). ¹H NMR (400 MHz, DMSO-*d₆*) δ 8.09-8.00 (m, 2H), 7.87 (br s, 1H), 7.75 (s, 1H), 7.35-7.25 (m, 2H), 4.54-4.45 (m, 1H), 3.14-2.92 (m, 1H), 2.87-2.17 (m, 5H), 1.98-0.98 (m, 11H) ppm ¹³C NMR (400 MHz, DMSO-*d₆*) δ 180.1, 165.6, 162.6 (d, *J* = 246.4 Hz), 154.7, 131.2 (d, *J* = 3.0 Hz), 128.7 (d, *J* = 8.4 Hz), 118.2, 115.7 (d, *J* = 21.8 Hz), 70.6, 55.3, 52.8, 46.9, 45.9, 29.9, 25.2, 24.2, 19.2 ppm Purity: 100 % UPLCMS (210 nm & 254 nm); retention time 0.82 min; (M+1) 390.

### Example 12: (S)-quinuclidin-3-yl 2-(4-(4-fluorophenyl)thiazol-2-yl)propan-2-vlcarbamate (Compound 12)

To a stirred solution of ethyl 3-amino-3-thioxopropanoate (20.00g, 135.9 mmol) in ethanol (120 mL) was added 2-bromo-4'-fluoroacetophenone (29.49 g, 135.9 mmol). The mixture was refluxed for 1 hour, concentrated and partitioned between ethyl acetate (300 mL) and aqueous NaHCO₃ (400 mL). The organic layer was combined with a backextract of the aqueous layer (ethyl acetate, 1 × 100 mL), dried (Na₂SO₄) and concentrated. The resulting light brown solid was purified by flash chromatography using a hexane/ethyl acetate gradient to afford ethyl 2-(4-(4-fluorophenyl)thiazol-2-yl)acetate as an off-white solid (29.92 g, 83%).

To a stirred and cooled (-78 °C) solution of ethyl 2-(4-(4-fluorophenyl)thiazol-2-yl)acetate (10.00 g, 37.69 mmol) in THF (250 mL) was added a solution of potassium t-butoxide in THF [1.0 M] (136 mL, 136 mmol), dropwise over 15 minutes, followed by 18-crown-6 (1.6 mL, 7.5 mmol). After an additional 30 minutes at -78 °C, iodomethane (8.5 mL) was added, dropwise over 5 minutes. The reaction was stirred cold for another 2 hours before pouring into water (450 mL) and extracting with ethyl acetate (2 x 150 mL). The combined extracts were washed with brine (1 × 200 mL), dried (Na₂SO₄) and concentrated. The resulting brown oil was purified by flash chromatography using a hexane/ethyl acetate gradient to afford ethyl 2-(4-(4-fluorophenyl)thiazol-2-yl)-2-methylpropanoate as a pale amber oil (8.64 g, 78%).

To a stirred solution of ethyl 2-(4-(4-fluorophenyl)thiazol-2-yl)-2-methylpropanoate (0.900 g, 3.07 mmol) in 1:1:1 THF/ethanol/water (15 mL) was added lithium hydroxide monohydrate (0.451 g, 10.7 mmol). After overnight stirring, the reaction was concentrated and redissolved in water (80 mL). The solution was washed with ether (1 × 50 mL), acidified with the addition of 1N HCl (15 mL) and extracted with ethyl acetate (2 x 50 mL). The combined extracts were dried (Na₂SO₄) and concentrated to afford 2-(4-(4-fluorophenyl)thiazol-2-yl)-2-methylpropanoic acid as a pale golden solid (0.808 g, 99%).

To stirred and cooled (0 °C) solution of 2-(4-(4-fluorophenyl)thiazol-2-yl)-2-methylpropanoic acid ( 0.784 g, 2.96 mmol) in THF (25 mL) was added triethylamine (0.82 mL, 5.9 mmol) followed by isobutyl chloroformate (0.58 mL, 4.4 mmol). The reaction was stirred cold for another 1 hour before adding a solution of sodium azide (0.385 g, 5.92 mmol) in water (7 mL). The reaction was stirred overnight, allowing the cooling bath to slowly warm to room temperature. The mixture was then diluted with water (100 mL) and extracted with ethyl acetate (2 x 60 mL). The combined extracts were washed with aqueous NaHCO₃ (1 × 150 mL) and brine (1 × 100 mL), dried (Na₂SO₄) and concentrated. After coevaporating with toluene (2 x 30 mL), the resulting off-white solid was taken up in toluene (25 mL) and refluxed for 4 hours. (S)-3-quinuclidinol (0.753 g, 5.92 mmol) was then added and reflux was continued for 3 hours. The reaction was concentrated and the residue was purified by flash chromatography using a chloloform/methanol/ammonia gradient to afford the title compound as a white solid (0.793 g, 69%). ¹H NMR (400 MHz, CDCl₃) δ 7.90-7.81 (m, 2H), 7.32 (s, 1H), 7.14-7.05 (m, 2H), 5.76 (br s, 1H), 4.72-4.65 (m, 1H), 3.26-3.10 (m, 1H), 3.03-2.37 (m, 5H), 2.05-1.23 (m, 11H) ppm. ¹³C NMR (400 MHz, CDCl₃) δ 177.6, 162.6 (d, *J* = 248.4 Hz), 154.8, 153.6, 130.8 (d, *J* = 3.2 Hz), 128.1 (d, *J* = 8.1 Hz), 115.9 (d, *J* = 21.7 Hz), 112.2, 71.6, 55.7, 47.4, 46.5, 29.1, 25.4, 24.7, 19.6 ppm. Purity: 100 % UPLCMS (210 nm & 254 nm); retention time 0.82 min; (M+1) 390.

### Example 13: Ouinuclidin-3-vl (2-(4'-(2-methoxyethoxy)-[1,1'-biphenyl]-4-yl)propan-2-yl)carbamate (Compound 13)

Using General Procedure F and the reaction inputs 2-(4'-(2-methoxyethoxy)-[1,1'-biphenyl]-4-yl)-2-methylpropanoic acid (prepared as described in Example 3) and quinuclidin-3-ol, the title compound was generated as a colourless, glassy solid (23%). NMR data matched that of Example 3. Purity: 100%, 99.1% (210 & 254 nm) UPLCMS; retention time: 0.87 min; (M+H⁺) 439.0.

### Example 14: (S)-quinuclidin-3-yl (2-(3'-(2-methoxyethoxy)-[1,1'-biphenyl]-4-yl)propan-2-yl)carbamate (Compound 14)

Exchanging 4-(2-methoxyethoxy)phenylboronic acid for 3-(2-methoxyethoxy)phenylboronic acid, the reaction sequence outlined in Example 3 was used to prepare 2-(3'-(2-methoxyethoxy)-[1,1'-biphenyl]-4-yl)-2-methylpropanoic acid. This intermediate and quinuclidin-3-ol were reacted according to General Procedure F to generate the title compound as a glassy, colourless solid. ¹H NMR (400 MHz, DMSO-*d₆*) δ 7.63-7.31 (m, 6H), 7.24-7.10 (m, 2H), 6.92 (dd, *J* = 8.2, 1.9 Hz, 1H), 4.51-4.34 (m, 1H), 4.21-4.08 (m, 2H), 3.72-3.64 (m, 2H), 3.32 (s, 3H), 3.09-2.26 (m, 5H), 2.04-1.22 (m, 9H) ppm. ¹³C NMR (100 MHz, DMSO-*d₆*) δ 158.9, 154.6, 147.6, 141.5, 137.6, 129.9, 126.3, 125.2, 118.9, 113.2, 112.5, 70.4, 70.0, 66.9, 58.2, 55.4, 54.2, 46.9, 45.9, 29.4, 25.3, 24.2, 19.2 ppm. Purity: 100%, 100% (210 & 254 nm) UPLCMS; retention time: 0.91 min; 15 (M+H⁺) 439.4.

### Example 15: Ouinuclidin-3-vl (2-(4'-(2-memoxyethoxy)-[1,1'-biphenyl]-3-yl)propan-2-yl)carbamate (Compound 15)

Exchanging ethyl 2-(4-bromophenyl)-2-methylpropanoate for ethyl 2-(3-bromophenyl)-2-methylpropanoate, the reaction sequence outlined in Example 3 was used to prepare 2-(4'-(2-methoxyethoxy)-[1,1'-biphenyl]-3-yl)-2-methylpropanoic acid. This intermediate and quinuclidin-3-ol were reacted according to General Procedure F to generate the title compound as a yellow solid. ¹H NMR (400 MHz, DMSO-*d₆*) δ 7.62-7.20 (m, 7H), 7.03 (*d, J* = 8.7 Hz, 2H), 4.48-4.35 (m, 2H), 4.18-4.08 (m, 2H), 3.72-3.62 (m, 2H), 3.32 (s, 3H), 3.10-2.19 (m, 6H), 2.10-1.10 (m, 11H) ppm. ¹³C NMR (100 MHz, DMSO-*d₆*) δ 158.0, 154.6, 148.8, 139.5, 133.1, 128.5, 127.7, 123.8, 123.2, 122.7, 114.8, 70.4, 69.9, 67.0, 58.2, 55.3, 54.5, 47.0, 45.9, 29.4, 25.3, 24.2, 19.2 ppm. Purity: 97.4%, 94.6% (210 & 254 nm) UPLCMS; retention time: 0.88 min; (M+H⁺) 439.3.

### Example 16: Quinuclidin-3-yl (2-(4'-(3-methoxypropoxy)-[1,1'-biphenyl]-4-yl)propan-2-vl)carbamate (Compound 16)

To a stirred solution of 4-iodophenol (10.05 g, 45.68 mmol) in acetonitrile (100 mL) was added potassium carbonate (6.95 g, 50.2 mmol) and 1-chloro-3-methoxypropane (6.4 mL, 57.1 mmol). The mixture was heated at reflux overnight and then concentrated. The residue was taken up in water and extracted with ethyl acetate. The combined extracts were washed with aqueous sodium bicarbonate solution, dried (Na₂SO₄) and concentrated. The crude material was purified by flash chromatography over silica using a hexane/ethyl acetate eluent to afford 1-iodo-4-(3-methoxypropoxy)benzene as a colourless oil (4.39 g, 33%). This intermediate and ethyl 2-methyl-2-(4-(4,4,5,5-tetramethyl-1,3,2-dioxaborolan-2-yl)phenyl)propanoate were reacted according to General Procedure E to generate ethyl 2-(4'-(3-methoxypropoxy)-[1,1'-biphenyl]-4-yl)-2-methylpropanoate. To a stirred solution of this compound (0.693 g, 1.94 mmol) in 1:1:1 (v/v/v) tetrahydrofuran/ethanol/water (10 mL) was added lithium hydroxide monohydrate (0.326 g, 7.77 mmol). The mixture was heated at reflux overnight and then concentrated. The residue was dissolved in water, treated with 1N hydrochloric acid (10 mL) and extracted with ethyl acetate. The combined organic layers were washed with brine, dried (Na₂SO₄) and concentrated to afford 2-(4'-(3-methoxypropoxy)-[1,1'-biphenyl]-4-yl)-2-methylpropanoic acid as a waxy, off-white solid (0.630 g, 99%). This intermediate and quinuclidin-3-ol were reacted according to General Procedure F to generate the title compound as a glassy, colourless solid (62%). ¹H NMR (400 MHz, DMSO-*d₆*) δ 7.61-7.29 (m, 7H), 7.00 (d, *J*= 8.8 Hz, 2H), 4.47-4.36 (m, 1H), 4.05 (t, *J*= 6.4 Hz, 2H), 3.48 (t, *J*=6.3 Hz, 2H), 3.26 (s, 3H), 3.10-2.25 (m, 6H), 2.04-1.74 (m, 4H), 1.65-1.23 (m, 9H) ppm.¹³C NMR (100 MHz, DMSO-*d*6) δ 158.0, 154.5, 146.7, 137.4, 132.4, 127.5, 125.7, 125.2, 114.8, 69.9, 68.5, 64.6, 57.9, 55.4, 54.2, 46.9, 46.0, 29.4, 29.0, 25.2, 24.1, 19.2 ppm Purity: 97.7%, 98.2% (210 & 254 nm) UPLCMS; retention time: 0.96 min; (M+H⁺) 453.5.

### Example 17: Quinuclidin-3-yl (2-(4'-(hydroxymethyl)-[1,1'-biphenyl]-4-yl)propan-2-yl)carbamate (Compound 17)

Using General Procedure E and the reaction inputs ethyl 2-(4-bromophenyl)-2-methylpropanoate and 4-formylphenylboronic acid, ethyl 2-(4'-formyl-[1,1'-biphenyl]-4-yl)-2-methylpropanoate was prepared as a pale amber solid. This intermediate and quinuclidin-3-ol were reacted according to General Procedure F to generate quinuclidin-3-yl (2-(4'-formyl-[1,1'-biphenyl]-4-yl)propan-2-yl)carbamate as foamy, yellow solid. To a stirred solution of this material (0.755 g, 1.92 mmol) in 2:1 (v/v) tetrahydrofuran/ethanol (15 mL) was added sodium borohydride (0.073 g, 1.93 mmol). After 45 minutes, the reaction was diluted with water and extracted with chloroform. The combined extracts were dried (Na₂SO₄) and concentrated onto silica. Flash chromatography over silica using a chloroform/methanol/ammonia eluent provided the title compound as a white solid (0.323 g, 43%). ¹H NMR (400 MHz, DMSO-*d₆*) δ 7.66-7.29 (m, 9H), 5.18 (t, *J*= 5.7 Hz, 1H), 4.53 (d, *J*= 5.7 Hz, 2H), 4.46-4.37 (m, 1H), 3.11-2.19 (m, 6H), 2.11-1.10 (m, 11H) ppm. ¹³C NMR (100 MHz, DMSO-*d₆*) δ 154.7, 147.3, 141.5, 138.4, 137.7, 127.0, 126.2, 126.1, 125.3, 70.0, 62.6, 55.4, 54.2, 46.9, 45.9, 29.4, 25.3, 24.2, 19.2 ppm Purity: 97.5%, 99.1 % (210 & 254 nm) UPLCMS; retention time: 0.73 min; (M+H⁺) 395.

### Example 18: Ouinuclidin-3-vl (2-(4'-(2-hydroxyethyl)-[1,1'-biphenyl]-4-yl)propan-2-yl)carbamate (Compound 18)

Using General Procedure E and the reaction inputs 1-(2-(benzyloxy)ethyl)-4-bromobenzene and ethyl 2-methyl-2-(4-( 4,4,5,5-tetramethyl-1,3,2-dioxaborolan-2-yl)phenyl)propanoate, ethyl 2-(4'-(2-(benzyloxy)ethyl)-[1,1'-biphenyl]-4-yl)-2-methylpropanoate was prepared as a colourless gum. To a stirred solution of this compound (1.34 g, 3.33 mmol) in 1:1:1 (v/v/v) tetrahydrofuran/ethanol/water (18 mL) was added lithium hydroxide monohydrate (0.698 g, 16.6 mmol). After heating at reflux overnight, the reaction was concentrated and partitioned between water and diethyl ether. The resulting emulsion was extracted repeatedly with 0.2 N aqueous sodium hydroxide solution (5 x 50 mL). The clear portion of the aqueous layer was removed each time. The combined aqueous layers were then treated with 1.0 N hydrochloric acid (80 mL) and the resulting suspension of white solid was extracted with ethyl acetate. The combined organic layers were dried (Na₂SO₄) and concentrated to afford 2-(4'-(2-(benzyloxy)ethyl)-[1,1'-biphenyl]-4-yl)-2-methylpropanoic acid as a white solid (1.20 g, 96%). This compound and quinuclidin-3-ol were reacted according to General Procedure F to generate quinuclidin-3-yl (2-(4'-(2-benzyloxyethyl)-[1,1-biphenyl]-4-yl)propan-2-yl)carbamate. To a stirred solution of this material (0.435 g, 0.806 mmol) in methanol was added 1.0 N hydrochloric acid (1 mL) and 10% palladium on carbon (50% water; 0.087 g). The mixture was cycled between vacuum and a nitrogen purge several times, refilling with hydrogen after the last evacuation. After 1.25 hours the reaction was filtered through Celite and concentrated. The residue was taken up in aqueous sodium carbonate solution and extracted with 4: 1 (v/v) chloroform/isopropanol. The combined extracts were dried (Na₂SO₄) and concentrated onto silica Flash chromatography over silica using a chloroform/methanol/ammonia gradient provided the purified title compound as a colourless solid. ¹H NMR (400 MHz, DMSO-*d₆*) δ 7.85-7.63 (m, 1H), 7.63-7.19 (m, 8H), 4.78-4.62 (m, 2H), 3.71-2.78 (m, 8H), 2.76 (t, *J*= 6.8 Hz, 2H), 2.26-1.96 (m, 2H), 1.96-1.40 (m, 9H) ppm ¹³C NMR (100 MHz, DMSO-*d₆*) δ 153.8, 146.8, 138.7, 137.9, 137.6, 129.4, 126.3, 126.1, 125.3, 66.2, 62.1, 54.4, 52.8, 45.4, 44.5, 38.6, 29.5, 29.2, 24.0, 19.9, 16.6 ppm. Purity: 100%, 100% (210 & 254 nm) UPLCMS; retention time: 0.75 min; (M+H⁺) 409.

### Example 19: Quinuclidin-3-yl (2-(2-(4-(3-methoxypropoxy)phenyl)thiazol-4-yl)propan-2-vl)carbamate (Compound 19)

To a stirred suspension of 4-methoxythiobenzamide (9.99 g, 59.7 mmol) in ethanol (75mL) was added ethyl 4-chloroacetoacetate (8.1 mL, 60 mmol). The mixture was heated at reflux for 4 hours before cooling, adding additional ethyl 4-chloroacetoacetate (0.81 mL, 6.0 mmol) and returning to reflux. After 4 more hours of heating the reaction was concentrated and partitioned between ethyl acetate and aqueous sodium bicarbonate solution. The organic layer was combined with additional ethyl acetate extracts, dried (Na₂SO₄) and concentrated. The crude product was purified by flash chromatography over silica using a hexane/ethyl acetate gradient to afford ethyl 2-(2-(4-methoxyphenyl)thiazol-4-yl)acetate as a pale amber oil (14.51 g, 87%). To a stirred solution of this compound (14.48 g, 52.2 mmol) in *N,N*-dimethylformamide (125 mL) was added sodium hydride (60% dispersion in mineral oil; 6.27 g, 157 mmol), portion wise over 15 minutes. The resulting red suspension was cooled (0 °C) and treated, dropwise over 10 minutes, with iodomethane (9.80 mL, 157 mmol). The cooling bath was removed and the reaction was allowed to stir 4 hours before concentrating and partitioning the residue between ethyl acetate and water. The organic layer was washed twice more with water, dried (Na₂SO₄) and concentrated. The residue was purified by flash chromatography over silica using a hexane/ethyl acetate gradient to afford ethyl 2-(2-(4-methoxyphenyl)thiazol-4-yl)-2-methylpropanoate as a pale amber oil (14.12 g, 89%). To a stirred solution of this intermediate (14.12 g, 46.24 mmol) in methylene chloride (250 mL) was added boron tribromide (11.0 mL, 116 mmol), dropwise over 5 minutes. After stirring overnight, the reaction was quenched by the slow addition of methanol (-20 mL) and then concentrated. The residue was taken up in methanol (250 mL) and concentrated sulfuric acid (7.0 mL). The stirred solution was heated at reflux for 2 hours, concentrated and partitioned between ethyl acetate and aqueous sodium bicarbonate solution. The organic layer was combined with a second ethyl acetate extract of the aqueous layer, dried (Na₂SO₄) and concentrated to afford methyl 2-(2-(4-hydroxyphenyl)thiazol-4-yl)-2-methylpropanoate as a white solid (12.56 g, 98%). To a stirred solution of 1-bromo-3-methoxypropane (1.66 g, 10.8 mmol) in acetone (30 mL) was added the phenol intermediate (2.00 g, 7.21 mmol) and potassium carbonate (1.25 g, 9.04 mmol). The mixture was heated overnight at reflux, filtered and concentrated. The residue was purified by flash chromatography over silica using a hexane/ethyl acetate gradient to afford methyl 2-(2-(4-(3-methoxypropoxy)phenyl)thiazol-4-yl)-2-methylpropanoate as a faint amber gum (2.47 g, 98%). To a stirred solution of this compound (2.45 g, 7.01 mmol) in 1:1:1 (v/v/v) tetrahydrofuran/ethanol/water (45 mL) was added lithium hydroxide monohydrate (1.47 g, 35.0 mmol). After overnight stirring, the reaction was concentrated and partitioned between water and diethyl ether. The aqueous layer was treated with 1.0 N hydrochloric acid (40 mL) and extracted with ethyl acetate. The combined extracts were dried (Na₂SO₄) and concentrated to afford 2-(2-(4-(3-methoxypropoxy)phenyl)thiazol-4-yl)-2-methylpropanoic acid as a white solid (2.19 g, 40 93%). This compound and quinuclidin-3-ol were reacted according to General Procedure F to generate the title compound as a soft, faint amber solid. ¹H NMR (400 MHz, DMSO-*d₆*) δ 7.82 (d, *J* = 8.9 Hz, 2H), 7.36 (br s, 1H), 7.24 (br s, 1H), 7.03 (d, *J* = 8.9 Hz, 2H), 4.49-4.41 (m, 1H), 4.07 (t, *J* = 6.4 Hz, 2H), 3.48 (t, J = 6.4 Hz, 2H), 3.26 (s, 3H), 3.09-2.26 (m, 6H), 2.02-1.91 (m, 2H), 1.91-1.03 (m, 11H) ppm. ¹³C NMR (100 MHz, DMSO-*d₆*) δ 165.8, 162.4, 160.0, 154.6, 127.5, 126.1, 114.9, 112.1, 70.1, 68.4, 64.8, 57.9, 55.4, 53.5, 46.9, 45.9, 28.9, 28.3, 25.2, 24.2, 19.2 ppm. Purity: 100%, 100% (210 & 254 nm) UPLCMS; retention time: 0.87 min; (M+H⁺) 460.

### Example 20: Ouinuclidin-3-yl (2-(2-(4-(2-methoxyethoxy)phenyl)thiazol-4-yl)propan-2-vl)carbamate (Compound 20)

To a stirred solution of 2-bromoethyl methyl ether (1.88 g, 13.5 mmol) in acetone was added methyl 2-(2-(4-hydroxyphenyl)thiazol-4-yl)-2-methylpropanoate (prepared as described in Example 19, 2.00 g, 7.21 mmol) and potassium carbonate (1.56 g, 11.3 mmol). After heating at reflux overnight, the mixture was treated with additional 2-bromo ethyl methyl ether (1.88 g, 13.5 mmol) and potassium carbonate (1.56 g, 11.3 mmol). The reaction was heated at reflux for a second night, filtered and concentrated. The residue was purified by flash chromatography over silica using a hexane/ethyl acetate gradient to afford methyl 2-(2-(4-(2-methoxyethoxy)phenyl)thiazol-4-yl)-2-methylpropanoate as a white solid (2.71 g, 90%). To a stirred solution of this compound (2.71 g, 8.08 mmol) in 1:1:1 (v/v/v) tetrahydrofuran/ethanol/water (50 mL) was added lithium hydroxide monohydrate (1.70 g, 40.5 mmol). After overnight stirring, the reaction was concentrated and partitioned between water and diethyl ether. The aqueous layer was treated with 1.0 N hydrochloric acid (41 mL) and extracted with ethyl acetate. The combined extracts were dried (Na₂SO₄) and concentrated to afford 2-(2-(4-(2-methoxyethoxy)phenyl)thiazol-4-yl)-2-methylpropanoic acid as a white solid (2.57 g, 99%). This compound and quinuclidin-3-ol were reacted according to General Procedure F to generate the title compound as a pale amber solid. ¹H NMR (400 MHz, DMSO-*d₆*) δ 7.82 (d, *J* = 8.8 Hz, 2H), 7.36 (br s, 1H), 7.24 (br s, 1H), 7.04 (d, *J* = 8.8 Hz, 2H), 4.49-4.41 (m, 1H), 4.19-4.12 (m, 2H), 3.71-3.65 (m, 2H), 3.32 (s, 3H), 3.11-2.87 (m, 1H), 2.86-2.19 (m, 5H), 1.92-1.16 (m, 11H) ppm ¹³C NMR (100 MHz, DMSO-*d₆*) δ 165.7, 162.9, 159.9, 154.6, 127.5, 126.2, 114.9, 112.2, 70.3, 70.1, 67.1, 58.2, 55.4, 53.5, 46.9, 45.9, 28.3, 25.2, 24.3, 19.2 ppm. Purity: 100%, 100% (210 & 254 nm) UPLCMS; retention time: 0.85 min; (M+H⁺) 446.

### Example 21: Quinuclidin-3-yl 2-(5-(4-(2-methoxyethoxy)phenyl)pyridin-2-yl)propan-2-vlcarbamate (Compound 21)

Using General Procedure E and the reaction inputs 5-bromopicolinonitrile and 2-(4-(2-methoxyethoxy)phenyl)-4,4,5,5-tetramethyl-1,3,2-dioxaborolane, 5-(4-(2-methoxyethoxy)phenyl)picolinonitrile was prepared. Cercium trichloride (8.05 g, 21.6 mmol) was loaded into a flask and dried by heating (170 °C) under vacuum for 3 hours. The solid was taken up in tetrahydrofuran (20 mL) and stirred vigorously for 30 minutes. The suspension was cooled to -78 °C and treated, dropwise, with a 3.0 M solution of methyllithium in diethyl ether (7.2 mL, 21.6 mmol). Following addition, the reaction was stirred at -78 °C for 1 hour before adding a solution of the above arylborate (1.83 g, 7.20 mmol) in tetrahydrofuran (20 mL). The mixture was maintained at -78 °C for 2 hours and then allowed to warm to room temperature. At this time, the reaction was quenched by the addition of aqueous ammonium hydroxide (10 mL) and filtered through a plug of Celite. The filtrate was extracted with ethyl acetate and the combined extracts were washed with brine, dried (Na₂SO₄) and concentrated. The residue was purified by flash chromatography over silica using ethyl acetate eluent to afford 2-(5-(4-(2-methoxyethoxy)phenyl)pyridin-2-yl)propan-2-amine as a yellow solid (0.800 g, 39%). To a stirred suspension of this intermediate (0.500 g, 1.75 mmol) in water (10 mL) and concentrated hydrochloric acid (0.44 mL) was added toluene (10 mL). The mixture was cooled (0 °C) and treated with, simultaneously over 1 hour, solutions of triphosgene (0.776 g, 2.62 mmol) in toluene (10 mL) and sodium bicarbonate (2.2 g, 26 mmol) in water (20 mL). Following the additions, the reaction was stirred for an additional 30 minutes before the upper toluene layer was removed and dried (N a₂SO₄). At the same time, a stirred solution of quinuclidin-3-ol (0.445 g, 3.64 mmol) in tetrahydrofuran (10 mL) was treated with sodium hydride (60% dispersion in mineral oil; 0.154 g, 3.85 mmol). This mixture was stirred for 5 minutes and then added to the solution of crude isocyanate in toluene. The reaction was stirred for 10 minutes, quenched with the addition of brine (5 mL) and extracted with ethyl acetate. The combined extracts were dried (Na₂SO₄) and concentrated. The residue was purified by flash chromatography over reversed phase silica to afford the title compound as a light yellow solid (0.100 g, 13%). ¹H NMR (500 MHz, CDCl₃) δ 8.70-8.70 (d, *J* = 2.0 Hz, 1H), 7.83-7.81 (m, 1H), 7.49-7.47 (d, *J* = 9.0 Hz, 2H), 7.45-7.43 (d*, J* = 8.0 Hz, 1H), 7.03-7.01 (d*, J* = 8.5 Hz, 2H), 6.63 (br s, 1H), 4.68-4.66 (m, 1H), 4.16 (t, *J* = 5.0 Hz, 2H), 3.77 (t, *J* = 5.0 Hz, 2H), 3.45 (s, 3H), 3.19-2.70 (m, 6H), 2.15-1.89 (m, 2H), 1.76 (s, 6H), 1.73-1.36 (m, 3H) ppm ¹³C NMR (125 MHz, CDCl₃) δ 162.7, 158.9, 154.9, 145.9, 134.8, 134.3, 130.1, 128.1, 11.9.2, 115.2, 71.0, 70.8, 67.4, 59.2, 55.9, 55.7, 47.4, 46.5, 46.4, 27.9, 25.4, 24.6, 19.5 ppm Purity: >99% (214 & 254 nm) LCMS; retention time: 1.32 min; (M+H⁺) 440.2.

### Example 22: Ouinuclidin-3 vl (2-(4'-(3-cyanopropoxy)-[1,1'-biphenyl]-4-yl)propan-2-yl)carbamate (Compound 22)

To a stirred solution of 4-bromophenol (17.1 g, 98.8 mmol) in acetonitrile (150 mL) was added 1-bromobutylnitrile (12.3 mL, 124 mmol) and potassium carbonate (15.0 g, 109 mmol). The mixture was heated to reflux overnight, cooled and concentrated. The residue was taken up in water and extracted with ethyl acetate. The combined extracts were dried (Na₂SO₄) and concentrated and the crude material was purified by flash chromatography over silica using a hexane/ethyl acetate eluent to afford 4-(4-bromophenoxy)butanenitrile as a white solid (20.8 g, 88%). To a stirred solution of this product in *N*,*N-*dimethylformamide (100 mL), was added bis(pinacolato)diboron (4.60 g, 18.1 mmol), potassium acetate (7.41 g, 75.5 mmol) and [1,1'-bis(diphenylphosphino)ferrocene]-dichloropalladium(II) complex with dichloromethane (0.616 g, 1.04 mmol). The mixture was heated to reflux overnight and then concentrated. The residue was taken up in ethyl acetate and washed with water and brine. The organic layer was dried (Na₂SO₄) and concentrated and the crude product was purified by flash chromatography over silica using a hexane/ethyl acetate eluent to afford 4-(4-(4,4,5,5-tetramethyl-1,3,2-dioxaborolan-2-yl)phenoxy)butanenitrile as a white solid (3.43 g, 79%). This product and quinuclidin-3-yl (2-(4-bromophenyl)propan-2-yl)carbamate (prepared by reacting quinuclidin-3-ol and 2-(4-bromophenyl)propan-2-amine using General Procedure F) were reacted according to General Procedure E to generate the title compound as a white solid. ¹H NMR (400 MHz, DMSO-*d₆*) δ 7.67-7.26 (m, 7H), 7.02 (d, *J* = 8.8 Hz, 2H), 4.50-4.33 (m, 1H), 4.08 (t, *J* = 6.0 Hz, 2H), 3.14-2.18 (m, 8H), 2.04 (quin, *J* = 6.7 Hz, 2H), 1.94-1.70 (m, 11H) ppm ¹³C NMR (100 MHz, DMSO-*d₆*) δ 157.7, 154.5, 146.8, 137.4, 132.7, 127.6, 125.7, 125.2, 120.2, 114.9, 70.0, 65.8, 55.4, 54.2, 46.9, 45.9, 29.4, 25.3, 24.7, 24.2, 19.2, 13.4 ppm Purity: 100%, 98.9% (210 & 254 nm) UPLCMS; retention time: 0.88 min; (M+H⁺) 448.6.

### Example 23: Quinuclidin-3-yl (2-(4'-(cyanomethoxy)-[1,1'-biphenyl]-4-yl)propan-2-yl)carbamate (Compound 23)

Using General Procedure E and the reaction inputs quinuclidin-3-yl (2-(4-bromophenyl)propan-2-yl)carbamate (prepared by reacting quinuclidin-3-ol and 2-(4-bromophenyl)propan-2-amine using General Procedure F) and 4-(cyanomethoxy)phenylboronic acid, the title compound was prepared as a pale amber solid. ¹H NMR (400 MHz, DMSO-*d₆*) δ 7.65 (d, *J*= 8.2 Hz, 2H), 7.60-7.31 (m, 5H), 7.15 (d, *J* = 8.9 Hz, 2H), 5.21 (s, 2H), 4.53-4.30 (m, 1H), 3.18-2.19 (m, 6H), 2.05-1.18 (m, 11H) ppm ¹³C NMR (100 MHz, DMSO-*d₆*) δ 155.8, 154.6, 147.2, 137.2, 134.4, 127.8, 126.0, 125.3, 116.7, 115.3, 70.0, 55.4, 54.2, 53.5, 46.9, 45.9, 29.4, 25.2, 24.2, 19.2 ppm. Purity: 100%, 100% (210 & 254 nm) UPLCMS; retention time: 0.85 min; (M+H⁺) 420.3.

### Example 24: Tissue distribution of Compound 1 in a mouse model of proteinopathies

A mouse model has been described (*Gba1*^{*D409V*/*D409V*}) that exhibits progressive accumulation of proteinase K-resistant α-synuclein, ubiquitin and tau aggregates in the central nervous system This is reminiscent of the protein deposits seen, for example, in Lewy neurites in patients with Parkinson's disease and Lewy body dementia. These mice also display a demonstrable hippocampal memory deficit. The distribution of in brain and liver tissue of these mice was investigated following oral administration of **Compound 1.**

### Methods

*Gba1*^{*D409V*/*D409V*} mice (harboring a point mutation at residue 409 in the murine *Gba1* gene) were bred under a protocol approved by the Institutional Animal Care and Use Committee. Treatments were administered as described and the animals were humanely sacrificed at pre-determined time points or upon reaching a humane endpoint.

A subset of *Gba1*^{*D409V*/*D409V*} mice received **Compound 1** administered in food using a formulation calculated to provide 60 mg/kg/day. Drug administration was initiated when pups were weaned at 4 weeks of age and continued until euthanasia at 4 or 10 months of age. The concentration of **Compound 1** in brain and liver tissues was determined by mass spectrometry (see *e.g.* Ramanathan et al. "The emergence of high-resolution MS as the premier analytical tool in the pharmaceutical bioanalysis arena" Bioanalysis. Mar 2012;4(5):467-469).

### Results

Mice fed with a diet containing **Compound 1** demonstrated tissue exposure of 217 ± 12 ng / g tissue in the cortex; and 10512 ± 603 ng / g tissue in the liver, *i.e.* the concentration of **Compound 1** in the brain was approximately 2% of the concentration in the liver. These results demonstrate that **Compound 1** crosses the blood-brain barrier.

### Example 25: Administration of Compound 1 improves memory deficit in Gba1^{D409/D409V} mice

The extent of memory deficit in *Gba1*^{*D409V*/*D409V*} mice was evaluated using novel object recognition (NOR) and fear conditioning (FC) tests.

### Methods

*Gba1*^{*D409V*/*D409V*} mice were bred and treated according to Example 24.

Mice were fed the control diet or the **Compound 1** diet as described in Example 24.

In the NOR test, four month-old wild type (WT) and *Gba1*^{*D409V*/*D409V*} mice were dosed with **Compound 1** starting at 4 weeks of age and were subjected to the NOR test at 3 months (2 months post-treatment).

Mice were individually habituated to explore an open-field box for 5 minutes. During the first training session (T1), two identical objects were symmetrically placed into the open field 14 inches from each other. Animals were allowed to explore for 5 minutes. The number of investigations was recorded by a blinded investigator. After a 24 hour retention period, animals were tested (T2) for their recognition of a novel object. Mice were placed back into the open-field box for 5 minutes, and the time spent investigating the familiar and novel objects was recorded.

Statistical analyses were performed by Student's *t*-test or analysis of variance (ANOVA) followed by Newman-Keuls' post-hoc test. Preference for novelty was defined as investigating the novel object more than 50% of the time by a one-sample t-test. All statistical analyses were performed with GraphPad Prism v4.0 (GraphPad Software, San Diego, CA). Values of p<0.05 were considered significant.

Ten month-old wild type (WT) and *Gba1*^{*D409V*/*D409V*} mice were subjected to FC memory tests. For the FC tests, mice were trained in four of the Med Associates^{®} Near Infrared Fear Conditioning System chambers (St. Albans, VT). Mice were placed in the contextual fear chamber in "Context A," which consists of lighting, a neutral background and a stainless steel grid floor. The mice were trained with a 3-trial delay-cued protocol as defined below. The mice were first given 2 minutes to explore the chamber in Context A before a conditioned stimulus (CS) of a 2000 Hz cue was given. Thirty seconds later a one second unconditioned stimulus (US) of 0.6 mA foot shock was applied. With an inter-trial interval (ITI) of 30 sec, this US-CS response was repeated three times. After a 24 hour retention period, mice were brought back to the testing room and habituated to the room for 1 hour. The mice were again placed in Context A for 5 minutes and freezing to the context was recorded. Freezing (defined as the lack of movement, except for respiration) was recorded using a near-infrared camera system Mice were then removed from the chamber and placed back into their respective cages. After 1 hour, the mice were placed back into the chamber in a novel context, Context B. The mice were allowed to explore the cage for 3 minutes in the novel environment, followed by 3 minutes of the 3-tone auditory cue with the same ITI as the training protocol. Again, freezing to the novel environment and the cue were assessed with the near infrared camera system Contextual memory is defined as the freezing from the training context minus the freezing in the novel context. Cued memory is defined as the freezing to the cue in the novel context.

### Results

Results are of the NOR test are shown in Figure 1. Results are expressed as percentages of target investigations during training (T1) or testing (T2).

In detail, trial 1 (training, solid bars) revealed no object preference when exposed to two similar objects. After a 24 hour retention period, mice were presented with a novel object. In trial 2 (testing, hatched bars), WT mice investigated the novel object significantly more frequently (p<0.05). In contrast, untreated *Gba1*^{*D409V*/*D409V*} mice (middle hatched bar) showed no preference for the novel object, indicating a cognitive impairment. *Gba1*^{*D409V*/*D409V*} mice treated with **Compound 1** (right-hand hatched bar) exhibited a trend to reversal of their memory deficit when presented with the novel object during the testing trial. The results are represented as the means ± the SEM. The horizontal line demarcates 50% target investigations, which represents no preference for either object.

Results are of the FC tests are shown in Figure 2. Figure 2A shows the results relating to contextual memory. Figure 2B shows the results relating to cued memory.

Control *Gba1*^{*D409V*/*D409V*} mice (middle, hatched bars) showed decreased freezing responses in contextual and cued FC testing, confirming the memory impairment. Treatment with **Compound 1** (right-hand, solid bars) improved the freezing responses in the contextual paradigm (Fig. 2A), indicating an improved hippocampal memory. On the other hand, administration of **Compound 1** had no effect on cued memories (Fig. 2B), suggesting the amygdala fear responses are not affected by quinuclidine compounds as described herein.

### Example 26: Administration of Compound 1 improves memory deficit in mice overexpressing A53T α-synuclein

The extent of memory deficit was evaluated in mice overexpressing A53T α-synuclein using novel object recognition (NOR) and fear conditioning (FC) tests. These mice develop α-synuclein inclusions, similar to those observed in humans suffering from α-synucleinopathies, and present with neurodegeneration and severe motor impairment.

### Methods

*PrP-A53T-SNCA* transgenic mice ("A53T" mice) express human A53T-α-synuclein (line M83) under the transcriptional control of the murine PrP promoter (Giasson et al., "Neuronal alpha-synucleinopathy with severe movement disorder in mice expressing A53T human alpha-synuclein" Neuron (2002) 34(4):521-533). The A53T mice were bred and treated substantially as described in Example 24.

Mice were fed the control diet or the **Compound 1** diet as described in Example 24.

The NOR test was performed as described in Example 25, except that the mice were dosed with **Compound 1** at 6 weeks of age and were subjected to the NOR test at 4.5 months.

The FC test was also performed as described in Example 25, except that the mice were subjected to the test at eight months of age.

### Results

Results are of the NOR test are shown in Figure 3. Results are expressed as percentages of target investigations during testing (T2). During training, animals revealed no object preference when exposed to two similar objects (data not shown).

The WT mice investigated the novel object significantly more frequently (left-hand bar, p<0.05). In contrast, A53T mice showed no preference for the novel object, indicating a cognitive impairment (middle, hatched bar). A53T mice treated with **Compound 1** exhibited a trend to reversal of their memory deficit when presented with the novel object during the testing trial (right-hand, solid bar). The results are represented as the means ± the SEM. The horizontal line demarcates 50% target investigations, which represents no preference for either object.

Results are of the FC tests are shown in Figure 4. Figure 4A shows the results relating to contextual memory. Figure 4B shows the results relating to cued memory.

Control A53T mice (middle, hatched bars) showed decreased freezing responses in contextual and cued FC testing, confirming the memory impairment. Treatment with **Compound 1** improved the freezing responses in the contextual paradigm (Fig. 4A, right-hand, solid bar), indicating an improved hippocampal memory. Administration of **Compound 1** had only a marginal effect on cued memories (Fig. 4B, right-hand, solid bar), suggesting the amygdala fear responses are not affected by administration of quinuclidine compounds as described herein. Without wishing to be bound by theory, the inventors postulate that the effects on memory which are observed in these mouse models may be due to a reduction in protein aggregates within neural tissue of the mice concomitant with a reduction in levels of toxic substrates within those cells (quinuclidine compounds as described herein are capable of, for example, reducing intra-cellular levels of sphingolipids which may have an adverse impact on neural tissue).

### Example 27: Administration of Compound 1 reduces protein aggregation in the brain

The ability of quinuclidine compounds as described herein to reduce and/or reverse protein aggregation in the brains of *Gba1*^{*D409V*/*D409V*} mice was assessed.

### Methods

Wild-type (WT) and *Gba1*^{*D409V*/*D409V*} mice were fed the control diet or the **Compound 1** diet as described in Example 24. The accumulation of proteins (ubiquitin, α-synuclein and protein tau) was determined by hippocampal quantification and protein immuoreactivity both with and without treatment with **Compound 1.** Protein levels at 4 weeks of age in *Gba1*^{*D409V*/*D409V*} mice were used as baseline levels; protein levels were measured at 16 and at 40 weeks of age.

For histological analysis, mice were perfused with cold PBS. Brains were removed and post-fixed in 10% (v/v) neutral buffered formalin for 48 hours. Tissues were then placed in 30% sucrose, embedded and sectioned at 20 µm in a cryostat Some tissues were pretreated with proteinase K (1:4 dilution; DAKO, Carpinteria, CA) for 7 minutes at room temperature to expose α-synuclein and other aggregated proteins. Brain sections were blocked with 10% (v/v) serum for 1 hour at room temperature and incubated with the following antibodies: mouse anti-ubiquitin (1:500; Millipore, Billerica, MA), rabbit anti-alpha-synuclein (1:300; Sigma, St. Louis, MO), and mouse anti-tau (1:500, Tau-5, Millipore, Billerica, MA). Brain sections were then incubated for 1 hour at with either a donkey anti-mouse AlexaFluor-488 or donkey anti-rabbit AlexaFluor-555 secondary antibody (1:250 dilution; Invitrogen, Carlsbad, CA). For α-synuclein aggregate quantification, a cyanine 3-tyramide signal amplification kit was used (PerkinElmer, Waltham, MA). Nuclei were stained with DAPI (Sigma, St. Louis, MO). Sections were cover-slipped with aqua poly/mount (Polysciences, Warrington, PA).

For morphometric analysis, sections were imaged with a SPOT camera (Diagnostic Instruments, Sterling Heights, MI) paired with a Nikon Eclipse E800 fluorescence microscope equipped with a 20X objective lens. The stratum radiatum, external to the CA1 hippocampal cell layer, was imaged for each animal. Two or three sections were imaged per animal. All images were exposure-matched for Metamorph analysis (Molecular Devices, Sunnyvale, CA). For ubiquitin and tau quantification, the same threshold was used for all images such that the aggregates were positively counted and the threshold area was recorded for each image in pixels. For α-synuclein quantification, each image was analyzed individually using a range of threshold values to quantify the area of the aggregates accurately and eliminate variable background signals. These procedures were performed blind to genotype or treatment. The percent threshold area was calculated and expressed as mean ± SEM.

### Results

Hippocampal quantification of ubiquitin aggregates is shown in Figures 5A (16 weeks of age, n≥5 per group) and 5B (40 weeks of age, n≥8 per group). The results are represented as the means ± the SEM. Bars with different letters are significantly different from each other (p<0.05). The images in Figure 6 show ubiquitin immunoreactivity (green) in the hippocampi of 40 week-old *Gba1*^{*D409V*/*D409V*} mice control (Fig. 6A) or treated with **Compound 1** (Fig. 6B). DAPI nuclear staining is shown in blue.

Hippocampal quantification of proteinase K-resistant α-synuclein aggregates is shown in Figures 7A (16 weeks of age, n≥5 per group) and 7B (40 weeks of age, n>8 per group). The results are represented as the means ± the SEM. Bars with different letters are significantly different from each other (p<0.05). The images in Figure 8 show proteinase K-resistant α-synuclein immunoreactivity (red) in the hippocampi of 40 week-old *Gba1*^{*D409V*/*D409V*} mice control (Fig. 8A) or treated with **Compound 1** (Fig. 8B). DAPI nuclear staining is shown in blue.

Hippocampal quantification of protein tau aggregates is shown in Figures 9A (16 weeks of age, n≥5 per group) and 9B (40 weeks of age, n>8 per group). The results are represented as the means ± the SEM. Bars with different letters are significantly different from each other (p<0.05). The images in Figure 10 show tau immunoreactivity (green) in the hippocampi of 40 week-old *Gba1*^{*D409V*/*D409V*} mice control (Fig. 10A) or treated with **Compound 1** (Fig. 10B). DAPI nuclear staining is shown in blue.

The *Gba1*^{*D409V*/*D409V*} mice accumulate ubiquitin, α-synuclein and protein tau aggregates from 4 to 40 weeks of age. Treatment with quinuclidine compounds as described herein, (i) blocked the accumulation of ubiquitin aggregates at 40 weeks of age, reducing its levels to wild type controls; (ii) reduced the accumulation of α-synuclein aggregates at 40 weeks of age; and (iii) blocked the accumulation of tau aggregates at 40 weeks of age. Trends to these results were also observed at 16 weeks of age.

The results presented herein show the effects of quinuclidine compounds as described herein on neuronal α-synuclein and protein tau processing *in vivo* and demonstrate the therapeutic potential of administering quinuclidine compounds as described herein for treating proteinopathies.

### Example 28: Preparation of (S)-Quinuclidin-3-yl (2-(2-(4-fluorophenyl)thiazol-4-yl)propan-2-yl)carbamate free base

### Step 1: Dimethylation with methyl iodide

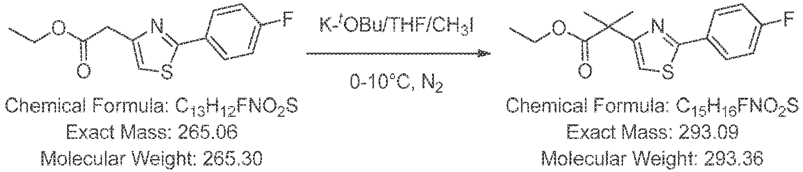

A 3N RB flask was equipped with a thermometer, an addition funnel and a nitrogen inlet. The flask was flushed with nitrogen and potassium tert-butoxide (MW 112.21, 75.4 mmol, 8.46 g, 4.0 equiv., white powder) was weighed out and added to the flask via a powder funnel followed by the addition of THF (60 mL). Most of the potassium tert-butoxide dissolved to give a cloudy solution. This mixture was cooled in an ice-water bath to 0-2°C (internal temperature). In a separate flask, the starting ester (MW 265.3, 18.85 mmol, 5.0 g, 1.0 equiv.) was dissolved in THF (18 mL + 2 mL as rinse) and transferred to the addition funnel. This solution was added dropwise to the cooled mixture over a period of 25-30 min, keeping the internal temperature below 5°C during the addition. The reaction mixture was cooled back to 0-2°C. In a separate flask, a solution of methyl iodide (MW 141.94, 47.13 mmol, 6.7 g, 2.5 equiv.) in THF (6 mL) was prepared and transferred to the addition funnel. The flask containing the methyl iodide solution was then rinsed with THF (1.5 mL) which was then transferred to the addition funnel already containing the clear colorless solution of methyl iodide in THF. This solution was added carefully dropwise to the dark brown reaction mixture over a period of 30-40 min, keeping the internal temperature below 10°C at all times during the addition. After the addition was complete, the slightly turbid mixture was stirred for an additional 1 h during which time the internal temperature dropped to 0-5°C. After stirring for an hour at 0-5°C, the reaction mixture was quenched with the slow dropwise addition of 5.0M aqueous HCl (8 mL) over a period of 5-7 min. The internal temperature was maintained below 20°C during this addition. After the addition, water (14 mL) was added and the mixture was stirred for 2-3 min. The stirring was stopped and the two layers were allowed to separate. The two layers were then transferred to a 250 mL 1N RB flask and the THF was evaporated *in vacuo* as much as possible to obtain a biphasic layer of THF/product and water. The two layers were allowed to separate. A THF solution of the Step1 product was used in the next reaction.

### Step 2: Hydrolysis of the ethyl ester with LiOH monohydrate

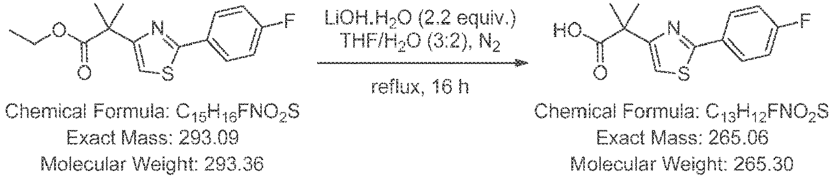

The crude ester in THF was added to the reaction flask. Separately, LiOH.H₂O (MW 41.96, 75.0 mmol, 3.15 grams, 2.2 equiv.) was weighed out in a 100 mL beaker to which a stir bar was added. Water (40 mL) was added and the mixture was stirred till all the solid dissolved to give a clear colorless solution. This aqueous solution was then added to the 250 mL RB flask containing the solution of the ester in tetrahydrofuran (THF). A condenser was attached to the neck of the flask and a nitrogen inlet was attached at the top of the condenser. The mixture was heated at reflux for 16 hours. After 16 hours, the heating was stopped and the mixture was cooled to room temperature. The THF was evaporated *in vacuo* to obtain a brown solution. An aliquot of the brown aqueous solution was analyzed by HPLC and LC/MS for complete hydrolysis of the ethyl ester. Water (15 mL) was added and this aqueous basic solution was extracted with TBME (2 x 40 mL) to remove the t-butyl ester. The aqueous basic layer was cooled in an ice-water bath to 0-10°C and acidified with dropwise addition of concentrated HCl to pH - 1 with stirring. To this gummy solid in the aqueous acidic solution was added TBME (60 mL) and the mixture was shaken and then stirred vigorously to dissolve all the acid into the TBME layer. The two layers were transferred to a separatory funnel and the TBME layer was separated out. The pale yellow aqueous acidic solution was re-extracted with TBME (40 mL) and the TBME layer was separated and combined with the previous TBME layer. The aqueous acidic layer was discarded. The combined TBME layers are dried over anhydrous Na₂SO₄, filtered and evaporated *in vacuo* to remove TBME and obtain the crude acid as an orange/dark yellow oil that solidified under high vacuum to a dirty yellow colored solid. The crude acid was weighed out and crystallized by heating it in heptane/TBME (3:1, 5 mL/g of crude) to give the acid as a yellow solid.

### Step 3: Formation of hydroxamic acid with NH₂OH.HCl

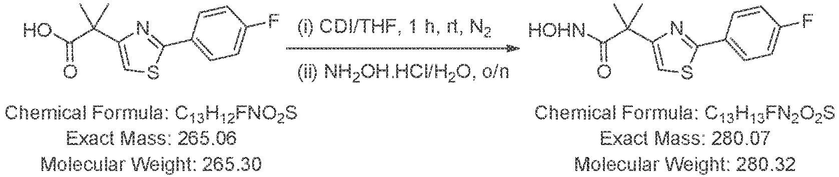

The carboxylic acid (MW 265.3, 18.85 mmol, 5.0 g, 1.0 equiv.) was weighed and transferred to a 25 mL 1N RB flask under nitrogen. THF (5.0 mL) was added and the acid readily dissolved to give a clear dark yellow to brown solution. The solution was cooled to 0-2°C (bath temperature) in an ice-bath and N, N'-carbonyldiimidazole (CDI; MW 162.15, 20.74 mmol, 3.36 g, 1.1 equiv.) was added slowly in small portions over a period of 10-15 minutes. The ice-bath was removed and the solution was stirred at room temperature for 1 h. After 1 h of stirring, the solution was again cooled in an ice-water bath to 0-2°C (bath temperature). Hydroxylamine hydrochloride (NH₂OH.HCl; MW 69.49, 37.7 mmol, 2.62 g, 2.0 equiv.) was added slowly in small portions as a solid over a period of 3-5 minutes as this addition was exothermic. After the addition was complete, water (1.0 mL) was added to the heterogeneous mixture dropwise over a period of 2 minutes and the reaction mixture was stirred at 0-10°C in the ice-water bath for 5 minutes. The cooling bath was removed and the reaction mixture was stirred under nitrogen at room temperature overnight for 20-22 h. The solution became clear as all of the NH₂OH.HCl dissolved. After 20-22 h, an aliquot of the reaction mixture was analyzed by High Pressure Liquid Chromatography (HPLC). The THF was then evaporated in *vacuo* and the residue was taken up in dichloromethane (120 mL) and water (60 mL). The mixture was transferred to a separatory funnel where it was shaken and the two layers allowed to separate. The water layer was discarded and the dichloromethane layer was washed with 1N hydrochloride (HCl; 60 mL). The acid layer was discarded. The dichloromethane layer was dried over anhydrous Na₂SO₄, filtered and the solvent evaporated *in vacuo* to obtain the crude hydroxamic acid as a pale yellow solid that was dried under high vacuum overnight.

### Step 3 continued: Conversion of hydroxamic acid to cyclic intermediate (not isolated)

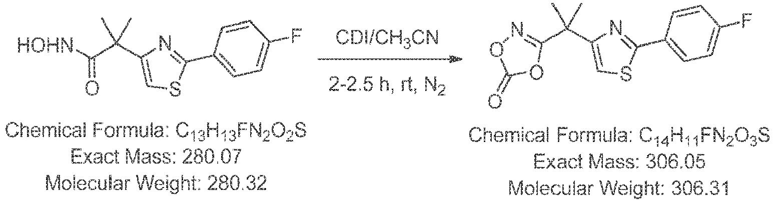

The crude hydroxamic acid (MW 280.32, 5.1 g) was transferred to a 250 mL 1N RB flask with a nitrogen inlet. A stir bar was added followed by the addition of acetonitrile (50 mL). The solid was insoluble in acetonitrile. The yellow heterogeneous mixture was stirred for 2-3 minutes under nitrogen and CDI (MW 162.15, 20.74 mmol, 3.36 g, 1.1 equiv.) was added in a single portion at room temperature. No exotherm was observed. The solid immediately dissolved and the clear yellow solution was stirred at room temperature for 2-2.5 h. After 2-2.5 h, an aliquot was analyzed by HPLC and LC/MS which showed conversion of the hydroxamic acid to the desired cyclic intermediate.

The acetonitrile was then evaporated *in vacuo* to give the crude cyclic intermediate as reddish thick oil. The oil was taken up in toluene (60 mL) and the reddish mixture was heated to reflux for 2 hours during which time, the cyclic intermediate released CO₂ and rearranged to the isocyanate (see below).

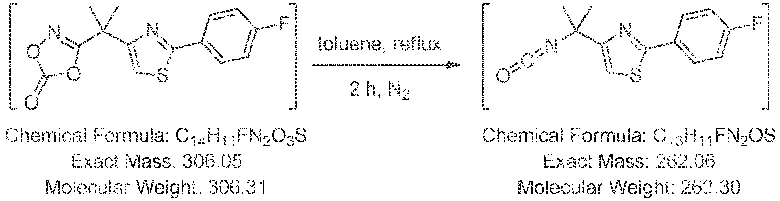

### Step 3 continued : Conversion of the isocyanate to the free base

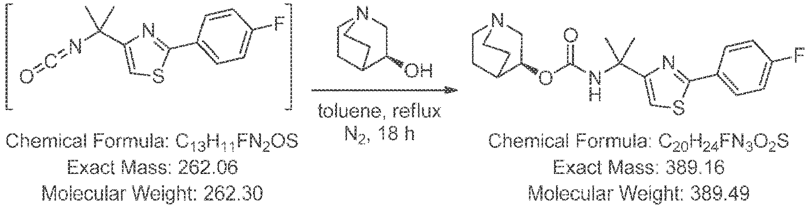

The reaction mixture was cooled to 50-60°C and (S)-(+)-quinuclidinol (MW 127.18, 28.28 mmol, 3.6 g, 1.5 equiv.) was added to the mixture as a solid in a single portion. The mixture was re-heated to reflux for 18 h. After 18 h, an aliquot was analyzed by HPLC and LC/MS which showed complete conversion of the isocyanate to the desired product. The reaction mixture was transferred to a separatory funnel and toluene (25 mL) was added. The mixture was washed with water (2 x 40 mL) and the water layers were separated. The combined water layers were re-extracted with toluene (30 mL) and the water layer was discarded. The combined toluene layers were extracted with 1*N* HCl (2 x 60 mL) and the toluene layer (containing the O-acyl impurity) was discarded. The combined HCl layers were transferred to a 500 mL Erlenmeyer flask equipped with a stir bar. This stirring clear yellow/reddish orange solution was basified to pH 10-12 by the dropwise addition of 50% w/w aqueous NaOH. The desired free base precipitated out of solution as a dirty yellow gummy solid which could trap the stir bar. To this mixture was added isopropyl acetate (100 mL) and the mixture was stirred vigorously for 5 minutes when the gummy solid went into isopropyl acetate. The stirring was stopped and the two layers were allowed to separate. The yellow isopropyl acetate layer was separated and the basic aqueous layer was re-extracted with isopropyl acetate (30 mL). The basic aqueous layer was discarded and the combined isopropyl acetate layers were dried over anhydrous Na₂SO₄, filtered into a pre-weighed RB flask and the solvent evaporated *in vacuo* to obtain the crude free base as beige to tan solid that was dried under high vacuum overnight.

### Step 3 continued: Recrystallization of the crude free base

The beige to tan colored crude free base was weighed and re-crystallized from heptane/isopropyl acetate (3:1, 9.0 mL of solvent/g of crude free base). The appropriate amount of heptane/isopropyl acetate was added to the crude free base along with a stir bar and the mixture was heated to reflux for 10 min (free base was initially partially soluble but dissolved to give a clear reddish orange solution when heated to reflux). The heat source was removed and the mixture was allowed to cool to room temperature with stirring when a white precipitate formed. After stirring at room temperature for 3-4 h, the precipitate was filtered off under hose vacuum using a Buchner funnel, washed with heptane (20 mL) and dried under hose vacuum on the Buchner funnel overnight. The precipitate was the transferred to a crystallizing dish and dried at 55°C overnight in a vacuum oven. ¹H NMR (400 MHz, CDCl₃) δ 8.04 - 7.83 (m, 2H), 7.20 - 6.99 (m, 3H), 5.53 (s, 1H), 4.73 - 4.55 (m, 1H), 3.18 (dd, *J* = 14.5, 8.4 Hz, 1H), 3.05 - 2.19 (m, 5H), 2.0 - 1.76 (m, 11H) ppm ¹³C NMR (100 MHz, CDCl₃) δ 166.38, 165.02, 162.54, 162.8-155.0 (d, C-F), 130.06, 128.43, 128.34, 116.01, 115.79, 112.46, 71.18, 55.70, 54.13, 47.42, 46.52, 27.94, 25.41, 24.67, 19.58 ppm

### Example 29: Preparation of crystalline forms of (S)-Quinuclidin-3-yl (2-(2-(4-fluorophenyl)thiazol-4-yl)propan-2-yl)carbamate salts

Crystalline salts of (*S*)-Quinuclidin-3-yl (2-(2-(4-fluorophenyl)thiazol-4-yl)propan-2-yl)carbamate may be formed from the free base prepared as described in Example 28.

For example, the free base of (*S*)-Quinuclidin-3-yl (2-(2-(4-fluorophenyl)thiazol-4-yl)propan-2-yl)carbamate (about 50 mmol) is dissolved IPA (140 ml) at room temperature and filtered. The filtrate is added into a 1 L r.b. flask which is equipped with an overhead stirrer and nitrogen in/outlet. L-malic acid (about 50 mmol) is dissolved in IPA (100 + 30 ml) at room temperature and filtered. The filtrate is added into the above 1 Liter flask. The resulting solution is stirred at room temperature (with or without seeding) under nitrogen for 4 to 24 hours. During this period of time crystals form. The product is collected by filtration and washed with a small amount of IPA (30 ml). The crystalline solid is dried in a vacuum oven at 55 °C for 72 hours to yield the desired malate salt.

Crystal forms of other salts, *e*.*g*. acid addition salts with succinic acid or HCl, may be prepared in an analogous manner.

### Example 30: Administration of Compound 1 affects the subcellular localization of α-synuclein in the brain of A53T mice

The ability of quinuclidine compounds as described herein to affect the subcellular localization of α-synuclein in the brains of A53T mice was assessed.

### Methods

*PrP-A53T-SNCA* transgenic mice ("A53T" mice) were bred and treated as described in Example 26, being dosed with **Compound 1** starting at 6 weeks of age until euthanasia at 8 months of age.

Mice were fed the control diet or the **Compound 1** diet as described in Example 24.

Cortical tissue homogenates from control and treated A53T mice were subjected to serial fractionation to separate soluble cytosolic (Tris-soluble), membrane-associated (Triton-soluble), and insoluble cytosolic (SDS-soluble) α-synuclein. The concentration of α-synuclein in each fraction was quantified using the human α-synuclein ELISA kit (Biolegend, San Diego, CA). Protein concentrations were determined using the microBCA assay (Thermo Scientific Pierce, Waltham, MA).

### Results

Quantification of α-synuclein in the different fractions is shown in Figure 11.

A small increase in the average level of cytosolic soluble α-synuclein was observed in mice treated with **Compound 1** (Fig. 11A: left-hand bar shows untreated control mice, right-hand bar shows treated mice having 114 ± 8% of the control value; n = 14, *P* = 0.17). The level of membrane-associated α-synuclein was significantly decreased in response to **Compound 1** treatment (Fig. 11B: left-hand bar shows untreated control mice, right-hand bar shows treated mice having 75 ± 8% of the control value, n = 14, *P <* 0.05). The level of insoluble α-synuclein was also significantly decreased in response to **Compound 1** treatment (Fig. 11C: left-hand bar shows untreated control mice, right-hand bar shows treated mice having 81 ± 3% of the control value, n = 14, *P* < 0.01).

These results demonstrate that the administration of a quinuclidine compound as described herein can affect neuronal α-synuclein processing and localization *in vivo* and illustrate the therapeutic potential of the quinuclidine compounds described herein for treating proteinopathies.

### Example 31: Administration of Compound 1 reduces protein aggregation in the brains of A53T mice

The effect of quinuclidine compounds as described herein on protein aggregation in the brains of A53T mice was assessed.

### Methods

A53T mice were bred, treated and fed as described in Example 30. The accumulation of proteins (ubiquitin and protein tau) was determined as described in Example 27, except that protein levels were measured at 6 weeks of age and 8 months of age.

### Results

Hippocampal quantification of ubiquitin aggregates is shown in Figure 12. The results are represented as the means ± the SEM. Bars with different letters are significantly different from each other (*p*<0.05). The far left-hand white bar shows the level of ubiquitin aggregates in the brains of 8 month old wild-type mice. The "Baseline" value shows the protein level at 6 weeks of age in the A53T mice. The black bar, second from right, shows the level of ubiquitin aggregates in the brains of 8 month old untreated A53T mice (control). The right-hand grey bar shows the level of ubiquitin aggregates in the brains of 8 month old A53T mice treated with **Compound 1.**

The images in Figure 13 show ubiquitin immunoreactivity in the hippocampi of 8 month old A53T mice, either untreated control mice (Fig. 13A) or mice treated with **Compound 1** (Fig. 13B).

Hippocampal quantification of protein tau aggregates is shown in Figure14. The results are represented as the means ± the SEM. Bars with different letters are significantly different from each other (*p*<0.05). The far left-hand white bar shows the level of protein tau aggregates in the brains of 8 month old wild-type mice. The "Baseline" value shows the protein level at 6 weeks of age in the A53T mice. The black bar, second from right, shows the level of protein tau aggregates in the brains of 8 month old untreated A53T mice (control). The right-hand grey bar shows the level of protein tau aggregates in the brains of 8 month old A53T mice treated with **Compound 1.**

The images in Figure 15 show tau immunoreactivity in the hippocampi of 8 month old A53T mice, either untreated control mice (Fig. 15A) or mice treated with **Compound 1** (Fig. 15B).

### Thus, treatment with quinuclidine compounds as described herein can reduce the accumulation of protein aggregates in the brains of A53T mice. In particular, a significant reduction in the level of protein tau aggregates is observed in mice treated with

**Compound 1.** These results demonstrate the effects of a quinuclidine compound described herein on neuronal protein processing *in vivo* and suggest that the quinuclidine compounds described herein can disrupt the pathogenic cycle of aberrant protein aggregation and functional deficits associated with proteinopathies. These results illustrate the therapeutic potential of quinuclidine compounds as described herein for treating proteinopathies.

### Example 32: Administration of Compound 1 reverses memory aberrations of post-symptomatic Gba1^{D409V/D409V} mice

The ability of quinuclidine compounds as described herein to correct the biochemical aberrations and memory deficits of symptomatic *Gba1*^{*D409V*/*D409V*} mice was assessed.

### Methods

*Gba1*^{*D409V*/*D409V*} mice were bred and treated according to Example 24. Mice were fed the control diet or the **Compound 1** diet as described in Example 24, except that drug administration was initiated when animals were approximately 6 months of age, and continued until euthanasia at 13 months of age.

Hippocampal memory was evaluated with the NOR test according to Example 25, except that the mice were tested at 6 months of age (before treatment) and again δ months later, at 12 months of age (after treatment).

### Results

Testing of the *Gba1*^{*D409V*/*D409V*} mice before treatment confirmed that they exhibited impairments in novel object recollection (not shown).

Results of the NOR test at 12 months (after treatment) are shown in Figure 16. The results are represented as the means ± the SEM. The horizontal line demarcates 50% target investigations, which represents no preference for either object.

Age-matched wild-type mice (left-hand black bar) investigated the novel object significantly more frequently (n=13, p<0.01). In contrast, untreated *Gba1*^{*D409V*/*D409V*} mice (middle white bar) showed no preference for the novel object, indicating a cognitive impairment. Symptomatic *Gba1*^{*D409V*/*D409V*} mice treated with **Compound 1** (right-hand grey bar) recovered the ability to investigate the unfamiliar object during the testing trial (n=13, p<0.05).

These results demonstrate that administration of quinuclidine compounds as described herein can reverse memory aberrations associated with proteinopathies, even when administration is initiated after symptoms of the proteinopathy are observed.

### Example 33: Administration of Compound 1 reduces protein aggregation in the brains of post-gymptomatic Gba1^{D409V/D409V} mice

The ability of quinuclidine compounds as described herein to reduce and/or reverse protein aggregation in the brains of symptomatic *Gba1*^{*D409V*/*D409V*} mice is assessed.

### Methods

Wild-type (WT) and *Gba1*^{*D409V*/*D409V*} mice are bred and treated substantially as described in Example 27, except that drug administration is initiated when animals are symptomatic for cognitive impairment, *e.g.* at approximately 6 months of age.

The accumulation of proteins (ubiquitin, α-synuclein and protein tau) is determined by hippocampal quantification and protein immunoreactivity substantially as described in Example 27.

### Results

Administration of quinuclidine compounds as described herein, *e.g*. **Compound 1,** is expected to lead to a measurable reduction in the accumulation of protein aggregates (ubiquitin, α-synuclein and/or protein tau) in the brains of *Gba1*^{*D409V*/*D409V*} mice, even when drug administration is initiated after symptoms of cognitive impairment are observed.

### Example 34: Administration of Compound 2 improves memory deficit in Gba1^{D409V/D409V} mice

The ability of quinuclidine compounds as described herein to improve memory deficit in *Gba1*^{*D409V*/*D409V*} mice is evaluated using novel object recognition (NOR) and fear conditioning (FC) tests.

### Methods

*Gba1*^{*D409V*/*D409V*} mice are bred and treated substantially as described in Example 24. Mice are fed a control diet or a diet containing a quinuclidine compound substantially as described in Example 25, except that the compound administered is **Compound 2.**

The NOR test and the FC test are performed substantially as described in Example 25.

### Results

Administration of **Compound 2** is expected to improve memory deficit in *Gba1*^{*D409V*/*D409V*} mice.

### Example 35: Administration of Compound 2 reduces protein aggregation in the brain

The ability of quinuclidine compounds as described herein to reduce and/or reverse protein aggregation in the brains of *Gba1*^{*D409V*/*D409V*} mice is assessed.

### Methods

Wild-type (WT) and *Gba1*^{*D409V*/*D409V*} mice are fed the control diet or a diet containing a quinuclidine compound substantially as described in Example 27, except that the compound administered is **Compound 2.**

The accumulation of proteins (ubiquitin, α-synuclein and protein tau) is determined by hippocampal quantification and protein immuoreactivity as described in Example 27.

### Results

Administration of quinuclidine compounds as described herein, *e.g.* **Compound 2,** is expected to lead to a measurable reduction in the accumulation of protein aggregates (ubiquitin, α-synuclein and/or protein tau) in the brains of *Gba1*^{*D409V*/*D409V*} mice.

It is to be understood that while the invention has been described in conjunction with the above embodiments, that the foregoing description and examples are intended to illustrate and not limit the scope of the invention. Other aspects, advantages and modifications within the scope of the invention, in as far as they are covered by the claims, will be apparent to those skilled in the art to which the invention pertains.

In addition, where features or aspects of the invention are described in terms of Markush groups, those skilled in the art will recognize that the invention is also thereby described in terms of any individual member or subgroup of members of the Markush group.

In case of conflict with publications, patent applications, patents, and other references mentioned herein, the present specification, including definitions, will control.

## Claims

1. A compound of formula (I), or a pharmaceutically acceptable salt thereof, for use in a method of preventing, reducing or reversing loss of neural function in a subject diagnosed as having, or at risk of developing, a proteinopathy, 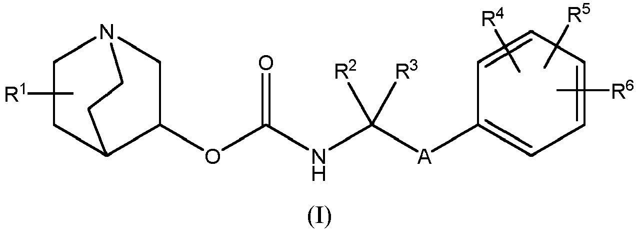 wherein:
R¹ is hydrogen;
a halogen, or a cyano, nitro, hydroxy, thio or amino group; or
a C₁₋₆-alkyl, C₂₋₆-alkenyl, C₂₋₆-alkynyl, C₁₋₆-alkyloxy, C₂₋₆-alkenyloxy or C₂₋₆-alkynyloxy group, optionally substituted by one or more (*e.g.* 1, 2 or 3) groups independently selected from a halogen; and a cyano, nitro, hydroxy, thio, or amino group;
R² and R³ are each independently selected from a C₁₋₃-alkyl group, optionally substituted by one or more halogens; or R² and R³ together form a cyclopropyl or cyclobutyl group, optionally substituted by one or more halogens;
R⁴, R⁵ and R⁶ are each independently selected from hydrogen; a halogen; a nitro, hydroxy, thio or amino group; and a C₁₋₆-alkyl or C₁₋₆-alkyloxy group, optionally substituted by one or more groups selected from a halogen; a hydroxy or cyano group; and a C₁₋₆-alkyloxy group; and
A is a 5- or 6-membered aryl or heteroaryl group,
wherein the loss of neural function comprises loss of cognitive function.

2. The compound or pharmaceutically acceptable salt for use according to claim 1, wherein R¹ is hydrogen; fluorine; or a methyl or ethyl group optionally substituted by a halogen, or a hydroxy, thio or amino group.

3. The compound or pharmaceutically acceptable salt for use according to claim 1 or 2, wherein:
(i) R² and R³ are each independently selected from methyl and ethyl groups, optionally substituted with one or more fluorine atoms;
(ii) R⁴ is selected from a halogen; and a C₁₋₃-alkyl or C₁₋₃-alkyloxy group, optionally substituted by one or more groups selected from a halogen and a C₁₋₃-alkyloxy group; and/or
(iii) R⁵ and R⁶ are both hydrogen.

4. The compound or pharmaceutically acceptable salt for use according to any one of claims 1 to 3, wherein R⁴ is fluorine or a 2-methoxyethoxy group, and R⁵ and R⁶ are hydrogen.

5. The compound or pharmaceutically acceptable salt for use according to any one of claims 1 to 4, wherein R⁴ is in a position on the benzene ring *para* to the group A.

6. The compound or pharmaceutically acceptable salt for use according to any one of claims 1 to 5, wherein A is phenyl, optionally substituted with 1, 2 or 3 groups independently selected from a halogen; and a hydroxy, thio, amino, nitro, oxo or methyl group, optionally wherein the groups -C(R²R³)- and -(C₆H₂R⁴R⁵R⁶) are attached to group A in a 1,3- or a 1,4- relationship.

7. The compound or pharmaceutically acceptable salt for use according to any one of claims 1 to 5, wherein A is a 5-membered heteroaryl group which contains 1 or 2 heteroatoms selected from N and S, optionally wherein the groups -C(R²R³)- and -(C₆H₂R⁴R⁵R⁶) are attached to group A in a 1,3- relationship.

8. The compound or pharmaceutically acceptable salt for use according to any one of claims 1 to 7, wherein said compound or pharmaceutically acceptable salt is a compound of formula (II), (III) or (IV), 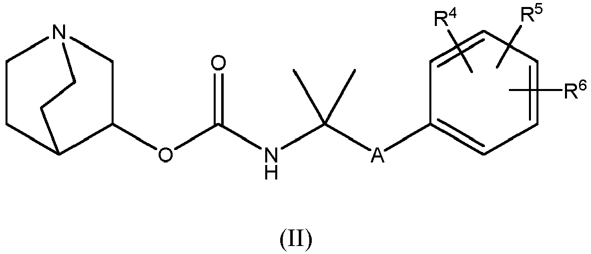 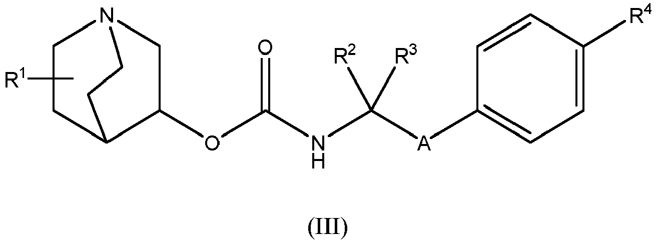 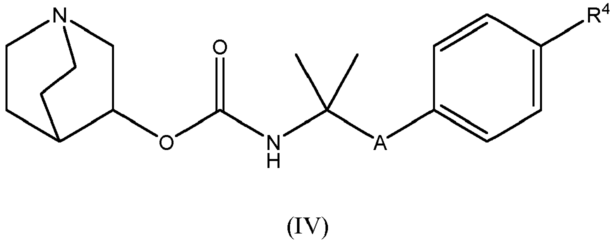 or a pharmaceutically acceptable salt thereof, optionally wherein said compound or pharmaceutically acceptable salt is a compound of formula (V), 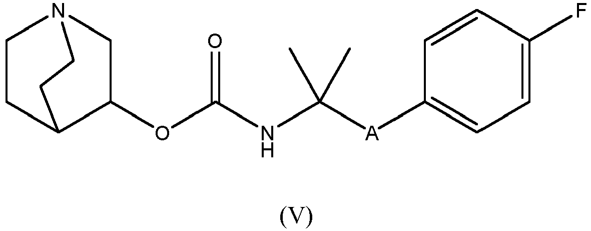 or a pharmaceutically acceptable salt thereof.

9. The compound or pharmaceutically acceptable salt for use according to any one of claims 1 to 7, wherein said compound or pharmaceutically acceptable salt is a compound of formula (VI), (VII) or (VIII), 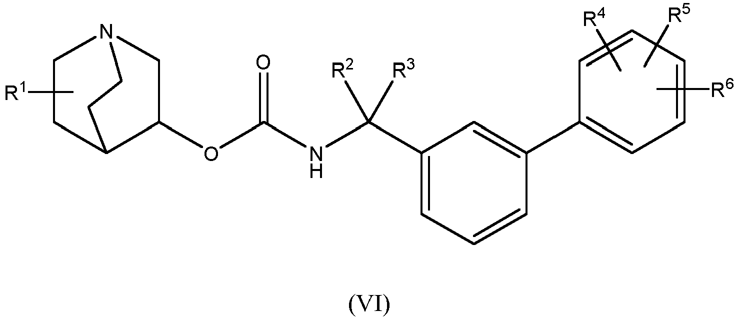 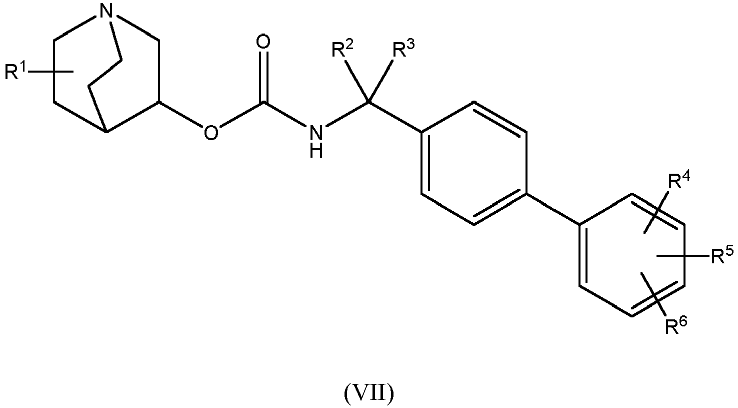 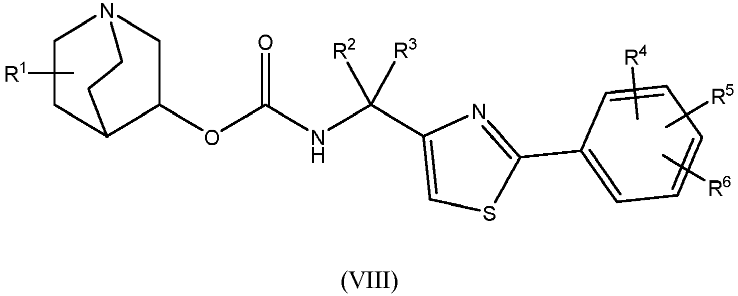 or a pharmaceutically acceptable salt thereof.

10. The compound or pharmaceutically acceptable salt for use according to claim 9, wherein said compound, pharmaceutically acceptable salt is a compound of formula (IX) or (XI), 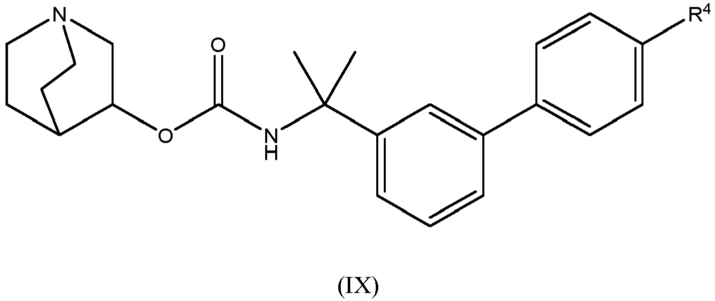 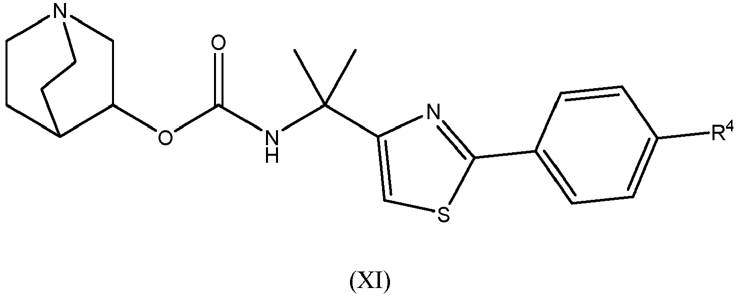 or a pharmaceutically acceptable salt thereof.

11. The compound or pharmaceutically acceptable salt for use according to claim 10, wherein R⁴ is fluorine.

12. The compound or pharmaceutically acceptable salt for use according to claim 1, wherein said compound or pharmaceutically acceptable salt is selected from: quinuclidin-3-yl (2-(4'-fluoro-[1,1'-biphenyl]-3-yl)propan-2-yl)carbamate; (*S*)-quinuclidin-3-yl (2-(2-(4-fluorophenyl)thiazol-4-yl)propan-2-yl)carbamate; (*S*)-quinuclidin-3-yl (2-(4'-(2-methoxyethoxy)-[1,1'-biphenyl]-4-yl)propan-2-yl)carbamate; and the pharmaceutically acceptable salts thereof.

13. The compound or pharmaceutically acceptable salt for use according to any one of claims 1 to 12, wherein said proteinopathy is a tauopathy.

14. The compound or pharmaceutically acceptable salt for use according to claim 13, wherein said tauopathy is selected from Parkinson's disease, Alzheimer's disease, Lewy Body Dementia, Pick's disease, progressive supranuclear palsy, dementia pugilistica, parkinsonism linked to chromosome 17, Lytico-Bodig disease, tangle predominant dementia, Argyrophilic grain disease, ganglioglioma, gangliocytoma, meningioangiomatosis, subacute sclerosing panencephalitis, lead encephalopathy, tuberous sclerosis, Hallervorden-Spatz disease, lipofuscinosis, corticobasal degeneration, frontotemporal dementia, frontotemporal lobar degeneration and Huntington's disease.

15. The compound or pharmaceutically acceptable salt for use according to any one of claims 1 to 12, wherein said proteinopathy is a synucleinopathy.

16. The compound or pharmaceutically acceptable salt for use according to claim 15, wherein said synucleinopathy is selected from Lewy Body Dementia, Parkinson's disease and multiple system atrophy.

## Patentansprüche

1. Verbindung der Formel (I) oder pharmazeutisch unbedenkliches Salz davon zur Verwendung in einem Verfahren zur Prävention, Verminderung oder Umkehr des Verlusts der Nervenfunktion bei einem Subjekt, bei dem eine Proteinopathie diagnostiziert wurde oder das Risiko des Auftretens einer Proteinopathie besteht, 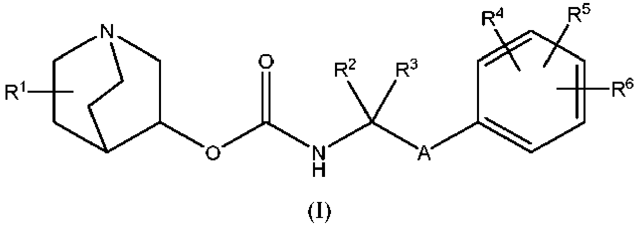 wobei:
R¹ für Wasserstoff,
ein Halogen oder eine Cyano-, Nitro-, Hydroxy-, Thio- oder Aminogruppe oder
eine C₁₋₆-Alkyl-, C₂₋₆-Alkenyl-, C₂₋₆-Alkinyl-, C₁₋₆-Alkyloxy-, C₂₋₆-Alkenyloxy- oder C₂₋₆-Alkinyloxygruppe, gegebenenfalls substituiert durch eine oder mehrere (z. B. 1, 2 oder 3) unabhängig voneinander aus einem Halogen und einer Cyano-, Nitro-, Hydroxy-, Thio- oder Aminogruppe ausgewählte Gruppen, steht,
R² und R³ jeweils unabhängig voneinander aus einer C₁₋₃-Alkylgruppe, gegebenenfalls substituiert durch ein oder mehrere Halogene, ausgewählt sind oder R² und R³ zusammen eine Cyclopropyl- oder Cyclobutylgruppe, gegebenenfalls substituiert durch ein oder mehrere Halogene, bilden, R⁴, R⁵ und R⁶ jeweils unabhängig voneinander aus Wasserstoff, einem Halogen, einer Nitro-, Hydroxy-, Thio- oder Aminogruppe und einer C₁₋₆-Alkyl- oder C₁₋₆-Alkyloxygruppe, gegebenenfalls substituiert durch eine oder mehrere aus einem Halogen, einer Hydroxy- oder Cyanogruppe und einer C₁₋₆-Alkyloxygruppe ausgewählte Gruppen, ausgewählt sind und
A für eine 5- oder 6-gliedrige Aryl- oder Heteroarylgruppe steht,
wobei der Verlust der Nervenfunktion den Verlust der kognitiven Funktion umfasst.

2. Verbindung oder pharmazeutisch unbedenkliches Salz zur Verwendung nach Anspruch 1, wobei R¹ für Wasserstoff, Fluor oder eine Methyl- oder Ethylgruppe, gegebenenfalls substituiert durch ein Halogen oder eine Hydroxy-, Thio- oder Aminogruppe, steht.

3. Verbindung oder pharmazeutisch unbedenkliches Salz zur Verwendung nach Anspruch 1 oder 2, wobei:
(i) R² und R³ jeweils unabhängig voneinander aus Methyl- und Ethylgruppen, gegebenenfalls substituiert durch ein oder mehrere Fluoratome, ausgewählt sind,
(ii) R⁴ aus einem Halogen und einer C₁₋₃-Alkyl- oder C₁₋₃-Alkyloxygruppe, gegebenenfalls substituiert durch ein oder mehrere aus einem Halogen und einer C₁₋₃-Alkyloxygruppe ausgewählte Gruppen, ausgewählt ist und/oder
(iii) R⁵ und R⁶ beide für Wasserstoff stehen.

4. Verbindung oder pharmazeutisch unbedenkliches Salz zur Verwendung nach einem der Ansprüche 1 bis 3, wobei R⁴ für Fluor oder eine 2-Methoxyethoxygruppe steht und R⁵ und R⁶ für Wasserstoff stehen.

5. Verbindung oder pharmazeutisch unbedenkliches Salz zur Verwendung nach einem der Ansprüche 1 bis 4, wobei R⁴ am Benzolring in einer Position *para* zur Gruppe A steht.

6. Verbindung oder pharmazeutisch unbedenkliches Salz zur Verwendung nach einem der Ansprüche 1 bis 5, wobei A für Phenyl, gegebenenfalls substituiert durch 1, 2 oder 3 unabhängig voneinander aus einem Halogen und einer Hydroxy-, Thio-, Amino-, Nitro-, Oxo- oder Methylgruppe ausgewählte Gruppen, steht, wobei die Gruppen -C(R²R³)- und -(C₆H₂R⁴R⁵R⁶) gegebenenfalls in einer 1,3- oder einer 1,4-Beziehung an Gruppe A gebunden sind.

7. Verbindung oder pharmazeutisch unbedenkliches Salz zur Verwendung nach einem der Ansprüche 1 bis 5, wobei A für eine 5-gliedrige Heteroarylgruppe mit 1 oder 2 aus N und S ausgewählten Heteroatomen steht, wobei die Gruppen -C(R²R³)- und -(C₆H₂R⁴R⁵R⁶) gegebenenfalls in einer 1,3-Beziehung an Gruppe A gebunden sind.

8. Verbindung oder pharmazeutisch unbedenkliches Salz zur Verwendung nach einem der Ansprüche 1 bis 7, wobei es sich bei der Verbindung oder dem pharmazeutisch unbedenklichen Salz um eine Verbindung der Formel (II), (III) oder (IV) 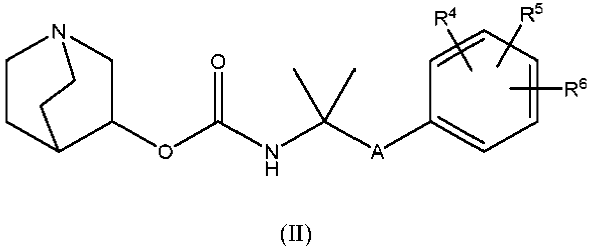 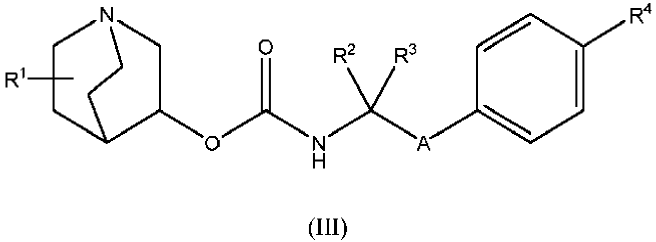 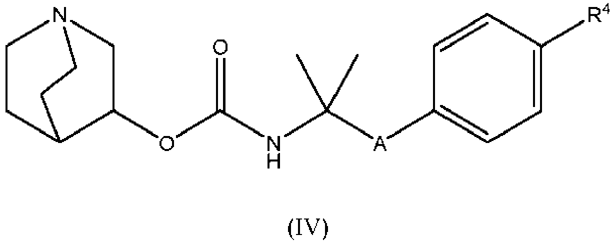 bzw. ein pharmazeutisch unbedenkliches Salz davon handelt, wobei es sich bei der Verbindung oder dem pharmazeutisch unbedenklichen Salz gegebenenfalls um eine Verbindung der Formel (V) 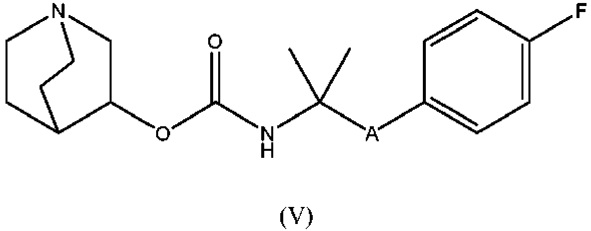 bzw. ein pharmazeutisch unbedenkliches Salz davon handelt.

9. Verbindung oder pharmazeutisch unbedenkliches Salz zur Verwendung nach einem der Ansprüche 1 bis 7, wobei es sich bei der Verbindung oder dem pharmazeutisch unbedenklichen Salz um eine Verbindung der Formel (VI), (VII) oder (VIII) 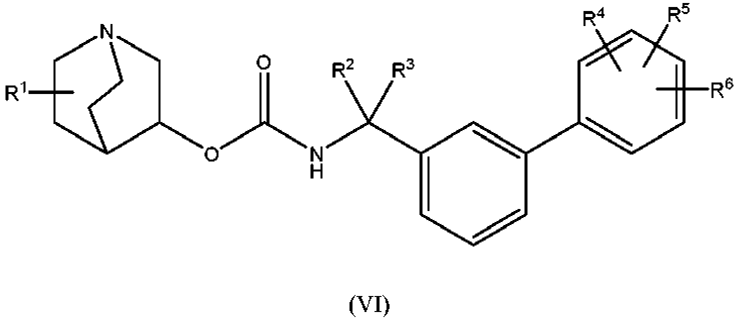 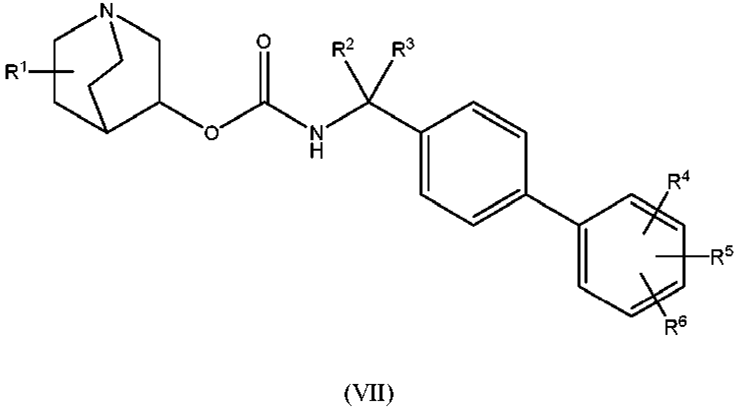 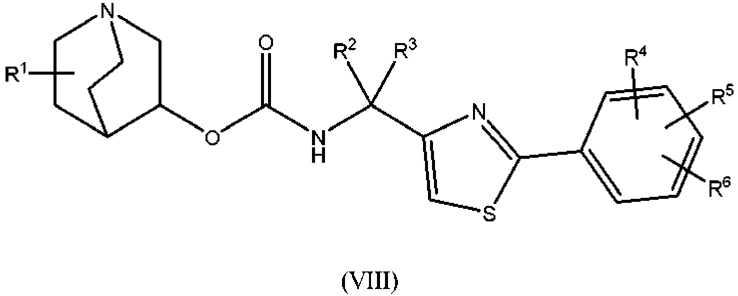 bzw. ein pharmazeutisch unbedenkliches Salz davon handelt.

10. Verbindung oder pharmazeutisch unbedenkliches Salz zur Verwendung nach Anspruch 9, wobei es sich bei der Verbindung, dem pharmazeutisch unbedenklichen Salz um eine Verbindung der Formel (IX) oder (XI) 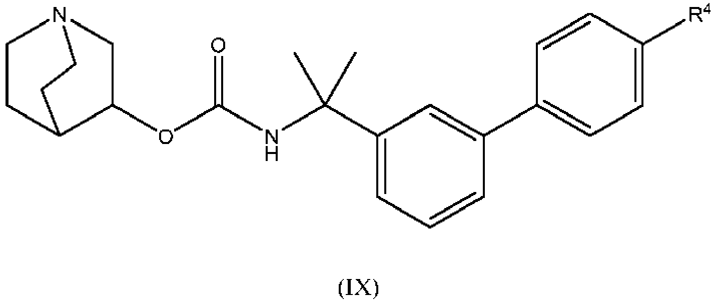 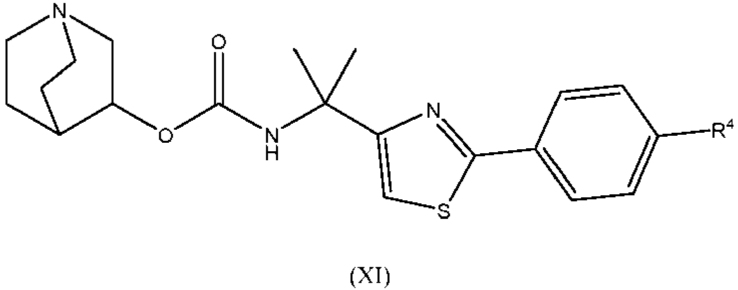 bzw. ein pharmazeutisch unbedenkliches Salz davon handelt.

11. Verbindung oder pharmazeutisch unbedenkliches Salz zur Verwendung nach Anspruch 10, wobei R⁴ für Fluor steht.

12. Verbindung oder pharmazeutisch unbedenkliches Salz zur Verwendung nach Anspruch 1, wobei die Verbindung oder das pharmazeutisch unbedenkliche Salz ausgewählt ist aus: (2-(4'-Fluor-[1,1'-biphenyl]-3-yl)propan-2-yl)carbamidsäurechinuclidin-3-ylester, (2-(2-(4-Fluorphenyl)thiazol-4-yl)propan-2-yl)carbamidsäure-(S)-chinuclidin-3-ylester, (2-(4'-(2-Methoxyethoxy)-[1,1'-biphenyl]-4-yl)propan-2-yl)carbamidsäure-(S)-chinuclidin-3-ylester und den pharmazeutisch unbedenklichen Salzen davon.

13. Verbindung oder pharmazeutisch unbedenkliches Salz zur Verwendung nach einem der Ansprüche 1 bis 12, wobei es sich bei der Proteinopathie um eine Tauopathie handelt.

14. Verbindung oder pharmazeutisch unbedenkliches Salz zur Verwendung nach Anspruch 13, wobei die Tauopathie aus Parkinson-Krankheit, Alzheimer-Krankheit, Lewy-Körper-Demenz, Pick-Krankheit, progressiver supranukleärer Lähmung, Dementia pugilistica, mit Chromosom 17 verbundenem Parkinsonismus, Lytico-Bodig, Tangle-Demenz, Silberkornkrankheit, Gangliogliom, Gangliozytom, Meningioangiomatose, subakuter sklerosierender Panenzephalitis, Bleienzephalopathie, tuberöser Sklerose, Hallervorden-Spatz-Krankheit, Lipofuszinose, kortikobasaler Degeneration, frontotemporaler Demenz, frontotemporaler Lobärdegeneration und Chorea Huntington ausgewählt ist.

15. Verbindung oder pharmazeutisch unbedenkliches Salz zur Verwendung nach einem der Ansprüche 1 bis 12, wobei es sich bei der Proteinopathie um eine Synucleinopathie handelt.

16. Verbindung oder pharmazeutisch unbedenkliches Salz zur Verwendung nach Anspruch 15, wobei die Synucleinopathie aus Lewy-Körper-Demenz, Parkinson-Krankheit und Multisystematrophie ausgewählt ist.

## Revendications

1. Composé de formule (I), ou sel pharmaceutiquement acceptable correspondant, pour une utilisation dans un procédé de prévention, de réduction ou d'inversion de la perte d'une fonction neuronale chez un sujet diagnostiqué comme étant atteint d'une protéinopathie, ou à risque de développer une protéinopathie, 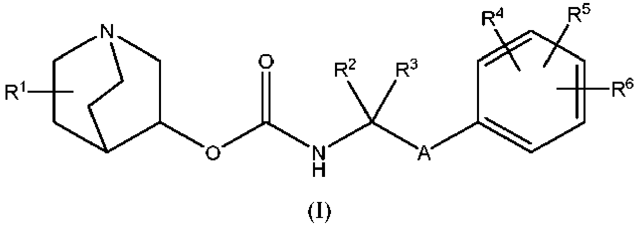
R¹ étant hydrogène ;
un halogène ou un groupe cyano, nitro, hydroxy, thio ou amino ; ou
un groupe C₁₋₆-alkyle, C₂₋₆-alcényle, C₂₋₆-alcynyle, C₁₋₆-alkyloxy, C₂₋₆-alcényloxy ou C₂₋₆-alcynyloxy, éventuellement substitué par un ou plusieurs *(par ex. 1,* 2 ou 3) groupes indépendamment choisis parmi un halogène ; et un groupe cyano, nitro, hydroxy, thio, ou amino ;
R² et R³ étant chacun indépendamment choisis parmi un groupe C₁₋₃-alkyle, éventuellement substitué par un ou plusieurs halogènes ; ou R² et R³ formant ensemble un groupe cyclopropyle ou cyclobutyle, éventuellement substitué par un ou plusieurs halogènes ;
R⁴, R⁵ et R⁶ étant chacun indépendamment choisis parmi hydrogène ; un halogène ; un groupe nitro, hydroxy, thio ou amino ; et un groupe C₁₋₆-alkyle ou C₁₋₆-alkyloxy, éventuellement substitué par un ou plusieurs groupes choisis parmi un halogène ; un groupe hydroxy ou cyano ; et un groupe C₁₋₆-alkyloxy ; et
A étant un groupe aryle ou hétéroaryle à 5 ou 6 chaînons, la perte d'une fonction neuronale comprenant la perte d'une fonction cognitive.

2. Composé ou sel pharmaceutiquement acceptable pour une utilisation selon la revendication 1, R¹ étant hydrogène ; fluor ; ou un groupe méthyle ou éthyle éventuellement substitué par un halogène, ou un groupe hydroxy, thio ou amino.

3. Composé ou sel pharmaceutiquement acceptable pour une utilisation selon la revendication 1 ou 2,
(i) R² et R³ étant chacun indépendamment choisis parmi des groupes méthyle et éthyle, éventuellement substitués par un ou plusieurs atomes de fluor ;
(ii) R⁴ étant choisi parmi un halogène ; et un groupe C₁₋₃-alkyle ou C₁₋₃-alkyloxy, éventuellement substitué par un ou plusieurs groupes choisis parmi un halogène et un groupe C₁₋₃-alkyloxy ; et/ou
(iii) R⁵ et R⁶ étant tous deux hydrogène.

4. Composé ou sel pharmaceutiquement acceptable pour une utilisation selon l'une quelconque des revendications 1 à 3, R⁴ étant fluor ou un groupe 2-méthoxyéthoxy, et R⁵ et R⁶ étant hydrogène.

5. Composé ou sel pharmaceutiquement acceptable pour une utilisation selon l'une quelconque des revendications 1 à 4, R⁴ étant dans une position sur le cycle benzénique *para* par rapport au groupe A.

6. Composé ou sel pharmaceutiquement acceptable pour une utilisation selon l'une quelconque des revendications 1 à 5, A étant phényle, éventuellement substitué par 1, 2 ou 3 groupes indépendamment choisis parmi un halogène ; et un groupe hydroxy, thio, amino, nitro, oxo ou méthyle, éventuellement les groupes -C(R²R³)- et -(C₆H₂R⁴R⁵R⁶) étant fixés au groupe A en une relation 1,3 ou une relation 1,4.

7. Composé ou sel pharmaceutiquement acceptable pour une utilisation selon l'une quelconque des revendications 1 à 5, A étant un groupe hétéroaryle à 5 chaînons qui contient 1 ou 2 hétéroatomes choisis parmi N et S, éventuellement les groupes -C(R²R³)- et -(C₆H₂R⁴R⁵R⁶) étant fixés au groupe A en une relation 1,3.

8. Composé ou sel pharmaceutiquement acceptable pour une utilisation selon l'une quelconque des revendications 1 à 7, ledit composé ou sel pharmaceutiquement acceptable étant un composé de formule (II), (III) ou (IV), 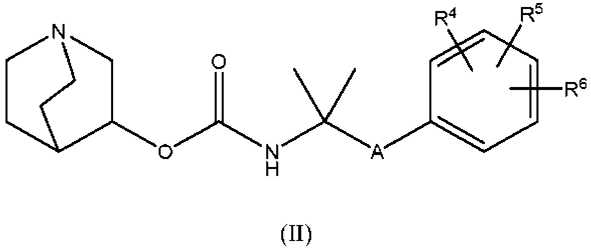 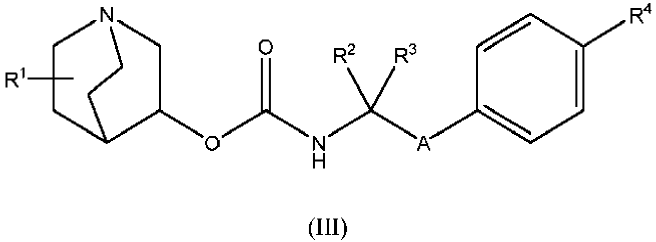 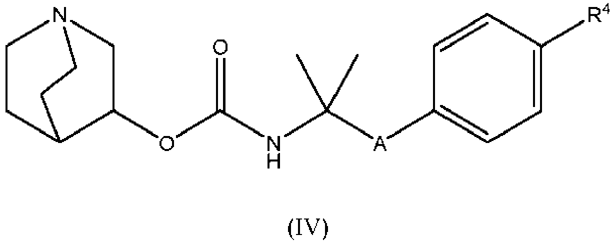 ou un sel pharmaceutiquement acceptable correspondant, éventuellement ledit composé ou sel pharmaceutiquement acceptable étant un composé de formule (V), 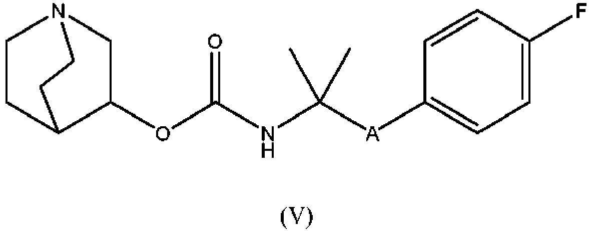 ou un sel pharmaceutiquement acceptable correspondant.

9. Composé ou sel pharmaceutiquement acceptable pour une utilisation selon l'une quelconque des revendications 1 à 7, ledit composé ou sel pharmaceutiquement acceptable étant un composé de formule (VI), (VII) ou (VIII), 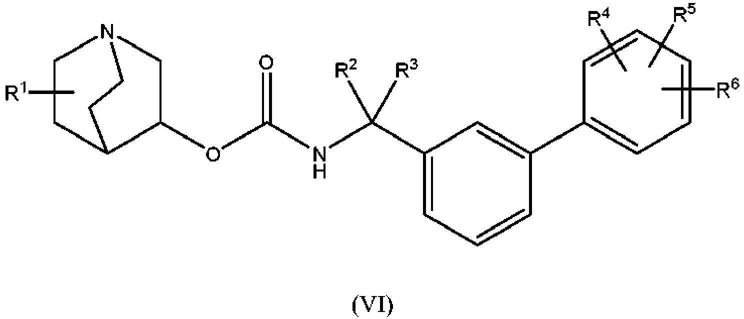 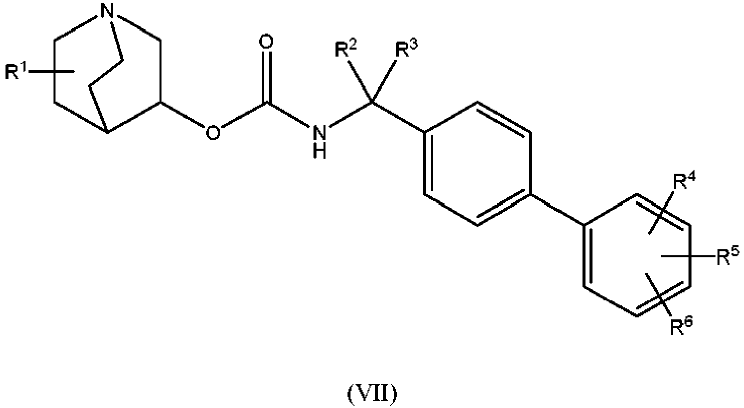 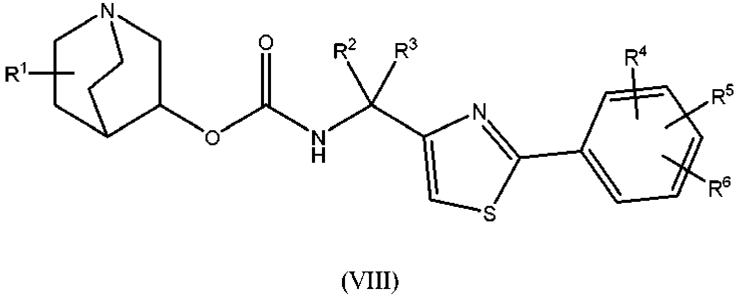 ou un sel pharmaceutiquement acceptable correspondant.

10. Composé ou sel pharmaceutiquement acceptable pour une utilisation selon la revendication 9, ledit composé ou sel pharmaceutiquement acceptable étant un composé de formule (IX) ou (XI), 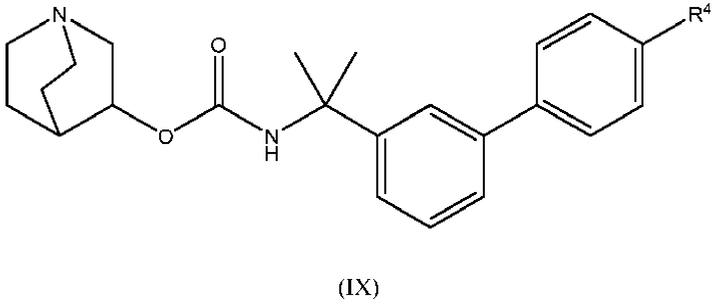 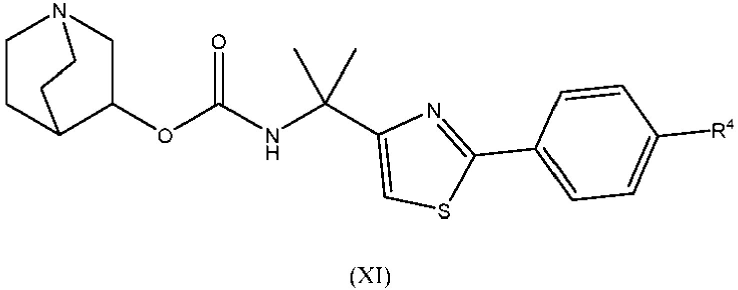 ou un sel pharmaceutiquement acceptable correspondant.

11. Composé ou sel pharmaceutiquement acceptable pour une utilisation selon la revendication 10, R⁴ étant fluor.

12. Composé ou sel pharmaceutiquement acceptable pour une utilisation selon la revendication 1, ledit composé ou sel pharmaceutiquement acceptable étant choisi parmi : (2-(4'-fluoro-[1,1'-biphényl]-3-yl)propan-2-yl)carbamate de quinuclidin-3-yle ; (2-(2-(4-fluorophényl)thiazol-4-yl)propan-2-yl)carbamate de (S)-quinuclidin-3-yle ; (2-(4'-(2-méthoxyéthoxy)-[1,1'-biphényl]-4-yl)propan-2-yl)carbamate de (S)-quinuclidin-3-yle ; et les sels pharmaceutiquement acceptables correspondants.

13. Composé ou sel pharmaceutiquement acceptable pour une utilisation selon l'une quelconque des revendications 1 à 12, ladite protéoinopathie étant une tauopathie.

14. Composé ou sel pharmaceutiquement acceptable pour une utilisation selon la revendication 13, ladite tauopathie étant choisie parmi la maladie de Parkinson, la maladie d'Alzheimer, la démence à corps de Lewy, la maladie de Pick, la paralysie supranucléaire progressive, l'encéphalopathie traumatique chronique, le parkinsonisme lié au chromosome 17, la maladie de Lytico-Bodig, la démence à prédominance enchevêtrée, la maladie des grains argyrophiles, le gangliogliome, le gangliocytome, la méningioangiomatose, la panencéphalite sclérosante subaiguë, l'encéphalopathie au plomb, la sclérose tubéreuse de Bourneville, la maladie de Hallervorden-Spatz, la lipofuscinose, la dégénérescence corticobasale, la démence frontotemporale, la dégénérescence lobaire frontotemporale et la maladie de Huntington.

15. Composé ou sel pharmaceutiquement acceptable pour une utilisation selon l'une quelconque des revendications 1 à 12, ladite protéoinopathie étant une synucléinopathie.

16. Composé ou sel pharmaceutiquement acceptable pour une utilisation selon la revendication 15, ladite synucléinopathie étant choisie parmi la démence à corps de Lewy, la maladie de Parkinson et l'atrophie multisystématisée.
